(19) Europäisches Patentamt
European Patent Office
Office européen des brevets

(11) Publication number: 0 090 578 B1

# (12) EUROPEAN PATENT SPECIFICATION

(45) Date of publication of patent specification: 12.07.89

(21) Application number: 83301584.5

(22) Date of filing: 22.03.83

(51) Int. Cl.[4]: A 23 K 1/17, A 61 K 37/02, C 07 K 1/00, C 12 P 21/00 // (C12P21/00, C12R1:465)

## (54) A41030 antibiotics and their production.

(30) Priority: 24.03.82 US 361301
24.03.82 US 361302
22.11.82 US 443496

(43) Date of publication of application:
05.10.83 Bulletin 83/40

(45) Publication of the grant of the patent:
12.07.89 Bulletin 89/28

(84) Designated Contracting States:
AT BE CH DE FR GB IT LI LU NL SE

(56) References cited:
EP-A-0 055 069
EP-A-0 055 070

(73) Proprietor: ELI LILLY AND COMPANY
307, East McCarty Street
Indianapolis Indiana 46285 (US)

(72) Inventor: Kastner, Ralph Emil
243 Webb Drive
Indianapolis Indiana 46227 (US)
Inventor: Michel, Karl Heinz
225 East North Street Apt. 2205
Indianapolis Indiana 46204 (US)
Inventor: Nakatsukasa, Walter Mitsuo
2118 Crossman Drive
Indianapolis Indiana 46227 (US)

(74) Representative: Hudson, Christopher Mark et al
Erl Wood Manor
Windlesham Surrey GU20 6PH (GB)

EP 0 090 578 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel fermentation products having antimicrobial properties and to the method of preparing those antibiotics by cultivation of a previously unknown microorganism of the *Streptomyces* family.

These new compounds are similar to the antibiotics described in U.S. Patent Nos. 4,322,343 and 4,322,406.

There is a great need to develop new antibiotics because of the great possibility and constant threat that antibiotic-specific resistant strains of pathogenic microorganisms will emerge. In particular, pathogens within the gram positive genera *Staphylococcus* and *Streptococcus* often are resistant to commonly used antibiotics such as penicillin and erythromycin; see for example, W. O. Foye, *Principles of Medicinal Chemistry,* pp 684—686 (1974).

In accordance with the invention, the A41030 complex and its Factors A, B, C, D, E, F and G or the pharmaceutically-acceptable salts thereof, have been found to be effective antimicrobial agents.

These new antibiotics can be prepared by aerobic fermentation of the previously undescribed microorganism, *Streptomyces virginiae* NRRL 12525, or its parent, *Streptomyces virginiae* NRRL 15156, in a culture medium containing assimilable sources of carbon, nitrogen and inorganic salts. These organisms and others produce an antibiotic complex comprising several antibiotic factors, including those designated as Factors A, B, C, D, E, F and G. For convenience, the cultured microorganism, *Streptomyces virginiae* NRRL 12525, has been designated in our laboratory as culture A41030.4. *Streptomyces virginiae* NRRL 15156 has been designated in our laboratory as A41030.

The infrared absorption spectra of the individual factors are presented in the accompanying drawings as follows:

Figure 1 — A41030 factor A (in KBr pellet)
Figure 2 — A41030 factor B (in KBr pellet)
Figure 3 — A41030 factor C (in KBr pellet)
Figure 4 — A41030 factor D (in KBr pellet)
Figure 5 — A41030 factor E (in KBr pellet)
Figure 6 — A41030 factor F (in KBr pellet)
Figure 7 — A41030 factor G (in KBr pellet).

The term "complex", as used in the fermentation art, and in this specification, refers to a mixture of coproduced individual antibiotic factors. As will be recognized by those familiar with antibiotic production by fermentation, the number and ratio of the individual factors produced in an antibiotic complex will vary, depending upon the fermentation conditions and the strain used.

Culture A41030.4, a chemically-induced mutant of a strain of the *Streptomyces virginiae* (A41030) culture which was initially isolated from a soil sample collected on Indianapolis, Indiana, was deposited on 4th September 1981 and made a part of the stock culture collection of the Northern Regional Research Center, U.S. Department of Agriculture, Agricultural Research Service, Peoria, Illinois 61604, from which it is available to the public under the number NRRL 12525. The complex-producing parent culture, *Streptomyces virginiae* (A41030) was deposited also at the Northern Regional Research Center on 22nd September 1982 and is publicly available under the accession number NRRL 15156.

Culture A41030.4 was obtained by treatment of culture A41030 sequentially with mitomycin C and then N-methyl-N'-nitro-N-nitrosoguanidine.

It will be recognized by those skilled in the art that it would be possible to generate additional strains which have essentially the same biosynthetic capabilities as *Streptomyces virginiae* NRRL 12525, by subjecting culture A41030 to other mutagenic treatments. In addition to mitomycin C, other suitable agents include acriflavines, acridine orange, ethidium bromide, and similar chemical agents. Although N-methyl-N'-nitro-N-nitrosoguanidine was used, along with mitomycin C, to obtain NRRL 12525, other known mutagens such as ultraviolet, high-frequency, and radioactive radiations, X-rays and other chemical agents could be used to induce a similar mutagenesis.

A41030 is classified as a strain of *Streptomyces virginiae,* and culture A41030.4 is classified as a chemically-induced mutant of a strain of *Streptomyces virginiae,* based upon a simultaneous culturing of *Streptomyces avidinii* ATCC 27419; *Streptomyces columbiensis* ATCC 27425; *Streptomyces goshikiensis* ATCC 23914; *Streptomyces griseolavendus* ATCC 25457; *Streptomyces lavendulae* ATCC 8664; *Streptomyces toxytricini* ATCC 19813; and *Streptomyces virginiae,* Grundy, Whitman, Rdzok, Hanes and Sylvester 1952, ATCC 19817. The methods and media recommended by Shirling and Gottlieb, ["Methods of Characterization of *Streptomyces* Species", *Int. J. Syst. Bacteriol. 16(3),* 313—340 (1966)], along with certain supplementary tests were used. Culture A41030.4 was also compared with published descriptions of the above-named strains appearing in "Bergey's Manual of Determinative Bacteriology" (8th Edition, edited by R. E. Buchanan and N. E. Gibbons, The Williams and Wilkins Co., Baltimore, Maryland); and by Shirling and Gottlieb, "Cooperative Description of Type Strains of *Streptomyces", Int. J. Syst. Bacteriol. 18(2),* 178 (1968).

Since Culture A41030.4 produces no aerial mycelia and no spores on any medium, it differs from all the above-named species.

CHARACTERIZATION OF THE A41030 AND A41030.4 CULTURES

Morphology

A41030 produces well-developed aerial mycelia and sporophores which are both coiled and exhibit hooks and loops. A41030 is placed in the spirales (s) section of Pudham *et al.,* "A Guide for the Classification of Streptomycetes According to Selected Groups", *Appl. Microbiol. 6,* 52—79 (1957), as a primary morphology type, and in the Tetinaculum-Apertum (RA) section of Pridham *et al.* as a secondary morphology type. A41030.4 produces no aerial mycelia and no spores.

Cultural Characteristics

The growth characteristics of culture A41030, A41030.4, and *S. Virginiae* ATCC 19817 on various media are presented in Table 1.

Color names were assigned according to the ISCC—NBS Centroid Color Charts Standard Sample No. 2106 (National Bureau of Standards, U.S Department of Commerce, 1958), and the Color Harmony Manual, 4th Edition (Color Standards Department, Container Corporation of America, Chicago, Illinois, 1958).

TABLE 1
Cultural Characteristics of
A41030, A41030.4, and ATCC 19817

| Medium | | A41030 | A41030.4 | ATCC 19817 |
|---|---|---|---|---|
| | G | Abundant | Good | Abundant |
| ISP | R | 72.d.OY | 90.gy.Y | 75.deep yBr |
| No. 2 | Am | Abun:63.1.brGY | None | Abun:63.1.brGY |
| | Sp | None | None | None |
| | G | Good | Poor | Fair |
| ISP | R | 93.yGray | 93.yGray | 90.gy.Y |
| No. 3 | AM | Good:63.1.brGY | None | Fair:63.1.brGY |
| | Sp | None | None | None |
| | G | Abundant | Poor | Good |
| ISP | R | 89.p.Y | 89.p.Y | 91.d.gy.Y |
| No. 4 | Am | Abun:63.1.brGY | None | Good:63.1.brGY |
| | Sp | None | None | None |
| | G | Abundant | Poor | Good |
| ISP | R | 89.p.Y | 89.p.Y | 89.p.Y |
| No. 5 | Am | Abun:22.rGY | None | Good:63.1.brGY |
| | Sp | None | None | None |
| | G | Fair | Not grown | Poor |
| Czapek's | R | 264.1.Gray | - | 264.1.Gray |
| agar | Am | Poor:10.pKGY | - | Poor:10.pKGY |
| | Sp | None | - | None |
| | G | Abundant | Good | Abundant |
| TPO | R | 72.d.OY | 54.brO | 75.deep yBr |
| | Am | Abun:63.1.brGY | None | Abun:63.1.brGY |
| | Sp | None | None | None |

G = growth R = reverse Am = aerial mycelia Sp = soluble pigment

The Culture A41030.4 was studied for selected physiological properties in accordance with standard procedures. The properties observed and characteristics found are recorded in Table 2.

TABLE 2

PHYSIOLOGICAL PROPERTIES OF A41030.4

| | |
|---|---|
| Melanoid-like Pigment Production on: | |
| 1. Tryptone yeast extract broth (ISP #1) | Melanoid-like pigment |
| 2. Peptone yeast extract iron agar slants (ISP #6) | Melanoid-like pigment |
| 3. Tyrosine agar slants (ISP #7) | No melanoid-like pigment |
| Nitrate Reduction | Negative reaction |
| Gelatin Liquefaction | Negative reaction |
| NaCl Tolerance | 3% |
| Starch Hydrolysis | Negative reaction |
| Skim Milk | Partial hydrolysis |
| Temperature Requirements | 10-34° C. |

A comparison of the carbon utilization patterns of culture A41030, culture A41030.4 and *Streptomyces virginiae* ATCC 19817 was conducted using ISP No. 9 basal medium to which filter-sterilized carbon sources were added to equal a final concentration of 1.0%. Plated were read after fourteen days incubation at 30°C. The results are set forth in Table 3.

TABLE 3

CARBON UTILIZATION PATTERNS OF A41030, OF A41030.4, AND STREPTOMYCES VIRGINIAE ATCC 19817

| Carbon Source | A41030 | A41030.4 | ATCC 19817 |
|---|---|---|---|
| Acetate-Na | - | - | - |
| D-Arabinose | - | - | - |
| L-Arabinose | - | - | - |
| Cellobiose | + | + | + |
| D-Fructose | ± | - | ± |
| D-Galactose | + | + | - |
| D-Glucose | + | + | + |
| i-Inositol | - | - | - |
| Lactose | - | - | - |
| D-Maltose | + | + | + |
| D-Mannitol | - | - | - |
| Melibiose | - | - | - |
| Raffinose | - | - | - |
| Rhamnose | - | - | - |
| D-Ribose | + | - | + |
| Salicin | + | ± | + |
| Succinate-Na | + | ± | + |
| Sucrose | - | - | - |
| D-Xylose | - | - | - |

Key: - = no utilization
+ = utilization
± = partial utilization

Using hydrolyzed whole cells of the organism, the isomers of diaminopimelic acid were determined according to the method of Becker *et al., Appl. Microbiol, 11,* 421—423 (1964). The results of this study are set forth below.

| Test | Result Observed |
|---|---|
| Isomers of 2,6-diamino-pimelic acid | LL-isomer |

A comparison of the similarities and differences between culture A41030, culture A41030.4 and *Streptomyces virginiae* ATCC 19817 is set forth in Table 4.

Color names are assigned as described above, and the morphology as set forth in Pudham, *et al., supra.*

TABLE 4

Similarities and Differences of A41030, A41030.4, and S. virginiae ATCC 19817

| Character | A41030 | A41030.4 | ATCC 19817 |
|---|---|---|---|
| Aerial spore color | GY | none | Gy |
| Carbon utilization | + | + | + |
| (galactose) | + | + | - |
| (fructose) | + | - | + |
| (ribose) | + | - | + |
| Gelatin liquefaction | + | - | + |
| Melanin production | + | + | + |
| ISP No. 1 | + | + | - |
| ISP No. 6 | + | + | + |
| ISP No. 7 | - | - | - |
| Morphology | (RA) (S) | none | (RA) (S) |
| NaCl tolerance percent | 4 | 3 | 5 |
| Nitrate reduction | - | - | - |
| Optimum growth medium | + | - | + |
| Reverse color | p.Y | p.Y | p.Y |
| Skim milk | + | + | + |
| Soluble pigment | - | - | - |
| Spore shape | oblong | none | oblong |
| Spore surface | smooth | none | smooth |
| Starch hydrolysis | - | - | - |
| Temperature range °C. | 10-37 | 10-34 | 15-37 |

The A41030 complex can be produced by culturing the previously undescribed microorganism *Streptomyces virginiae* NRRL 15156, *Streptomyces virginiae* NRRL 12525, or an A41030-producing mutant or variant thereof, in a culture medium containing assimilable source of carbon, nitrogen, and inorganic salts, under submerged aerobic fermentation conditions until a substantial level of antibiotic activity is produced. Most of the antibiotic activity is found in the broth, while minor amounts of antibiotic activity may be associated with the mycelia. The A41030 complex is most readily separated from the fermentation mixture by removal of the mycelia, i.e., the biomass, by filtration. The mycelia generally are discarded. The antibiotic complex is then isolated from the filtered fermentation broth preferably by column chromatography, over a suitable adsorbent using for example methanol:water (1:1) as the eluting agent.

Suitable adsorbents include carbon, alumina, anion and cation exchange resins, silica gel, polyamide, carboxymethylcelluloses, highly porous copolymers of styrene and divinylbenzene such as Diaion HP-20, the Amberlite XAD resins, and the Duolite resins such as ES-865 and the like, as well as Sephadex resins, the hydrophilic, insoluble, molecular-sieve chromatographic mediums made by cross-linking dextran, and also TSK Gels. The Diaion resins are a product of Mitsubishi Chemical Industries, Limited, Tokyo, Japan. The Amberlite XAD resins are produced by Rohm and Haas, Philadelphia, Pennsylvania. The Duolite resins are products of Diamond Shamrock, Redwood City, California. Sephadex resins are manufactured by Pharmacia Fine Chemicals AB, Uppsala, Sweden. The TSK Gels are available from E. Merck, Darmstadt, and from Bio-Rad, 2200 Wright Ave., Richmond, California, 94804.

The A41030 antibiotic complex can be purified further and separated into its individual factors by chromatographic techniques.

A number of different media may be used with *Streptomyces virginiae* (NRRL 12525 or *S. virginiae* NRRL 15156, to produce the A41030 complex. For economy in production, optimal yield, and ease of product isolation, however, certain culture media are preferred. These media should contain assimilable sources of carbon, nitrogen, and inorganic salts. Suitable carbon sources include dextrin, starch, mannose, glycerol, and cottonseed oil. Optimum levels of carbon sources are from about 2 to about 3 percent by weight.

Preferred nitrogen sources include soybean grits, soybean flour, peanut meal, fish meal, meat peptone, and pork blood meal.

Essential trace elements necessary for the growth and develpment of the organism may occur as impurities in other constituents of the media in amounts sufficient to meet the growth and biosynthetic requirements of the organism. However, it may be beneficial to incorporate in the culture media additional soluble nutrient inorganic salts capable of yielding sodium, potassium, magnesium, calcium, ammonium, chloride, carbonate, phosphate, sulfate, nitrate and like ions.

Addition to the fermentation medium of Tween 80 (oily liquid polyoxyethylene sorbitan monooleate, a product of ICI Americas, Inc., Wilmington, Del.), at a level of 2—4% serves to increase the yield by about 300%. However, difficulty is experienced in isolating the A41030 antibiotic under these conditions.

Although small quantities of the A41030 antibiotic may be obtained by shake-flask culture, submerged aerobic fermentation in tanks is preferred for producing substantial quantities of the A41030 antibiotic. For tank fermentation, it is preferable to use a vegetative inoculum. The vegetative inoculum is prepared by inoculating a small volume of culture medium with the spore form, or mycelial fragments, to obtain a fresh, actively growing culture of the organism. The vegetative inoculum is then transferred to a larger tank where, after a suitable incubation time, the A41030 antibiotic is produced in optimal yield.

An alternate method of providing inoculum for the vegetative medium consists of substituting a lyophilized pellet for the aqueous spore suspension. Lyophilized pellets are prepared in a manner known in the art. Preparation of the spore suspension for lyophilization is similar to the preparation of the aqueous spore suspension, except that sterile calf serum is substituted for sterile distilled water.

The A41030-producing organism can be grown over a broad temperature range of from about 10 to about 34°C. Optimum production of A41030 antibiotic complex appears to occur at a temperature of about 30°C.

As is customary in aerobic submerged culture processes, sterile air is dispersed through the culture medium. For efficient growth of the organism, the volume of the air used in tank production is in the range of from about 0.1 to about 0.5 volumes of air per volume of culture medium per minute (v/v/m), with from about 100 to about 300 RPM agitation. An optimum rate in a 165-liter vessel containing 100 liters of fermentation medium is about 0.25 v/v/m, with agitation provided by an impeller rotating at about 200 RPM.

Antibiotic activity generally is present after about 48 hours and remains present for at least 144 hours during the fermentation period. Peak antibiotic production occurs at from about 96 hours to about 120 hours fermentation time.

Production of the A41030 antibiotic can be monitored during the fermentation by either agar diffusion using *B. subtilis*, or a turbidimetric method using *Staphylococcus aureus* ATCC 9114.

The antibiotic complex, and individual factors thereof, provided by this invention are produced by either *S. virginiae* NRRL 15156 or *S. virginiae* NRRL 12525 under fermentation conditions of temperature, duration, and media ingredients which are substantially equivalent. However, it appears that under such conditions, *S. virginiae* NRRL 12525 produces the antibiotic complex in somewhat greater abundance.

In order to illustrate more fully the invention, the following non-limiting Examples are provided.

Example 1

Preparation of First Stage Inoculum.

The following medium was prepared for use in the agar slant culture of *Streptomyces virginiae* NRRL 12525 and *Streptomyces virginiae* NRRL 15156:

| Ingredient | Amount (g/L.) |
|---|---|
| Dextrin[1] | 10.0 |
| Yeast extract | 1.0 |
| Enzyme-hydrolyzed casein[2] | 2.0 |
| Beef extract | 1.0 |
| $CoCl_2 \cdot 6H_2O$ | 0.01 |
| Agar | 20.0 |
| Deionized water | q.s. to 1 liter |

[1]Matheson Coleman & Bell, Norwood, Ohio 45212
[2]N-Z-Amine A (Humko Sheffield Chemical Co., Memphis, Tenn.).

The pH of the medium as prepared was 6.5, and was adjusted to 7.3 using 5N aqueous sodium hydroxide before autoclaving. After autoclaving, the pH of the medium was 6.9.

The spores of each microorganism were inoculated on an agar slant made up of the above-identified ingredients, and this slant was incubated for about six days at a temperature of about 30°C. The mature slant culture was then covered with sterile distilled water and scraped with a sterile tool to loosen the spores and the mycelium. One milliliter of the resulting spore suspension was used to inoculate 50 ml. of vegetative medium. An alternate method of providing inoculum for the vegetative medium consisted of substituting a lyophilized pellet for the aqueous spore suspension. The composition of the vegetative medium for NRRL 12525 was as follows:

| Ingredient | Amount (g/L.) |
|---|---|
| Glucose | 20.0 |
| Soybean grits (or soybean flour) | 15.0 |
| Corn steep liquor | 10.0 |
| $CaCO_3$ | 2.0 |
| Tap water | q.s. to 1 liter. |

The composition of the vegetative medium for NRRL 15156 was as follows:

| Ingredient | Amount (g/L.) |
|---|---|
| Glucose | 15.0 |
| Dextrin | 20.0 |
| Soybean grist (or soybean flour) | 15.0 |
| Corn steep liquor | 10.0 |
| $CaCO_3$ | 2.0 |
| Tap water | q.s. to 1 liter |

The unadjusted pH of the medium was 5.5, which was adjusted to pH 6.5 with 5N aqueous sodium hydroxide before autoclaving. The pH of the medium after autoclaving was 7.0.

Each vegetative inoculum was incubated in a 250 ml. wide-mouth Erlenmeyer flask containing 50 ml. of medium at about 30°C. for about 48 hours on a shaker rotating through an arc 2 inches in diameter at 250 RPM. This incubated medium is used either to inoculate small fermenters (the inoculum being approximately 1% per volume of fermenter medium) or to inoculate a second stage medium having the same composition as the vegetative medium for the production of a larger volume of culture.

Fermentation of NRRL 15156

Fifty milliliters of a production medium was inoculated with 1% (0.5 ml.) of the incubated vegetative medium from above. The production medium had the following composition:

| Ingredient | Amount (g/L.) |
|---|---|
| Dextrin[1] | 30.0 |
| Soybean grits | 6.0 |
| $K_2HPO_4$ | 1.0 |
| $FeSO_4 \cdot 7H_2O$ | 0.005 |
| $MgSO_4 \cdot 7H_2O$ | 1.0 |
| $NaNO_3$ | 1.0 |
| $CaCO_3$ | 2.0[2] |
| Deionized water | q.s. to 1 liter |

[1]The dextrin may be either tapioca or potato dextrin.
[2]For the fermentation of NRRL 12525, the concentration of $CaCO_3$ was 4.0 g./L.

The $K_2HPO_4$ was dissolved in water, the solution sterilized separately, and the requisite amount of the solution added to the other ingredients of the medium which had been autoclaved.

The inoculated fermentation medium, 50 ml., was incubated in a 250 ml. Erlenmeyer flask at about 30°C. for about 4—5 days on a shaker rotating through an arc 2 inches in diameter at 250 RPM.

The *Streptomyces virginiae* NRRL 12525 was also incubated in a fermentation carried out on a larger scale in 165-liter and 1600 gallon tanks using the production medium described immediately hereinabove.

The inoculated production medium was allowed to ferment in 165-liter fermentation tank containing 100 liters of medium for about 210 hours (8.75 days) at a temperature of about 32°C. The fermentation medium was aerated with sterile air at a rate of 0.25 v/v/m and was stirred with conventional agitators at about 200 RPM.

This large scale fermentation of NRRL 12525 was the source from which the A41030 antibiotics were separated, as described below.

Example 2

Separation of A41030 Antibiotics

Whole fermentation broth (4215 liters), obtained as described in Example 1, was filtered using a filter aid (Hyflo Supercel, a diatomaceous earth, Johns-Manville Products Corporation) in a filter press. The filtered broth was applied to a column containing 100 L. of Diaion HP-20 (a highly porous styrenedivinylbenzene copolymer in bead form, Mitsubishi Chemical Industries, Limited, Tokyo, Japan) at a flow rate of 4 L./min. The column was washed successively with 300 L. of water and 1000 L. of methanol:water (1:3) at a rate of 4 L./min. Elution was performed with methanol:water (1:1) at the rate of 6 L./min., collecting 100—L. fractions. Each fraction was analyzed for biological activity. The bioassay was performed by a paper disc assay on agar plates seeded with *Bacillus subtilis.* Fraction 1 was discarded. Fractions 2—15, inclusive, were combined, concentrated under reduced pressure, and the concentrate lyophilized to give 220 g. of crude antibiotic complex.

A portion of this complex, 110 g., was dissolved in 5 L. of methanol:water (1:1), by adjustment to pH 10 with aqueous sodium hydroxide, and the mixture was filtered. The filtrate was applied at 50 ml./min. to a 30—L. column (0.2 × 1 m.) of coarse Sephadex G-50 (a hydrophilic, insoluble, molecular-sieve chromatographic medium, made by cross linking dextran, and sold by Pharmacia Fine Chemicals, Piscataway, NJ 08854), previously equilibrated with methanol:water (1:1). The column was eluted with methanol:water (1:1) at 50 ml./min., collecting 3—L. fractions. Fractions 1—12, inclusive, were discarded. Fractions 13—24, inclusive, which were active against *B. subtilis,* were combined, concentrated under reduced pressure, and lyophilized to give 35.7 g. of the A41030 antibiotic complex.

The antibiotic substances produced in accordance with this invention are arbitrarily designated herein as A41030 antibiotics. The A41030 complex contains several individual factors which are designated A41030 factors A, B, C, D, E, F, and G. In discussions of utility, the term "A41030 antibiotic" will be used, for the sake of brevity, to denote a member selected from the group consisting of A41030 complex, and A41030 factors A, B, C, D, E, F, and G.

As many as seven antibiotic factors are recovered from the fermentation and are obtained as a mixture, the A41030 complex. It will be recognized that the ratio of the factors in the A41030 complex will vary, depending upon the fermentation conditions used. The individual factors A, B, C, D, E, F and G are separated and isolated as individual compounds, as later described. The A41030 complex is soluble in solvents such as water, dilute aqueous acid, dilute aqueous base, methanol-water mixtures, ethanol-water mixtures, dimethylformamide and dimethylformamide-water mixtures, dimethylsulfoxide, dimethylsulfoxide-water mixtures, acetonitrile, acetone, ethyl acetate, tetrahydrofuran, or methylene chloride.

The following paragraphs describe the separation, as well as the physical and spectral properties of the A41030 factors which have thus far been characterized.

Example 3

Isolation of A41030 Factor A

An 8 g. portion of the A41030 complex from Example 2 was dissolved in 200 ml. of a solvent consisting of water:acetonitrile:sodium chloride (84:16:2 g./L.) and filtered. The filtrate was applied to a stainless steel column (8 × 100 cm.) packed with 4 L. of 10—20 micron LP-1/$C_{18}$ reversed-phase silica gel which was prepared in our laboratories by a special procedure described in Examples 6 and 7 of U.S. Patent No. 4,299,763. The column was part of a Chromatospac Prep-100 unit (Jobin Yvon, 16—18 Rue du Canal 91160 Longjumeau, France). The column was eluted at 60 ml./min. with water:acetonitrile:sodium chloride (84:16:2 g./L.) collecting 480 ml. fractions. The eluate was monitored at 254 nm using an ISCO Model UA-5 UV monitor with a Type 6 optical unit (Instrumentation Specialties Co., Lincoln, NE 68505). Selected fractions were analyzed for the presence of Factor A by analytical high performance liquid chromatography (HPLC) on a 4.6 × 250 mm. stainless steel column packed in our laboratories with 10 micron LP-1/$C_{18}$ which was prepared in our laboratories by the special procedure described above. The sample was applied with a Rheodyne Model 7120 injection valve (Rheodyne Inc., Berkeley, CA 94710). The solvent, consisting of water:acetonitrile:sodium acetate (81:19:0.03M) adjusted to pH 6 with glacial acetic acid, was supplied at 1 ml./min. (1200 psi) by a Milton Roy Duplex Minipump (Laboratory Data Control, Division of Milton Roy Co., Rivera Beach, FL 33404). Factor A was detected at 254 nm using an ISCO Model UA-5 UV detector. Fractions 1—51, inclusive, were discarded. Fractions 52—79, inclusive, rich in factor A were combined and concentrated under reduced pressure to a volume of 500 ml. The concentrate was adjusted to pH 8.2 with aqueous sodium hydroxide and filtered. The filtrate was applied at 15 ml./min. to 100 ml. of Diaion HP-20 resin in a column (2.8 × 22 cm.) previously equilibrated with water. The column was washed with water (400 ml. adjusted to pH 2.5 with formic acid) until no chloride was detected in the wash by precipitation as silver chloride. Elution was performed with water:acetonitrile (8:2) at 15 ml./min., collecting 1 L. fractions. Fractions were analyzed for activity against *B. subtilis.* Crystalline factor A, which formed in fraction 2 upon refrigeration, was recovered by filtration (389.6 mg.). Fraction 1 and the filtrate from fraction 2 were each concentrated under reduced pressure and lyophilized to give 731.8 mg. and 514 mg. of factor A, respectively.

Antibiotic A41030 factor A is a white, crystalline solid. Elemental analysis of A41030 factor A indicates that it has the following approximate percentage composition: 56.44% carbon, 3.58% hydrogen, 8.11% nitrogen, 23.20% oxygen, and 8.29% chlorine. As determined by field desorption and plasma desorption mass spectrometry, A41030 factor A has a molecular weight of 1231. Based on the elemental analysis and the molecular weight, an empirical formula of $C_{58}H_{44}Cl_3N_7O_{18}$ is assigned to factor A. Electrometric titration of factor A in 66% dimethylformamide in water indicated the presence of three titratable groups having $pK_a$ values of about 5.53, 7.60 and 10.37, with possible additional $pK_a$'s >10.5 (initial pH 7.83). Antibiotic A41030 factor A has the following specific rotation: $[\alpha]_D^{25}$ −19.6° (c, 9.0 in dimethylsulfoxide).

The infrared absorption spectrum of A41030 factor A in KBr pellet is shown in the accompanying drawings as Fig. 1. The following distinguishable absorption maxima are observed: 3448—3226 (strong, broad), 1653 (strong), 1610 (weak), 1587 (medium), 1515 (strong), 1488 (weak), 1429 (medium), 1227 (strong), 1139 (medium), 1064 (strong), and 1010 (strong) $cm^{-1}$.

The ultraviolet absorption maxima of A41030 factors A through G in methenol:water (1:1) under acid, neutral, and basic conditions are recorded in Table 5.

TABLE 5

UV Spectrophotometry of A41030 Factors

| Factor | Acidic or Neutral max nm (ε) | Basic max nm (ε) |
|---|---|---|
| A | 278 (11,100) | 298 (17,200) |
| B | 278 (9,600) | 298 (16,800) |
| C | 278 (8,400) | 298 (14,000) |
| D | 278 (10,600) | 298 (19,900) |
| E | 278 (8,500) | 298 (15,500) |
| F | 278 (9,300) | 298 (14,500) |
| G | 278 (15,000) | 298 (18,000) |

Antibiotic A41030 factor A is soluble in alcohol-water mixtures, in dimethylsulfoxide, in dimethylformamide, in dimethylsulfoxide-water mixtures, in dimethylformamide-water mixtures, in dilute aqueous acid, and in dilute aqueous base.

On the basis of the observed physical chemical data, the following structure has been assigned to A41030 factor A:

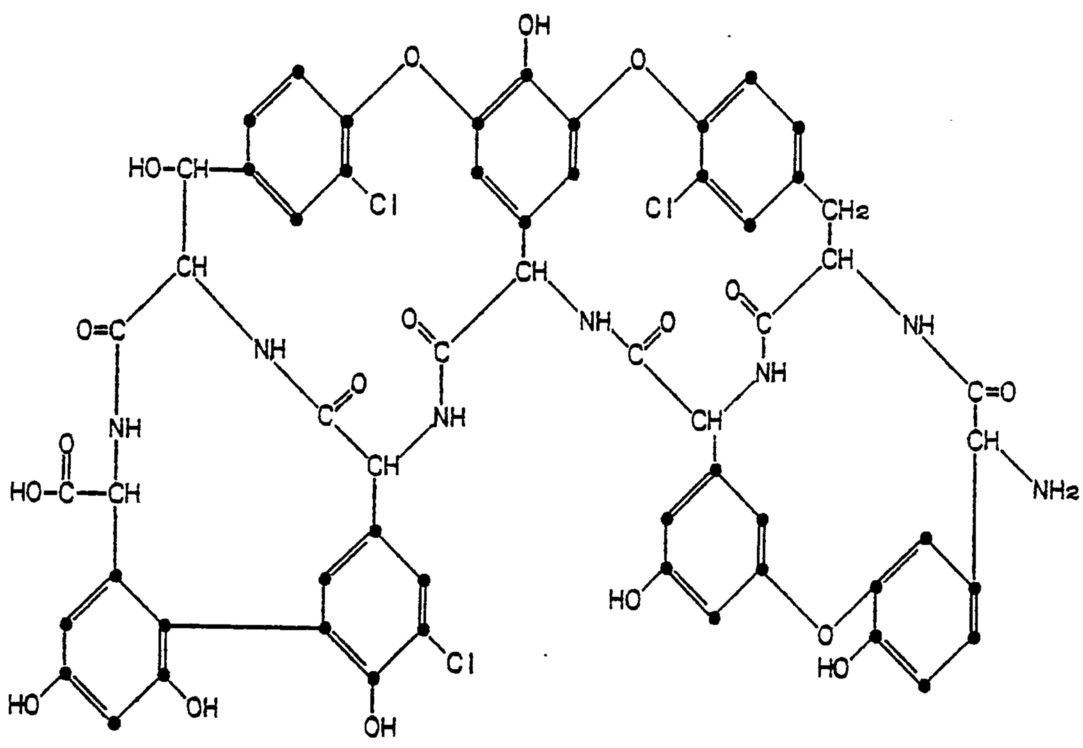

Using a biological assay and high performance liquid chromatography analysis, it has been found that factor A accounts for from about 94 to about 96% by weight of the antibiotic factors produced by culture A41030.4, with factors B, C, D, E, F, and G accounting for the remaining about 4 to about 6% by weight of the factors produced.

Example 4

Isolation of A41030 Factor B

A 1.0 g. portion of the A41030 complex was dissolved in 35 ml. of a solvent consisting of water:acetonitrile:sodium chloride (85:15:2 g./L.) and the solution was applied to a 4.7 × 45 cm. Michel-Miller high-performance-low-pressure-liquid-chromatography (HPLPLC) glass column (Ace Glass, Inc., Vineland, NJ 08360) packed in our laboratories with 25—40 micron LiChroprep RP-18 [hydrocarbon phase

($C_{18}$) chemically bonded to silica gel, from MC/B Manufacturing Chemists, Inc., Cincinnati, OH]. An FMI valveless piston pump (Fluid Metering Inc., Oyster Bay, NY 11771) was used to elute the column at 21 ml./min. (100 psi.), with the same solvent combination used for sample dissolution, collecting 21-ml. fractions. The elutate was monitored at 280 nm using an ISCO Model UA-5 UV detector. Fractions 1—183, inclusive, were discarded. Fractions 184—245, inclusive, rich in factor B, were combined and concentrated under reduced pressure to 25 ml. Concentrates from seven similar purifications were combined, diluted to 1.4 L. with water, and applied at 8—10 ml./min. to 100 ml. of Diaion HP-20 resin in a column, previously equilibrated with water. The column was washed with water (600 ml.) until no chloride was detected in the wash by precipitation as silver chloride. Elution was performed with water:methanol (1:1) at 8—10 ml./min., collecting 300 ml. fractions. Fractions were analyzed for activity against *B. subtilis.* Fractions 1—5 were combined, concentrated under reduced pressure, and lyophilized to give 523 mg. of crude factor B.

A 550 mg. portion of two combined crude preparations of factor B was dissolved in 10 ml. of a solvent consisting of water:acetonitrile:dibutylamine (75:25:0.03M, which solvent had been adjusted to pH 7.8 with phosphoric acid) by addition of tetrabutylammonium hydroxide until solution had been accomplished. The solution was applied to a 2.8 × 59 cm. Michel-Miller HPLPLC glass column packed with 25—40 micron LiChroprep RP-8 [hydrocarbon phase ($C_8$) chemically bonded to silica gel, from MC/B Manufacturing Chemists, Inc., Cincinnati, OH]. Using an FMI pump, the column was eluted at 5 ml./min. (35 psi) with the same solvent combination used for sample dissolution. The eluate was monitored at 254 nm using an ISCO Model UA-5 UV detector. Selected 27 ml. fractions were analyzed for the presence of factor B by analytical HPLC on a 4.6 × 250 mm. stainless steel column packed with 10 micron LiChrosorb RP-18 (a commercially available, reversed-phase silica gel, manufactured by E. Merck, Darmstadt, Germany). The sample was applied using a Rheodyne Model 7120 injection valve. The solvent, consisting of water:acetonitrile:dibutylamine (82:18:0.03M) adjusted to pH 2.5 with phosphoric acid, was supplied at 1 ml./min. (750 psi.) by a Constametric III pump (LDC-Laboratory Data Control, Division of Milton Roy Co., Riviera Beach, FL 33404). Factor B was detected at 225 nm using an LDC Spectro Monitor III variable wavelength UV detector. The portion of the RP-8 column eluate from 999—1296 ml., rich in factor B, was concentrated to a volume of 200 ml. The concentrate was diluted to a volume of 500 ml., adjusted to pH 2.0 with phosphoric acid, and sodium chloride (1 mg./ml.) was added as an ionic marker. This solution was applied at 20 ml./min. to 100 ml. of Diaion HP-20 resin in a column (2.8 × 22 cm.), previously equilibrated with water. The column was washed with water (500 ml.) adjusted to pH 2.5 with aqueous formic acid, until no chloride was detected in the wash by precipitation as silver chloride. The column was then eluted with 1 L. of water:acetonitrile (6:4) at 30 ml./min. The eluate was concentrated under reduced pressure and lyophilized to give 295.6 mg. of crude factor B.

A 285 mg. portion of this preparation was dissolved in 30 ml. dimethylformamide:water (4:6) by heating, cooled to room temperature, and refrigerated, resulting in precipitation of factor B. The precipitate was recovered by filtration, washed with acetone, and dried under vacuum, to yield 84 mg. of factor B.

Antibiotic A41030 factor B is a white solid, having an approximate elemental analysis as follows:

58.54% carbon, 4.21% hydrogen, 8.63% nitrogen, 5.96% chlorine, and by difference, 22.66% oxygen. Electrometric titration of factor B in 66% dimethylformamide in water indicated the presence of two titratable groups at $pK_a$ values of about 5.6 and 7.5, respectively, with possible additional $pK_a$'s >10 (initial pH 6.22). An observed molecular weight of about 1197 was obtained using fast atom bombardment mass spectrometry. Based on elemental analysis and the observed molecular weight, an empirical formula of $C_{58}H_{45}Cl_2N_7O_{18}$ is assigned to factor B.

The infrared absorption spectrum of antibiotic A41030 factor B in KBr pellet is shown in the accompanying drawings as Fig. 2. The following distinguishable absorption maxima are observed: 3448—3226 (strong, broad), 1653 (strong), 1610 (medium), 1587 (weak), 1515 (strong), 1488 (weak), 1429 (medium), 1290 (weak), 1227 (strong), 1139 (medium), 1064 (strong), and 1010 (strong) $cm^{-1}$.

The ultraviolet absorption maxima of A41030 factor B in neutral, acidic, and basic methanol:water (1:1) are recorded in the foregoing Table 5.

Antibiotic A41030 factor B is soluble in the same solvents as factor A.

On the basis of the observed physical chemical data, the following structure has been assigned to A41030 factor B:

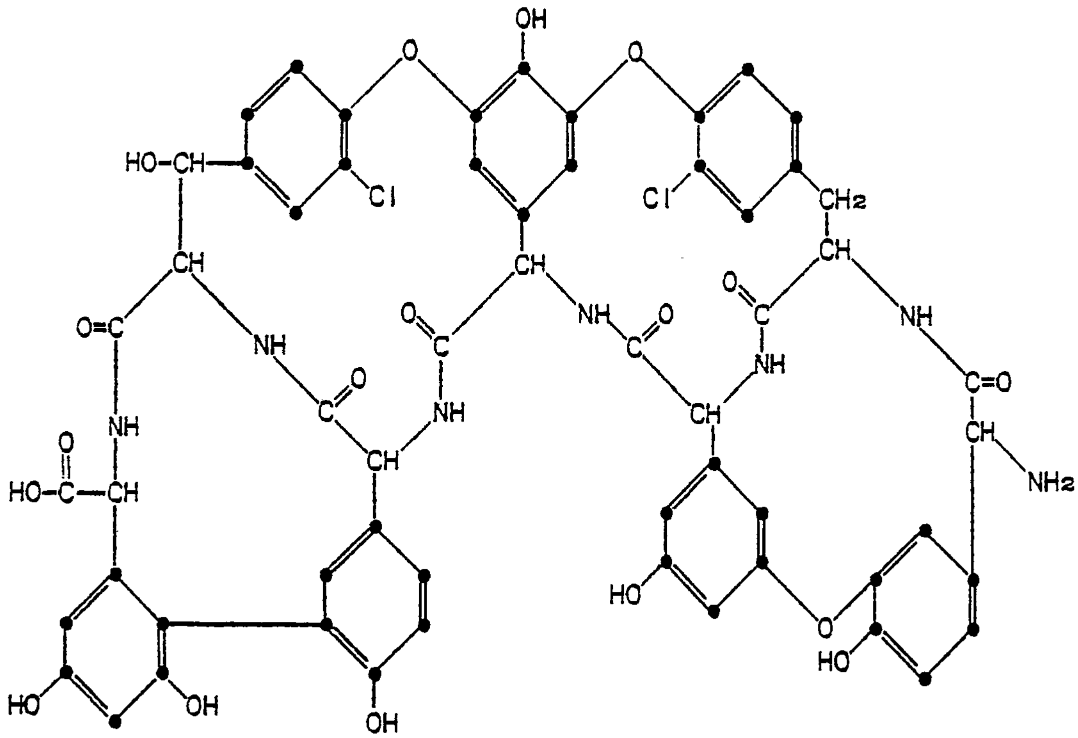

## Example 5

Isolation of A41030 Factor C

A 9.0 g. portion of the A41030 complex was dissolved in 200 ml. of a solvent consisting of water:acetonitrile:sodium chloride (83:17:2 g./L.) and the solution was filtered. The filtrate was applied to an 8 × 100 cm. stainless steel column packed with 4 L. of 10—20 micron LP-1/$C_{18}$ reversed-phase silica gel which was prepared in our laboratories by the special procedure described in Example 3. The column, part of a Chromatospac Prep-100 unit, was eluted at 60 ml./min. with the same solvent combination used for sample dissolution, and 480-ml. fractions were collected. The eluate was monitored at 254 nm using an ISCO Model UA-5 UV detector. Selected fractions were analyzed for the presence of factor C by analytical HPLPLC on an 0.8 × 30 cm. Michel-Miller glass column packed in our laboratories with 25—40 micron LiChroprep RP-8. The solvent, water:acetonitrile:sodium chloride (84:16:2 g./L.), was supplied at 4 ml./min. by an FMI pump. Factor C was detected at 254 nm using an ISCO Model UA-5 UV detector. Fractions 1—27, inclusive, were discarded. Fractions 28—52 inclusive, rich in factor C, were combined and concentrated under reduced pressure to a volume of 500 ml.

Concentrates from two similar purifications were combined, filtered, and applied at 10 ml./min. to 100 ml. of Diaion HP-20 resin in a column (2.8 × 22 cm.), previously equilibrated with water. The column was washed with water (2 L.) until no chloride was detected in the wash by precipitation as silver chloride. Elution was performed with 1 L. of water:acetonitrile (6:4) at 15 ml./min. The eluate was concentrated under reduced pressure and lyophilized to give 2.75 g. of a factor C-enriched mixture of factors. A 1.25 g. portion of this mixture was dissolved in 25 ml. of a solvent consisting of water:acetonitrile:dibutylamine (80:20:0.03M, which solvent had been adjusted to pH 7.8 with phosphoric acid) by addition of tetrabutylammonium hydroxide until solution had been accomplished. The sample was applied to a 2.8 × 59 cm. Michel-Miller glass column packed with 25—40 micron LiChroprep RP-8 and the column was eluted at 4 ml./min., using an FMI pump, with the same solvent combination used for sample dissolution. The eluate was monitored at 254 nm using an ISCO Model UA-5 UV detector. Selected 28-ml. fractions were analyzed for the presence of factor C by analytical HPLC on a 4.6 × 150 mm. stainless steel column packed in our laboratories with 10 micron Nucleosil $C_{18}$ (a commercially available, reversed-phase silica gel, manufactured by Rainin Instrument Co., Inc., Woburn, MA 01801). The sample was applied using a Rheodyne Model 7120 injection valve. The solvent, consisting of water:acetonitrile:sodium acetate (81:19:2 g./L.) adjusted to pH 6 with glacial acetic acid, was supplied at 1 ml./min. by a Milton Roy Duplex Minipump. Factor C was detected at 225 nm using an ISCO Model 1800 variable wavelength UV detector. The portion of the eluate from 4.2—5.1 L., rich in factor C, was concentrated under reduced pressure to a volume of 500 ml.

Concentrates from three similar purifications were combined and dissolved by addition of phosphoric acid to pH 1.7. Sodium chloride (1 mg./ml.) was added as an ionic marker. The sample was applied at 20 ml./min. to 100 ml. of Diaion HP-20 resin in a column (2.8 × 22 cm.), previously equilibrated with water. The

column was washed with aqueous formic acid of pH 2.5 (300 ml.), until no chloride was detected in the wash by precipitation as silver chloride. The column was eluted with 1 L. of water:acetonitrile (6:4) at 30 ml./min. The eluate was collected, concentrated under reduced pressure, and lyophilized to give 0.87 g. of partially purified factor C. This preparation was dissolved in 20 ml. of a solvent consisting of water:acetonitrile:dibutylamine (80:20:0.03M, which solvent had been adjusted to pH 7.8 with phosphoric acid) by addition of tetrabutylammonium hydroxide until solution had occurred. The sample was chromatographed on 25—40 micron LiChroprep RP-8 in a 2.8 × 59 cm. Michel-Miller glass column, as previously described. The portion of the eluate from 2.45—3.20 L. was concentrated under reduced pressure to a volume of 500 ml.

Concentrates from two similar purifications were combined and desalted on a column containing Diaion HP-20 resin in the fashion previously described. The eluate was concentrated under reduced pressure and lyophilized to give 688 mg. of factor C. A 678 mg. portion of this preparation was dissolved in 60 ml. water:acetonitrile (6:4) by heating. The solution was cooled and factor C precipitated upon refrigeration. The precipitate was recovered by filtration, washed with acetone, and dried under vacuum to give 428 mg. of factor C.

Antibiotic A41030 factor C is a white solid having an approximate elemental analysis as follows: 48.87% carbon, 4.39% hydrogen, 6.16% nitrogen, 6.96% chlorine, and 33.81% oxygen. Electrometric titration of factor C in 66% dimethylformamide in water indicated the presence of two titratable groups at $pK_a$ values of about 5.5 and 7.1, respectively, with possible additional $pK_a$'s >10 (initial pH 6.6). An observed molecular weight of about 1393 was obtained using fast atom bombardment mass spectrometry. Based on elemental analysis and the observed molecular weight, an empirical formula of $C_{64}H_{54}Cl_3N_7O_{23}$ is assigned to factor C.

The infrared absorption spectrum of antibiotic A41030 factor C in KBr pellet is shown in the accompanying drawings as Fig. 3. The following distinguishable absorption maxima are observed: 3448—3226 (strong, broad), 1653 (strong), 1610 (medium), 1587 (weak), 1504 (strong), 1481 (weak), 1429 (medium), 1220 (strong), 1136 (strong), 1064 (weak), 1053 (medium), and 1005 (strong) $cm^{-1}$.

The ultraviolet absorption maxima of A41030 factor C in neutral, acidic, and basic methanol:water (1:1) are recorded in Table 5 above.

Antibiotic A41030 factor C is soluble in the same solvents as factor A.

On the basis of the observed physical chemical data, the structure of A41030 factor C is the following:

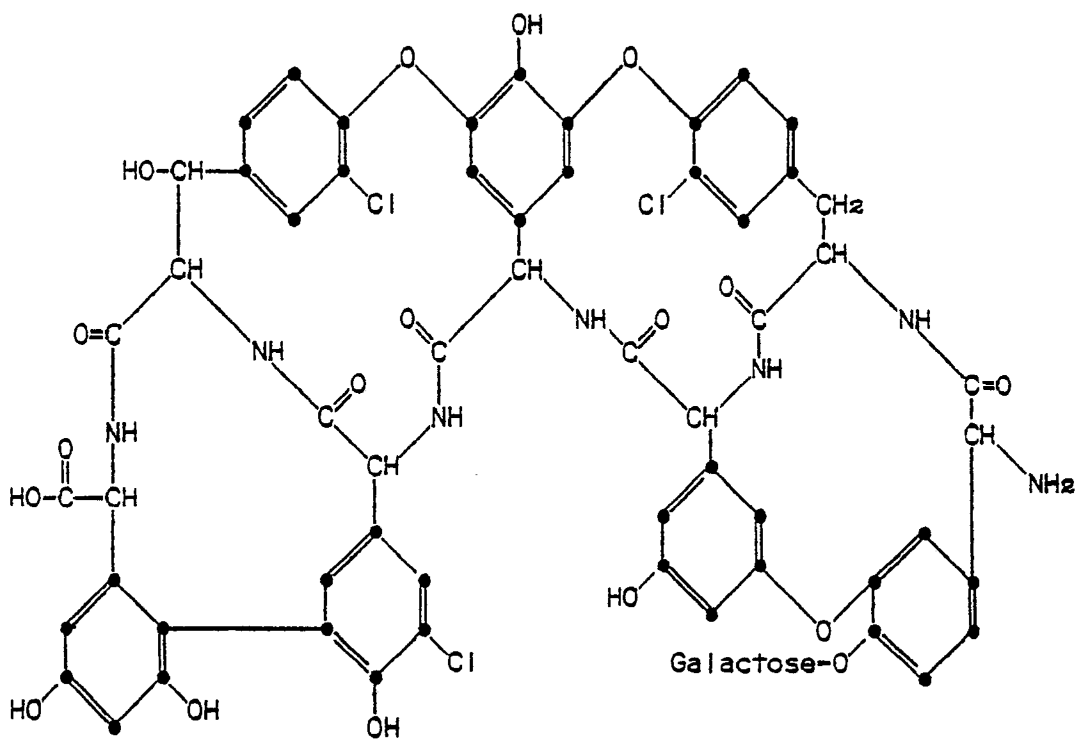

## Example 6

Isolation of A41030 Factor D

A 6.0 g. portion of the A41030 complex was dissolved in 200 ml. of a solvent consisting of water:acetonitrile:sodium chloride (83:17:2 g./L.) and the solution filtered. The filtrate was applied to an 8 × 100 cm. stainless steel column packed with 4 L. of 10—20 micron LP-1/$C_{18}$ reversed-phase silica gel which was prepared in our laboratories by the special procedure described in Example 3. The column, part of a Chromatospac Prep-100 unit, was eluted at 60 ml./min., with the same solvent combination used for

sample dissolution, and 480 ml. fractions were collected. The eluate was monitored at 254 nm using an ISCO Model UA-5 UV detector. Selected fractions were analyzed for the presence of Factor D by analytical HPLPLC on an 0.8 × 30 cm. Michel-Miller glass column packed in our laboratories with 25—40 micron LiChroprep RP-8. The solvent, water:acetonitrile:sodium chloride (84:16:2 g./L.), was supplied at 4 ml./min. using an FMI pump. Factor D was detected at 254 nm using an ISCO Model UA-5 UV detector. Fractions 1—34, inclusive, were discarded. Fractions 35—53, inclusive, rich in factor D, were combined and concentrated under reduced pressure to a volume of about 500 ml.

Concentrates from two similar purifications were combined, diluted to 3 L. with water, and applied at 8—10 ml./min. to 100 ml. of Diaion HP-20 resin in a column (2.8 × 22 cm.), previously equilibrated with water. The column was washed with water (300 ml.) until no chloride was detected in the wash by precipitation as silver chloride. Elution was performed with 1 L. of a solvent consisting of water:acetonitrile (6:4) at 8—10 ml./min. The eluate was concentrated under reduced pressure and lyophilized to give 2.33 g. of a factor D-enriched mixture of factors.

A 1.15 g. portion of this mixture was dissolved in 25 ml. of a solvent consisting of water:acetonitrile:dibutylamine (80:20:0.03M, which solvent had been adjusted to pH 7.8 with phosphoric acid) by addition of tetrabutylammonium hydroxide until solution occurred. The sample was applied to a 2.8 × 59 cm. Michel-Miller glass column packed with 25—40 micron LiChroprep RP-8, and the column was eluted at 5 ml./min., using an FMI pump, with the same solvent combination used for sample dissolution. The eluate was monitored at 254 nm using an ISCO Model UA-5 UV detector. Selected 25 ml. fractions were analyzed for the presence of factor D by analytical HPLC on a 4.6 × 25 mm. stainless steel column packed with 10 micron LiChrosorb RP-18 (a commercially available, reversed-phase silica gel, manufactured by E. Merck, Darmstadt, Germany). The sample was applied using a Rheodyne Model 7120 injection valve. The solvent, consisting of water:acetonitrile:dibutylamine (80:20:0.03M) adjusted to pH 2.5 with phosphoric acid, was supplied at 0.75 ml./min. using a Milton Roy Duplex Minipump. Factor D was detected at 225 nm using an ISCO Model 1800 variable wavelength UV detector. The portion of the eluate from 2.6—3.4 L., rich in factor D, was concentrated under reduced pressure to a volume of 300 ml.

Concentrates from three similar purifications were combined and dissolved by addition of phosphoric acid to pH 7.7. Sodium chloride (1 mg./ml.) was added as an ionic marker. The sample was applied at 20 ml./min. to 100 ml. of Diaion HP-20 resin in a column (2.8 × 22 cm.), previously equilibrated with water. The column was washed with water (300 ml.) adjusted to pH 2.5 with aqueous formic acid, until no chloride was detected in the wash by precipitation as silver chloride. The column was eluted with 1 L. of water:acetonitrile (6:4) at 30 ml./min. The eluate was concentrated under reduced pressure and lyophilized to give 0.63 g. of partially purified factor D. This preparation was dissolved in 15 ml. of a solvent consisting of water:acetonitrile:dibutylamine (80:20:0.03M, which solvent had been adjusted to pH 7.8 with phosphoric acid) by addition of tetrabutylammonium hydroxide until solution occurred. The solution was chromatographed on 25—40 micron LiChroprep RP-8 in a 2.8 × 59 cm. Michel-Miller glass column, in the manner previously described. The portion of the eluate from 2.5—3.0 L. was concentrated under reduced pressure to a volume of about 200 ml. This concentrate was desalted on a column containing Diaion HP-20 resin in the fashion previously described. The eluate was concentrated under reduced pressure and lyophilized to give 193 mg. of partially-purified factor D.

A 259 mg. portion of two combined partially purified factor D preparations was dissolved in 6 ml. of a solvent consisting of water:acetonitrile:dibasic sodium phosphate (82:18:0.03M, which solvent had been adjusted to pH 7.8 with phosphoric acid) and adjusted to pH 10 by addition of aqueous 5N NaOH. The solution was applied to a 2.8 × 59 cm. Michel-Miller glass column packed with 25—40 micron LiChroprep RP-8, and the column was eluted at 4 ml./min., using an FMI pump, with the same solvent combination used for sample dissolution. The eluate was monitored at 254 nm using an ISCO Model UA-5 UV detector. Selected 27 ml. fractions were analyzed for the presence of factor D by analytical HPLC on a 4.6 × 150 mm. stainless steel column packed in our laboratories with 10 micron Nucleosil $C_{18}$. The sample was applied using a Rheodyne Model 7120 injection valve. The same solvent combination used for the preparative elution was supplied at 0.6 ml./min. by a Milton Roy Duplex Minipump. Factor D was detected at 225 nm using an ISCO Model 1800 variable wavelength UV detector. The portion of the eluate from 405—1134 ml. was concentrated under reduced pressure to a volume of 500 ml., and desalted on a column containing Diaion HP-20 resin in the fashion previously described. The eluate was concentrated under reduced pressure and lyophilized to give 120 mg. of factor D.

Antibiotic A41030 factor D is a white, amorphous solid having an approximate elemental analysis as follows: 54.46% carbon, 4.35% hydrogen, 7.58% nitrogen, 4.27% chlorine, and by difference, 29.34% oxygen. Electrometric titration of factor D in 66% dimethylformamide in water indicated the presence of two titratable groups at $pK_a$ values of about 5.5 and 7.6, respectively, with possible additional $pK_a$'s >10 (initial pH 6.83). An observed molecular weight of about 1326 was obtained using fast atom bombardment mass spectrometry.

The infrared absorption spectrum of antibiotic A41030 factor D in KBr pellet is shown in the accompanying drawings as Fig. 4. The following distinguishable absorption maxima are observed: 3448—3226 (strong, broad), 2959 (weak), 1661 (strong), 1592 (strong), 1511 (strong), 1429 (weak), 1290 (weak), 1227 (weak), 1212 (medium), 1163 (weak), 1143 (weak), 1053 (medium), and 1010 (strong) $cm^{-1}$.

The ultraviolet absorption maxima of A41030 factor D in neutral, acidic, and basic methanol:water (1:1) are recorded in Table 5 above.

Antibiotic A41030 factor D is soluble in the same solvents as factor A.

On the basis of the observed physical chemical data, the structure of A41030 factor D is believed to be as follows:

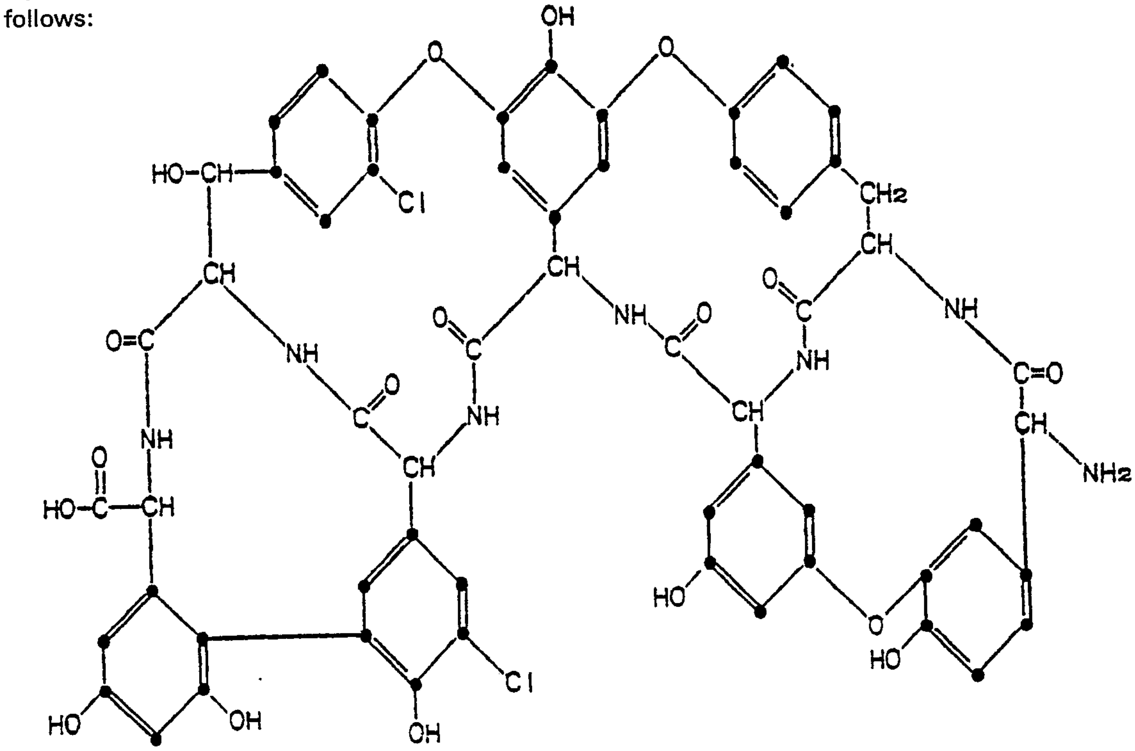

plus one or more *n*-butyl groups.

Example 7

Isolation of A41030 Factor E

A 0.3 g. portion of the A41030 complex was dissolved in 30 ml. of a solvent consisting of water:acetonitrile:sodium chloride (85:15:2 g./L.), and applied to a 2.8 × 59 cm. Michel-Miller glass column packed in our laboratories with 25—40 micron LiChroprep RP—8. An FMI pump was used to elute the column at 12 ml./min. (85 psi), with the same solvent combination used for sample dissolution, collecting 24 ml. fractions. The eluate was monitored at 254 nm using an ISCO Model UA—5 UV detector. Fractions 1—54, inclusive, were discarded. Fractions 55—122, inclusive, rich in factor E, were combined and concentrated under reduced pressure to a volume of 50 ml.

Concentrates from 13 similar purifications were combined, diluted to 1.5 L. with water, and applied at 5 ml./min. to 100 ml. of Diaion HP—20 resin in a column (2.8 × 22 cm.), previously equilibrated with water. The column was washed with water (900 ml.) until no chloride was detected in the wash by precipitation as silver chloride. Elution was then performed with water:methanol (1:1) at 10 ml./min., collecting 300 ml. fractions. Fractions were analyzed for activity against *B. subtilis*. Fractions 1—8, inclusive, were combined, concentrated under reduced pressure, and lyophilized to give 1.04 g. of a factor E-enriched mixture of factors. A 0.5 g. portion of this mixture was dissolved in 10 ml. of a solvent consisting of water:acetonitrile:sodium chloride (84:14:2 g./L.), and applied to a 2.8 × 59 cm. Michel-Miller glass column packed with 25—40 micron LiChroprep RP—8. An FMI pump was used to elute the column at 5 ml./min., with the same solvent combination used for sample dissolution, and 25 ml. fractions were collected. The eluate was monitored at 254 nm using an ISCO Model UA—5 UV detector. Selected fractions were analyzed for the presence of factor E by analytical HPLC on a 4.6 × 150 mm. stainless steel column packed in our laboratories with 5 micron ODS-Hyperspheres (Shandon Southern Products, Ltd., Cheshire, England). The sample was applied using a Rheodyne Model 7120 injection valve. The solvent, consisting of water:acetonitrile:sodium acetate (81:19:2 g./L.) adjusted to pH 6 with glacial acetic acid, was supplied at 0.65 ml./min. by a Milton Roy Duplex Minipump. Factor E was detected at 225 nm using an ISCO Model 1800 variable wavelength UV detector. The portion of the eluate from 1520—1780 ml. was concentrated under reduced pressure to a volume of 50 ml.

Concentrates from three similar purifications were combined, diluted to 1 L. with water, and applied at 10 ml./min. to 100 ml. of Diaion HP—20 resin in a column (2.8 × 22 cm.), previously equilibrated with water. The column was washed with water (200 ml.) adjusted with aqueous formic acid to pH 2.5, until no chloride was detected in the wash by precipitation as silver chloride. Elution was performed with 0.5 L. of water:acetonitrile (6:4) at 15 ml./min. The eluate was concentrated under reduced pressure and lyophilized

to give 202.2 mg. of partially purified factor E. This preparation was dissolved in 4 ml. of a solvent consisting of water:acetonitrile:sodium chloride (86:14:2 g./L.) and chromatographed at 4 ml./min. on a 2.8 × 59 cm. Michel-Miller glass column, packed with 25—40 micron LiChroprep RP—8, as previously described. The portion of the eluate from 2060—2480 ml., rich in factor E, was concentrated under reduced pressure to a volume of 50 ml. Concentrates from three similar purifications were combined and desalted on 100 ml. of Diaion HP—20 resin in a column, as previously described. The eluate was concentrated under reduced pressure and lyophilized to give 242 mg. of factor E.

Antibiotic A41030 factor E is a white solid having an approximate elemental analysis as follows: 56.06% carbon, 4.06% hydrogen, 8.53% nitrogen, 3.50% chlorine, and by difference, 27.85% oxygen. Electrometric titration of factor E in 66% dimethylformamide in water indicated the presence of two titratable groups at $pK_a$ values of about 5.8 and 7.7, respectively, with possible additional $pK_a$'s >10 (initial pH 6.57). An observed molecular weight of about 1163 was obtained using fast atom bombardment mass spectrometry. A tentative empirical formula of $C_{58}H_{46}ClN_7O_{18}$ is assigned to factor E.

The infrared absorption spectrum of antibiotic A41030 factor E in KBr pellet is shown in the accompanying drawings as Fig. 5. The following distinguishable absorption maxima are observed: 3448—3226 (strong, broad), 1653 (strong), 1600 (medium), 1504 (strong), 1429 (weak), 1290 (weak), 1198 (medium), 1136 (weak), 1064 (weak), and 1010 (strong) $cm^{-1}$.

The ultraviolet absorption maxima of A41030 factor E in neutral, acidic, and basic methanol:water (1:1) are recorded in Table 5 above.

Antibiotic A41030 factor E is soluble in the same solvents as factor A.

On the basis of the observed physical chemical data, the following structure has been assigned to A41030 factor E:

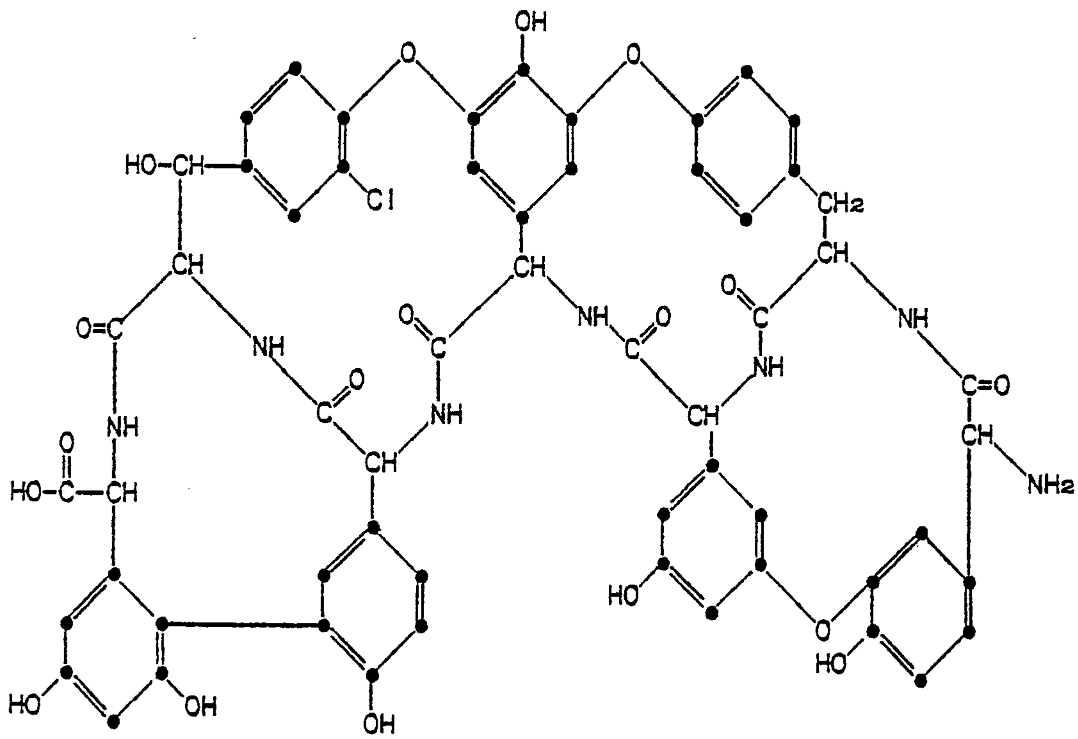

Example 8

Isolation of A41030 Factor F

A 9.0 g. portion of the A41030 complex was dissolved in 200 ml. of a solvent consisting of water:acetonitrile:sodium chloride (83:17:2 g./L.) and the solution was filtered. The filtrate was applied to an 8 × 100 cm. stainless steel column packed with 4 L. of 10—20 micron LP—1/$C_{18}$ reversed-phase silica gel which was prepared in our laboratories by the special procedure described in Example 3. The column, part of a Chromatospac Prep-100 unit, was eluted at 60 ml./min., with the same solvent combination used for sample dissolution, and 480 ml. fractions were collected. The eluate was monitored at 254 nm using an ISCO Model UA—5 UV detector. Selected fractions were analyzed for the presence of factor F by analytical HPLPLC on an 0.8 × 30 cm. Michel-Miller glass column packed in our laboratories with 25—40 micron LiChroprep RP—8. The solvent, water:acetonitrile:sodium chloride (84:16:2 g./L.), was supplied at 4 ml./min. by an FMI pump. Factor F was detected at 254 nm using an ISCO Model UA—5 UV detector. Fractions 1—25, inclusive, were discarded. Fractions 26—36, inclusive, rich in factor F, were combined and concentrated under reduced pressure to a volume of about 500 ml.

Concentrates from three similar purifications were combined, filtered, and the filtrate applied at 10 ml./min. to 100 ml. of Diaion HP—20 resin in a column (2.8 × 22 cm.), previously equilibrated with water. The column was washed with water (900 ml.) until no chloride was detected in the wash by precipitation as silver chloride. Elution was performed with 1 L. of water:acetonitrile (6:4) at 15 ml./min. The eluate was concentrated under reduced pressure and lyophilized to give 2.6 g. of partially purified factor F. A 500 mg. portion of this preparation was dissolved in 10 ml. of a solvent consisting of water:acetonitrile:sodium chloride (84:16:2 g./L.), by adjustment to pH 7.0 with aqueous sodium hydroxide. The solution was applied to a 4.7 × 45 cm. Michel-Miller glass column packed in our laboratories with 25—40 micron LiChroprep RP—18. An FMI pump was used to elute the column at 6 ml./min., with the same solvent combination used for sample dissolution, and 24 ml. fractions were collected. The eluate was monitored at 254 nm using an ISCO Model UA—5 UV detector. Selected fractions were analyzed for the presence of factor F, using the analytical HPLPLC system previously described. The portion of the eluate from 1940—2520 ml., rich in factor F was concentrated under reduced pressure to a volume of about 300 ml.

Concentrates from two similar purifications were combined and applied at 10 ml./min. to 100 ml. of Diaion HP—20 resin in a column (2.8 × 22 cm.), previously equilibrated with water. The column was washed with water (300 ml.) adjusted to pH 2.5 with formic acid until no chloride was detected in the wash by precipitation as silver chloride. Elution was performed with 0.75 L. of water:acetonitrile (6:4). The eluate was concentrated under reduced pressure and lyophilized to give 299 mg. of factor F.

Antibiotic A41030 factor F is a white solid having an approximate elemental analysis as follows: 51.39% carbon, 3.96% hydrogen, 6.45% chlorine, 6.45% nitrogen, and 28.65% oxygen. Electrometric titration of factor F in 66% dimethylformamide in water indicated the presence of two titratable groups at $pK_a$ values of about 5.4 and 7.1, respectively, with possible additional $pK_a$'s >10 (initial pH 5.93). An observed molecular weight of about 1555 was obtained using fast atom bombardment mass spectrometry. A tentative empirical formula of $C_{70}H_{64}Cl_3N_7O_{28}$ is assigned to factor F.

The molecular weight data suggest that factor F differs from factor A by the addition of two sugar moieties, and an uneven molecular weight indicates there is no amino sugar present.

The infrared absorption spectrum of antibiotic A41030 factor F in KBr pellet is shown in the accompanying drawings as Fig. 6. The following distinguishable absorption maxima are observed: 3448—3226 (strong, broad), 1653 (strong), 1600 (medium), 1504 (strong), 1429 (weak), 1258 (weak), 1227 (strong), 1136 (strong), 1075 (strong), 1053 (strong), and 1010 (strong) $cm^{-1}$.

The ultraviolet absorption maxima of A41030 factor F in neutral, acidic, and basic methanol:water (1:1) are recorded in Table 5 above.

Antibiotic A41030 factor F is soluble in the same solvents as factor A.

On the basis of the observed physical chemical data, the following structure has been assigned to A41030 Factor F:

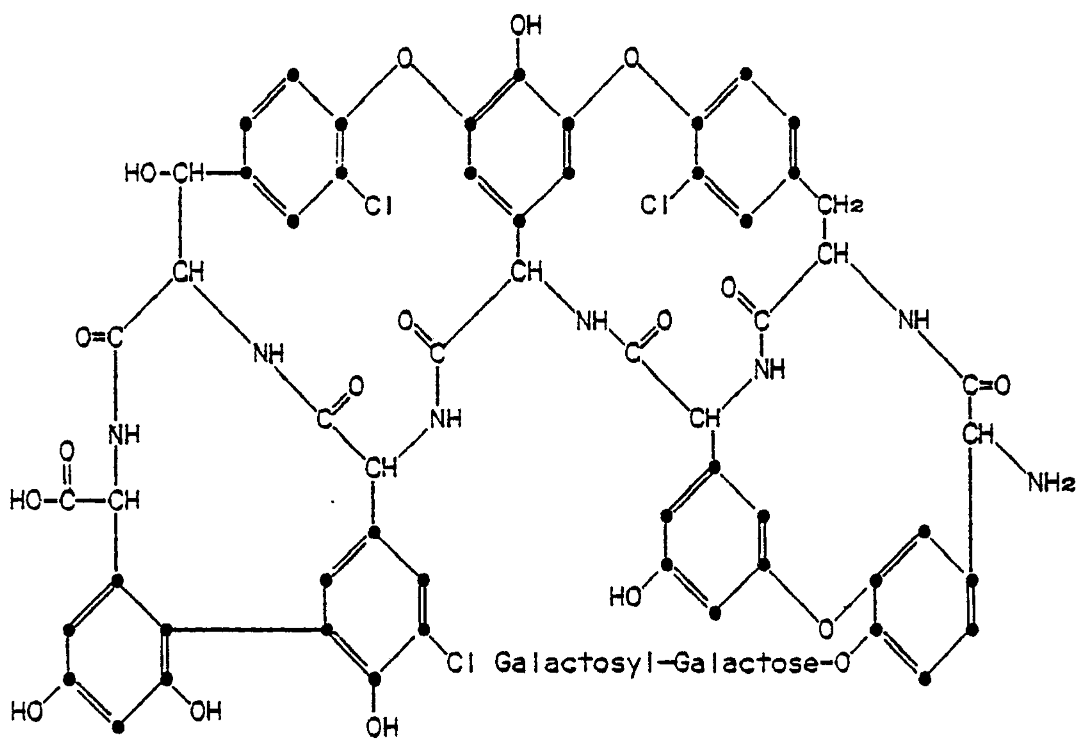

Example 9

Isolation of A41030 Factor G

An 8 g. portion of the A41030 complex from Example 2 was dissolved in 200 ml. of a solvent consisting of water:acetonitrile:sodium chloride (84:16:2 g./L.) and filtered. The filtrate was applied to a stainless steel column (8 × 100 cm.) packed with 4 L. of 10—20 micron LP—1/$C_{18}$ reversed-phase silica gel which was prepared in our laboratories by the special procedure described in Example 3. The column was part of a Chromatospac Prep-100 unit (see Example 3). The column was eluted at 60 ml./min. with water:acetonitrile:sodium chloride (84:16:2 g./L.), collecting 480 ml. fractions. The eluate was monitored at 254 nm as described in Example 3. Selected fractions were analyzed for the presence of factor G by an analytical high performance liquid chromatography (HPLC) procedure described in preceding Examples.

Fractions 22—35, inclusive, rich in factor G, were combined and concentrated under reduced pressure to a volume of 500 ml. Concentrates from three similar purifications were combined, adjusted to pH 8.5 with aqueous sodium hydroxide, and filtered. The filtrate was applied at 10 ml./min. to 100 ml. of Diaion HP—20 resin in a column (2.8 × 22 cm.), previously equilibrated with water. The column was washed with water (400 ml. adjusted to pH 2.5 with formic acid) until no chloride was detected in the wash by precipitation as silver chloride. Elution was performed with water:acetonitrile (6:4) at 15 ml./min., collecting 1 L. fractions. Fractions were analyzed for activity against *B. subtilis.* The active fractions were combined, concentrated under reduced pressure, and lyophilized to give 2.85 g. of material.

A 0.5 g. portion of this material was dissolved in 10 ml. of a solvent consisting of water:acetonitrile:dibutylamine (80:20:0.03M, which solvent had been adjusted to pH 7.8 with phosphoric acid) by addition of dibutylamine until solution had been accomplished (final pH 8.2). The solution was applied to a 2.8 × 59 cm. Michel-Miller HPLPLC glass column packed with 25—40 micron LiChroprep RP—8 (from MC/B Manufacturing Chemist, Inc., Cincinnati, OH).

Using an FMI pump, the column was eluted at 4 ml./min. with the same solvent combination used for sample dissolution. The eluate was monitored at 254 nm using an ISCO Model UA—5 UV detector. Selected 10 ml. fractions were analyzed for the presence of factor G by the analytical HPLC procedure described in preceding Examples.

Fractions 54—74, inclusive, rich in factor G, were combined with fractions from two similar purifications and applied at 10 ml./min. to 100 ml. of Diaion HP—20 resin in a column (2.8 × 22 cm.), previously equilibrated with water. The column was washed with water (300 ml.) adjusted to pH 2.5 with formic acid, until no chloride was detected in the wash by precipitation as silver chloride. Elution was performed with 0.75 L. of water:acetonitrile (6:4). The eluate was concentrated under reduced pressure and lyophilized to give 960 mg. of factor G.

Antibiotic A41030 factor G is a white solid having an approximate elemental analysis as follows: 50.02% carbon, 4.61% hydrogen, 4.74% chlorine, 6.11% nitrogen, and 30.70% oxygen. Electrometric titration of factor G in 66% dimethylformamide in water indicated the presence of titratable groups at $pK_a$ values of about 5.4 and 7.0, respectively, with possible additional $pK_a$'s $>10.5$ (initial pH 6.32). An observed molecular weight of about 1684 was obtained using fast atom bombardment mass spectrometry.

The infrared absorption spectrum of antibiotic A41030 factor G in KBr pellet is shown in the accompanying drawings as Fig. 7. The following distinguishable absorption maxima are observed: 3320 (very broad, strong), 2975 (sharp, weak), 2920 (sharp, weak), 1659 (normal, strong), 1594 (broad, strong), 1512 (sharp, strong), 1492 (shoulder), 1430 (sharp, weak), 1386 (broad, weak), 1337 (broad, weak), 1308 (sharp, weak), 1264 (sharp, weak), 1230 (broad, medium), 1145 (broad, medium), 1077 (sharp, medium), 1062 (sharp, medium), 1014 (sharp, medium), and 846 (broad, medium) $cm^{-1}$.

The ultraviolet absorption maxima of A41030 factor G in neutral, acidic, and basic methanol:water (1:1) are recorded in Table 5 above.

Antibiotic A41030 factor G is soluble in the same solvents as factor A.

Factors A, B, C, D, E, F and G of the A41030 complex can be separated and distinguished from one another by employing silica-gel thin-layer chromatography (TLC) and paper chromatography. *Bacillus subtilis* was the organism used for the bioautography. The ratio of movement (Rx) expressed relative to that of A41030 factor A, which was given a value of 1.00, is set forth in Table 6.

TABLE 6

| | Rx | |
|---|---|---|
| | Solvent System | |
| Factor | A | B |
| A | 1.00 | 1.00 |
| B | 0.76 | 0.75 |
| C | 0.68 | 0.44 |
| D | 0.65 | 0.91 |
| E | 0.49 | 0.63 |
| F | 0.21 | 0.25 |
| G | 0.21 | 0.25 |

System A
Paper: Whatman No. 1 (untreated).
Solvent: *n*-Butanol saturated with water:methanol (1:1).

System B
Sorbent: Merck-Darmstadt-Silica Gel 60.
Solvent: Acetonitrile:ethanol:water (8:1:1.5).

The high performance liquid chromatography (HPLC) retention times of A41030 factors A through G, inclusive, were determined using a stainless steel column having 10 micron LiChrosorb RP—18 as the packing, with a solvent consisting of water:acetonitrile:dibutylamine (82:18:0.03M) adjusted to pH 2.5 with phosphoric acid. The solvent was applied at a flow rate of 0.75 ml./min. The eluate was monitored by UV absorption at 225 nm. The relative retention values, which are the ratio of the retention time for each factor relative to that of A41030 factor A, are set forth in Table 7.

TABLE 7

| Factor | Cm. | Min. | Relative Retention |
|---|---|---|---|
| A | 6.4 | 19.2 | 1.00 |
| B | 4.1 | 12.3 | 0.64 |
| C | 5.4 | 16.2 | 0.84 |
| D | 3.8 | 11.4 | 0.59 |
| E | 2.7 | 8.1 | 0.42 |
| F | 4.5 | 13.5 | 0.70 |
| G | 4.5 | 13.5 | 0.70 |

Since the several factors of antibiotic A41030 are amphoteric, containing both an amino group and a carboxylic acid function, they are capable of forming salts with suitable acids and bases. The pharmaceutically acceptable salts so formed are also part of this invention. "Pharmaceutically-acceptable" salts are salts which are useful in the chemotherapy of warm-blooded animals. Representative and suitable salts of A41030 factors A, B, C, D, E, F and G include those acid addition salts formed by standard reaction with both organic and inorganic acids such as, for example, sulfuric, phosphoric, hydrochloric, acetic, succinic, citric, lactic, maleic, fumaric, palmitic, cholic, pamoic, mucic, D-glutamic, d-camphoric, glutaric, glycolic, phthalic, tartaric, lauric, stearic, salicyclic, methanesulfonic, benzenesulfonic, sorbic, picric, benzoic, cinnamic and like acids, as well as salts formed with the carboxylic acid function with such bases as sodium hydroxide, sodium carbonate, potassium carbonate, calcium hydroxide, potassium hydroxide, trimethylamine, ammonium hydroxide, diethanolamine, and like bases.

The antibiotic A41030 complex and its factors are active against gram positive microorganisms, including *Staphylococcus* and *Streptococcus* species. These antibiotics also show activity for growth promotion and improving feed efficiency in poultry, swine, and cattle.

The activity of the A41030 complex and the individual factors has been demonstrated by a number of tests which are described below.

Example 10

Sample Preparation for Biological Assay and Quantitative Analysis of A41030 Factor A in Dried Whole Broth

One liter of whole broth was concentrated to a volume of 200 ml. and lyophilized to give 31.5 g. of dried whole broth. A 400 mg. sample of the dried whole broth was extracted 3 times with 10 ml. portions of water at pH 8.5. The extracts were combined, concentrated to a volume of 10 ml., and portions of this concentrate used for biological assay. The turbidimetric assay was conducted on a semiautomated system (Autoturb® microbiological assay system, Elanco) described by N. R. Kuzel and F. W. Kavanaugh in *J. Pharmaceut. Sci. 60(5)*, 764 and 767 (1971). In testing the A41030 complex, the following test parameters were used: *Staphylococcus aureus* ATCC 9144 in a nutrient broth medium (pH 7), incubated for four hours at 37°C. Test samples and standard were dissolved in methanol:water (1:1). The standard, A41030 factor A, was presented to the Autoturb® carousel at concentrations of 0.4, 0.6, 0.9, 1.2, and 1.5 mcg./ml.

One milliliter of the above concentrate was purified by the following procedure to be used for analysis by HPLC.

(a) One C—18 SEP—PAK® cartridge (Silica gel cartridge, Waters Associates, Inc., Milford, Mass.) was washed with 10 ml. of methanol, using a 10 ml. syringe with a Luer fitting, as known to the art.

(b) Wash the same cartridge with 10 ml. of water.

(c) Apply 1 ml. of the concentrate from above to the cartridge at the rate of approximately 1 ml./min.

(d) Wash the cartridge with 1 ml. of water and blow the cartridge dry.

(e) Elute the cartridge with 1 ml. of a solution of tetrahydrofuran:water (1:1) at about 0.5 ml./min.

(f) Remove the tetrahydrofuran from the eluate *in vacuo*, or alternatively, under a nitrogen stream, and reconstitute the eluate to a volume of 1 ml. with water.

(g) Analyze the solution of HPLC procedure as described hereinbefore.

The results of the assay for biological activity and the HPLC analysis of the whole broth are recorded in Table 8.

TABLE 8

Biological Activity and HPLC Analysis of A41030A in Whole Broth

| Sample No. | Wt. | Concentr. of A41030A+ | Total Wt. of Activity* | Total Wt. of A41030A | % of A41030A+ |
|---|---|---|---|---|---|
| 1 | 106.8 kg | 4.42 mg/g | 491 g | 472 g | 96.1 |
| 2 | 146.5 kg | 10.8 mg/g | 1685 g | 1582 g | 93.9 |

*Total biological activity comprised of A41030 factors A, B, C, D, E, F and G.

+As determined by HPLC.

AGAR-DILUTION ASSAY PROCEDURE

The agar-dilution procedure described by the International Collaborative study (ICS) group was used to determine MIC values.

The results obtained from tests of antibiotic A41030 factor A in the agar dilution assay procedure are given in Table 9, which follows.

TABLE 9

ACTIVITY OF A41030 FACTOR A

| Test Organism | (µg./ml.) |
|---|---|
| *Staphylococcus aureus* 3055* | <0.5 |
| *Staphylococcus aureus* 3074** | <0.5 |
| *Streptococcus faecalis* X66 | <0.5 |

*benzylpenicillin-susceptible

**benzylpenicillin-resistant

DISC-PLATE SENSITIVITY PROCEDURE

Agar plates, inoculated with the test organism, were used; 6 mm. discs (0.02 ml. capacity) were saturated from log 2 dilutions of the antibiotic solution. Disc content was ⅕ or ⅟₅₀th of the concentration of the solution used, i.e., disc content of 100 mg. or 10 mg. obtained from a solution of 500 mg./ml. concentration. The size of the zone of inhibition produced by the A41030 factor A antibiotic for each disc content is reported in Table 10.

TABLE 10

ACTIVITY OF A41030 FACTOR A

| Test Organism | Zone diameter (mm) (at µg./disc) 100 | 10 |
|---|---|---|
| *Staphylococcus aureus* 3055* | 20.8 | 17.2 |
| *Staphylococcus aureus* 3074** | 19.8 | 16.7 |
| *Staphylococcus aureus* 3130*** | 21.4 | 17.4 |
| *Streptococcus pyogenes* C203 | 15.0 | 12.0 |
| *Streptococcus sp.* (Group D) 9960 | 20.5 | 16.4 |
| *Streptococcus pneumoniae* Park I | 17.0 | 15.0 |
| *Escherichia coli* EC14 | 8.0 | 0 |

*benzylpenicillin-susceptible

**benzylpenicillin-resistant

***benzylpenicillin-resistant, methicillin-resistant

Antibiotic A41030 factor A has shown activity against a number of strains of *Hemophilus influenzae*, as determined by the agar-dilution method. The results of the tests are recorded in Table 11.

TABLE 11

ACTIVITY OF A41030 FACTOR A AGAINST *HEMOPHILUS INFLUENZAE* STRAINS

| *Hemophilus influenzae* | MIC (μg./ml.) |
|---|---|
| R251 | 16 |
| R259 | 16 |
| R272 | 16 |
| R274 | 16 |
| 4842 | 8 |
| 75-90383 | 16 |
| P. Wylie | 16 |
| G. Newton | 32 |
| Bruno | 16 |
| 75-19300 | 16 |
| S. Ford | 16 |
| A. Hall | 16 |
| C. Steele | 16 |
| Miller | 16 |
| Howard | 16 |
| 75-312 | 8 |
| 75-313 | 16 |
| 75-364 | 16 |
| 75-465 | 32 |

Antibiotic A41030 factor A is active against *Neisseria sp.*, as determined by the agar-dilution method. The test results are recorded in Table 12.

TABLE 12

ACTIVITY OF A41030 FACTOR A AGAINST *NEISSERIA SP.*

| *Neisseria sp.* | MIC (μg./ml.) |
|---|---|
| L. Nance | 4.0 |
| Woods | 4.0 |
| Schultz | 8.0 |
| Mitchell | 4.0 |
| Sanders | 1.0 |

The activity of antibiotic A41030 factor A against a variety of bacteria, as determined by the agar-dilution method, is reported in Table 13.

TABLE 13

ACTIVITY OF A41030 FACTOR A
AGAINST A VARIETY OF BACTERIA

| Bacteria | MIC (μg./ml.) |
|---|---|
| *Staphylococcus aureus* | |
| 3055 | 0.13 |
| 3074* | ≤ 0.06 |
| 3131** | ≤ 0.06 |
| 3134** | ≤ 0.06 |
| H43* | ≤ 0.06 |
| V57* | ≤ 0.06 |
| H290 | ≤ 0.06 |
| *Streptococcus pyogenes* | |
| C203 | 0.25 |
| 9943 | 0.25 |
| 10389 | 0.25 |
| 12344 | 0.5 |
| M-6517 | 0.25 |
| *Streptococcus pneumoniae* | |
| Park I | 0.25 |
| Type 14 | 0.25 |
| 2764 | 0.25 |
| 6301 | 0.25 |
| BI-343 | 0.25 |
| *Staphylococcus epidermidis* | |
| Litton | 0.13 |
| Mencher | ≤ 0.06 |
| Britton | 0.13 |
| Mobley | ≤ 0.06 |
| *Viridans streptococcus* | |
| SM-1134 | 0.5 |
| SSI-910 | 0.25 |
| SSII-895 | 0.25 |
| *Streptococcus sp.* (Group D) | |
| 238 | 0.25 |
| 282 | 0.25 |
| 9901 | 0.25 |
| 12253F | 0.25 |
| Guze | 0.25 |

| *Shigella flexneri* | |
|---|---|
| SH-3 | 128 |
| SH-4 | 64 |

*Penicillin-resistant

**Penicillin, methicillin, and erythromycin resistant

The A41030 antibiotics, factors A, B and C, are active against a genus of anaerobic bacteria identified as *Bacteroides sp.*, the 24-hour MIC values being determined by the agar-dilution method, and are set forth in Table 14.

TABLE 14

ACTIVITY OF A41030 FACTORS AGAINST *BACTEROIDES SP.*

| Test Organism | MIC (µg./ml.) A | B | C |
|---|---|---|---|
| *B. fragilis* 1877 | 32 | 32 | 32 |
| *B. fragilis* 103 | 32 | 32 | 32 |
| *B. fragilis* 104 | 32 | 32 | 32 |
| *B. fragilis* 106 | 64 | 32 | 32 |
| *B. fragilis* 107 | 32 | 32 | 32 |
| *B. fragilis* 108 | 32 | 32 | 64 |
| *B. fragilis* 110 | 64 | 64 | 64 |
| *B. fragilis* 111 | 32 | 32 | 32 |
| *B. fragilis* 112 | 64 | 32 | 32 |
| *B. fragilis* 113 | 32 | 32 | 32 |
| *B. fragilis* 1451 | 64 | 64 | 64 |
| *B. fragilis* 1470 | 64 | 64 | 64 |
| *B. fragilis* 2 | 64 | 32 | 32 |
| *B. fragilis* 9 | 64 | 32 | 32 |
| *B. fragilis* 9032 | 64 | 32 | 32 |
| *B. corrodens* 1874 | 32 | 32 | 32 |
| *B. vulgatis* 1563 | 32 | 32 | 32 |
| *B. thetaiotaomicron* 1438 | 64 | 32 | 32 |
| *B. thetaiotaomicron* 1900A | 128 | 128 | 128 |

Antibiotic A41030 factors A, B and C have also been tested and found to be active against a genus of anaerobic bacteria identified as *Propionibacterium acnes.* The MIC values were determined by the 24-hour agar-dilution method, and are set forth in Table 15.

TABLE 15

ACTIVITY OF A41030 FACTORS AGAINST *PROPIONIBACTERIUM ACNES*

| Strain of *P. acnes* | MIC (µg./ml.) A | B | C |
|---|---|---|---|
| 44 | 0.125 | 0.06 | 0.125 |
| 79 | 0.125 | 0.06 | 0.125 |
| 101 | 0.125 | 0.06 | 0.125 |
| 103 | 0.125 | 0.06 | 0.125 |
| 104 | 0.25 | 0.25 | 0.25 |
| 105 | 0.125 | 0.06 | 0.125 |
| 106 | 0.125 | 0.06 | 0.125 |
| 107 | 0.06 | 0.06 | 0.125 |
| 5292 | 0.06 | 0.06 | 0.06 |
| 5170 | ≤0.03 | ≤0.03 | ≤0.03 |
| 5176 | ≤0.03 | 0.06 | ≤0.03 |
| 5187 | ≤0.03 | 0.06 | 0.06 |
| 5191 | 0.125 | 0.06 | 0.125 |
| 5197 | ≤0.03 | 0.06 | ≤0.03 |
| 5226 | 0.5 | 0.5 | 0.125 |
| 5227 | ≤0.03 | 0.06 | 0.06 |
| 5228 | 1.0 | 0.5 | 1.0 |
| 5229 | 0.5 | 0.25 | 0.5 |
| 5246 | 0.06 | 0.125 | 0.06 |

Antibiotic A41030 factors A, B, C, D, E, F and G have been tested and found to be active against a number of anaerobic bacteria, as recorded in Table 16; the MIC values were determined by the agar-dilution method.

TABLE 16

ACTIVITY OF A41030 FACTORS AGAINST ANAEROBIC BACTERIA

| Test Organism | MIC (µg./ml.) A | B | C | D | E | F | G |
|---|---|---|---|---|---|---|---|
| *Clostridium difficile* 2994 | 32 | 32 | 16 | 0.5 | 0.5 | 1.0 | 2 |
| *Clostridium perfringens* 81 | 0.5 | 0.5 | 1.0 | 0.5 | 1.0 | 1.0 | 2 |
| *Clostridium septicum* 1128 | 2 | 4 | 8 | 0.25 | 0.5 | 1.0 | 2 |
| *Eubacterium aerofaciens* 1235 | > 128 | > 128 | > 128 | 0.5 | 0.5 | 1.0 | 2 |
| *Peptococcus asaccharolyticus* 1302 | ≤ 0.125 | ≤ 0.25 | ≤ 0.25 | ≤ 0.125 | ≤ 0.25 | ≤ 0.25 | 1.0 |
| *Peptococcus prevoti* 1281 | ≤ 0.25 | ≤ 0.25 | ≤ 0.25 | 0.25 | 32 | 32 | 2 |
| *Peptostreptococcus anaerobius* 1428 | ≤ 0.25 | ≤ 0.25 | ≤ 0.25 | 0.5 | 32 | 32 | ≤ 0.5 |
| *Peptostreptococcus intermedius* 1264 | 1.0 | 0.5 | 0.5 | 1.0 | 32 | 1.0 | 2 |
| *Propionibacterium acnes* 79 | ≤ 0.25 | 16 | ≤ 0.25 | 0.25 | 0.5 | 1.0 | 1.0 |
| *Bacteroides fragilis* 111 | 128 | 64 | > 128 | 32 | 64 | 32 | 32 |
| *Bacteroides fragilis* 1877 | 32 | 32 | 16 | 32 | 32 | 32 | 32 |
| *Bacteroides fragilis* 1936B | 64 | 32 | 32 | 32 | 64 | 32 | 64 |
| *Bacteroides thetaiotaomicron* 1438 | 64 | 32 | 64 | 32 | 64 | 64 | 64 |
| *Bacteroides melaninogenicus* 1856/28 | > 128 | > 128 | > 128 | >64 | >128 | > 128 | >128 |
| *Bacteroides melaninogenicus* 2736 | 4 | 4 | 4 | 0.5 | 32 | 1.0 | 64 |
| *Bacteroides vulgatis* 1211 | 32 | 32 | 32 | 32 | 32 | 32 | 64 |
| *Bacteroides corrodens* 1874 | 64 | 64 | 32 | 32 | 64 | 32 | 32 |
| *Fusobacterium symbiosum* 1470 | 1.0 | 1.0 | 1.0 | 0.5 | 1.0 | 1.0 | 2 |
| *Fusobacterium necrophorum* 6054A | 8 | 8 | 16 | ≤ 0.125 | 0.5 | 1.0 | 2 |

Antibiotic A41030 factors A, B and C have shown activity against a number of species of two genera of anaerobic cocci identified as *Peptococcus* and *Peptostreptococcus,* respectively. The MIC values were determined by the agar-dilution method, and are recorded in Table 17.

TABLE 17

ACTIVITY OF A41030 FACTORS vs.
*PEPTOCOCCUS* AND *PEPTOSTREPTOCOCCUS SPECIES*

| | MIC (µg./ml.) | | |
|---|---|---|---|
| Test Organism | A | B | C |
| *Pc.*[1] *asaccharolyticus* 1302 | ≤ 0.03 | 0.125 | 0.125 |
| *Pc. asaccharolyticus* 1344 | 0.5 | 0.5 | 1.0 |
| *Pc. constellatus* 1468 | 0.5 | 0.5 | 0.5 |
| *Pc. magnus* 1401 | 0.125 | 0.125 | 0.25 |
| *Pc. magnus* 1421 | 0.125 | 0.125 | 0.25 |
| *Pc. magnus* 1477 | 0.5 | 0.25 | 0.5 |
| *Pc. prevoti* 1281 | 0.5 | 0.5 | 1.0 |
| *Pc. prevoti* 1293 | 1.0 | 0.25 | 0.5 |
| *Pc. prevoti* 1407 | 1.0 | 0.5 | 1.0 |
| *Ps.*[2] *anaerobius* 8 | 0.5 | 0.25 | 0.5 |
| *Ps. anaerobius* 52 | 0.5 | 0.5 | 0.25 |
| *Ps. anaerobius* 59 | 0.5 | 0.25 | 0.25 |
| *Ps. anaerobius* 1418 | ≤ 0.03 | 0.06 | ≤ 0.03 |
| *Ps. anaerobius* 1451 | 0.5 | 0.25 | 0.25 |
| *Ps. anaerobius* 1428 | 0.5 | 0.25 | 0.25 |
| *Ps. anaerobius* 1477 | 0.5 | 0.125 | 0.25 |
| *Ps. intermedius* 1264 | ≤ 0.03 | ≤ 0.03 | ≤ 0.03 |
| *Ps. intermedius* 1524 | 0.5 | 0.25 | 0.5 |
| *Ps. intermedius* 1624 | 1.0 | 0.5 | 1.0 |

[1]Pc = Peptococcus

[2]Ps = Peptostreptococcus

The A41030 antibiotic factors A, B, C, D, E, F and G, are also active against a number of strains of *Clostridium difficile*, as determined by the agar-dilution method. The results of the tests are recorded in Table 18, which follows.

TABLE 18

ACTIVITY OF A41030 FACTORS AGAINST *CLOSTRIDIUM DIFFICILE* STRAINS

| *Clostridium difficile* | MIC (μg./ml.) | | | | | | |
|---|---|---|---|---|---|---|---|
| | A | B | C | D | E | F | G |
| 8484 | 1.0 | 1.0 | 1.0 | ≤0.25 | 0.5 | 1.0 | 1.0 |
| 6890 | 1.0 | 1.0 | 2 | 0.5 | 0.5 | 1.0 | 1.0 |
| 2634 | 1.0 | 1.0 | 2 | 0.5 | 1.0 | 2 | 1.0 |
| 78 | 1.0 | 0.5 | 1.0 | ≤0.25 | 0.5 | 1.0 | 1.0 |
| A-194 | 1.0 | 1.0 | 2 | ≤0.25 | 0.5 | 1.0 | 1.0 |
| A-195 | 1.0 | 1.0 | 1.0 | ≤0.25 | 0.5 | 1.0 | 1.0 |
| A-196 | 1.0 | 1.0 | 2 | 0.5 | 1.0 | 2 | 1.0 |
| A-279 | 1.0 | 1.0 | 2 | ≤0.25 | 0.5 | 1.0 | 1.0 |
| A-280 | 1.0 | 0.5 | 1.0 | ≤0.25 | 0.5 | 1.0 | 1.0 |
| A-281 | 1.0 | 1.0 | 2 | 0.5 | 1.0 | 2 | 1.0 |
| WAL-2112 | 1.0 | 1.0 | 2 | ≤0.25 | 0.5 | 1.0 | 1.0 |
| WAL-3657 | 1.0 | 1.0 | 2 | ≤0.25 | 0.5 | 1.0 | 1.0 |
| WAL-4268 | 1.0 | 0.5 | 1.0 | ≤0.25 | 0.5 | 1.0 | 1.0 |
| 107B | 1.0 | 0.5 | 1.0 | ≤0.25 | 0.5 | 1.0 | 1.0 |
| 111F | 1.0 | 1.0 | 2 | ≤0.25 | 0.5 | 2 | 1.0 |
| 1153 | 1.0 | 1.0 | 2 | 1.0 | 1.0 | 2 | 1.0 |
| 3424-5B | 1.0 | 1.0 | 1.0 | 0.5 | 0.5 | 1.0 | 1.0 |
| 3816 | 1.0 | 1.0 | 2 | 0.5 | 0.5 | 1.0 | 1.0 |
| 3950D | 1.0 | 1.0 | 2 | 0.5 | 0.5 | 1.0 | 1.0 |

The *in vitro* activity of antibiotic A41030 factors A, B, C, D, E, F and G against a number of aerobic bacteria has been determined using a standard agar-dilution assay. The results after reading the end point after 24 hours are recorded in Table 19.

TABLE 19

ACTIVITY OF A41030 FACTORS AGAINST AEROBIC BACTERIA

| Test Organism | MIC (μg./ml.) | | | | | | |
|---|---|---|---|---|---|---|---|
| | A | B | C | D | E | F | G |
| *Staphylococcus aureus* 3055 | 0.125 | 0.125 | 0.5 | 0.25 | 0.125 | 0.5 | 0.5 |
| *Staphylococcus aureus* V41 | 0.125 | 0.125 | 0.5 | 0.25 | 0.25 | 0.5 | 1 |
| *Staphylococcus aureus* X400 | 0.5 | 0.25 | 1 | 0.25 | 0.25 | 1 | 2 |
| *Staphylococcus aureus* S13E | 0.25 | 0.125 | 0.5 | 0.25 | 0.25 | 1 | 1 |
| *Staphylococcus epidermidis* EPI1 | 0.125 | 0.125 | 0.5 | 0.25 | 0.25 | 0.25 | 0.5 |
| *Staphylococcus epidermidis* EPI2 | 1 | 0.5 | 2 | 1 | 2 | 2 | 0.5 |
| *Streptococcus pyogenes* C203 | 0.5 | 0.25 | 1 | 0.5 | 0.25 | 2 | 2 |
| *Streptococcus pneumoniae* Park I | 0.5 | 0.5 | 1 | 0.5 | 0.25 | 2 | 2 |
| *Streptococcus sp.* Group D X66 | 1 | 1 | 2 | 1 | 1 | 4 | 4 |
| *Streptococcus sp.* Group D 9960 | 2 | 1 | 4 | 1 | 1 | 4 | 4 |
| *Haemophilus influenzae* Brun | 8 | 8 | 16 | — | — | — | — |
| *Haemophilus influenzae* 251 | 2 | 2 | 4 | — | — | — | — |
| *Haemophilus influenzae* C.L. | — | — | — | 8 | 8 | 32 | 32 |
| *Haemophilus influenzae* 76 | — | — | — | 8 | 8 | 32 | 32 |
| *Shigella sonnei* N9 | >128 | 128 | >128 | >128 | >128 | >128 | >128 |
| *Escherichia coli* N10 | >128 | 128 | >128 | >128 | >128 | >128 | >128 |
| *Escherichia coli* EC14 | 128 | 128 | >128 | >128 | >128 | >128 | >128 |
| *Escherichia coli* TEM | >128 | 128 | >128 | 64 | >128 | >128 | >128 |
| *Klebsiella pneumoniae* X26 | >128 | >128 | >128 | 128 | >128 | >128 | 64 |
| *Klebsiella pneumoniae* KAE | >128 | >128 | >128 | >128 | >128 | >128 | >128 |

Table 19 (Continued)

| Test Organism | A | B | C | D | E | F | G |
|---|---|---|---|---|---|---|---|
| *Klebsiella pneumoniae* X68 | > 128 | 128 | > 128 | > 128 | > 128 | > 128 | >128 |
| *Enterobacter aerogenes* C32 | > 128 | 64 | > 128 | > 128 | > 128 | > 128 | >128 |
| *Enterobacter aerogenes* EB17 | > 128 | > 128 | > 128 | > 128 | > 128 | > 128 | >128 |
| *Enterobacter cloacae* EB5 | > 128 | 128 | > 128 | > 128 | > 128 | > 128 | >128 |
| *Enterobacter cloacae* 265A | > 128 | 128 | > 128 | > 128 | > 128 | > 128 | >128 |
| *Salmonella typhi* X514 | > 128 | > 128 | > 128 | > 128 | > 128 | > 128 | >128 |
| *Salmonella typhi* 1335 | > 128 | > 128 | > 128 | > 128 | > 128 | > 128 | >128 |
| *Pseudomonas aeruginosa* X528 | > 128 | > 128 | > 128 | > 128 | > 128 | > 128 | >128 |
| *Pseudomonas aeruginosa* X239 | > 128 | > 128 | > 128 | > 128 | > 128 | > 128 | >128 |
| *Pseudomonas aeruginosa* Ps18 | > 128 | > 128 | > 128 | > 128 | > 128 | > 128 | >128 |
| *Pseudomonas aeruginosa* Ps72 | -- | -- | -- | > 128 | > 128 | > 128 | >128 |
| *Serratia marcescens* X99 | > 128 | 128 | > 128 | > 128 | > 128 | > 128 | >128 |
| *Serratia marcescens* SE3 | > 128 | > 128 | > 128 | > 128 | > 128 | > 128 | >128 |
| *Proteus morganii* PR15 | > 128 | > 128 | > 128 | > 128 | > 128 | > 128 | >128 |
| *Proteus inconstans* PR33 | > 128 | > 128 | > 128 | > 128 | > 128 | > 128 | >128 |
| *Proteus rettgeri* PR7 | > 128 | > 128 | > 128 | -- | -- | -- | -- |
| *Proteus rettgeri* C24 | > 128 | > 128 | > 128 | > 128 | > 128 | > 128 | >128 |
| *Citrobacter freundii* CF17 | > 128 | 128 | > 128 | > 128 | > 128 | > 128 | >128 |
| *Bordetella bronchiseptica* 16 | > 128 | > 128 | > 128 | -- | -- | -- | -- |
| *Acinetobacter calcoaceticus* AC12 | -- | -- | -- | > 128 | > 128 | > 128 | >128 |

– = not tested

The *in vitro* activity of antibiotic A41030 factors A and B against a number of representative aerobic, gram-positive bacteria, including *Streptococcus* sp. Group D, has been determined using a standard agar-dilution assay. The results, determined by reading the end point after 24 hours, are recorded in Table 20.

TABLE 20

ACTIVITY OF A41030 FACTORS A AND B AGAINST AEROBIC BACTERIA

| Test Organism | MIC (μg./ml.) A | MIC (μg./ml.) B |
|---|---|---|
| *Staphylococcus aureus* | | |
| 3055 | 0.06 | 0.06 |
| X400 | 0.125 | 0.125 |
| V138 | 0.06 | 0.06 |
| V140 | 0.125 | 0.125 |
| V102 | 0.125 | 0.125 |
| *Staphylococcus epidermidis* | | |
| 222 | 0.125 | 0.125 |
| 270 | 0.06 | 0.06 |
| 285 | 0.06 | 0.03 |
| 219 | 0.06 | 0.03 |
| 269 | 0.06 | 0.03 |
| *Streptococcus* pyrogenes C203 | 0.25 | 0.125 |
| *Streptococcus* sp. ATCC 10389 | 0.125 | 0.25 |
| *Streptococcus* sp. Group B | | |
| 5 | 1 | 1 |
| 14 | 0.06 | 0.125 |
| *Streptococcus* sp. Group D | | |
| X66 | 0.25 | 0.25 |
| 9960 | 0.5 | 0.5 |
| 2041 | 0.5 | 0.5 |
| 8043 | 0.25 | 0.25 |
| 9901 | 1 | 1 |
| 12253F | 0.25 | 0.5 |
| Mx161 | 0.25 | 0.25 |
| 2058 | 0.5 | 0.5 |
| *Streptococcus pneumoniae* Park I | 0.125 | 0.125 |
| *Streptococcus pneumoniae* B1-438 | 0.25 | 0.5 |
| *Haemophilus parainfluenzae* 7901 | 16 | 16 |
| *Haemophilus parainfluenzae* 9796 | 16 | 16 |

| *Haemophilus influenzae* | | |
|---|---|---|
| C.L. | 4 | 8 |
| Mx366 | 2 | 4 |
| Mx371 | 2 | 4 |
| 76 | 4 | 4 |
| Bond | 2 | 4 |
| 16836 | 4 | 4 |
| 4842 | 2 | 4 |
| 312 | 2 | 4 |

The activity of antibiotic A41030 complex against a number of animal pathogens was determined by a standard *in vitro* antimicrobial broth microtiter test, and the results are set forth in Table 21.

TABLE 21

ACTIVITY OF A41030 COMPLEX AGAINST SEVERAL ANIMAL PATHOGENS

| Test Organism | MIC (µg./ml.) |
|---|---|
| *Staphylococcus sp.* 1130 | < 0.78 |
| *Streptococcus sp.* 80 | < 0.78 |
| *Pasteurella multocida* (bovine) | 3.12 |
| *Pasteurella hemolytica* | 6.25 |
| *Bordetella bronchiseptica* (Switzer) | 50.00 |
| *Escherichia coli* | 50.00 |
| *Mycoplasma synoviae* | 50.00 |
| *Mycoplasma hyorhinis* | 50.00 |
| *Pseudomonas* — fish | < 0.78 |
| *Aeromonas liquefaciens* | 50.00 |

All of the A41030 factors tested have shown *in vivo* antimicrobial activity against experimental bacterial infections. When two doses of test compound were administered subcutaneously to mice in illustrative infections, the activity observed is measured as an $ED_{50}$ value [effective dose in mg./kg. to protect fifty percent of the test animals: See Warren Wick, *et al.*, *J. Bacteriol.* *81*, 233—235 (1961)]. The $ED_{50}$ values observed for A41030 factors A, B, C, D, E, and F are given in Table 22, which follows.

TABLE 22

| | *Staph. aureus* | *S. pyrogenes* | *S. pneumoniae* |
|---|---|---|---|
| Antibiotic | $ED_{50}$ | $ED_{50}$ | $ED_{50}$ |
| A41030A | 1.4 | 2.8 | 1.68 |
| A41030B | < 0.43 | 1.4 | 1.4 |
| A41030C | < 0.43 | 10.4 | 6.7 |
| A41030D | 0.339 | 3.24 | 2.21 |
| A41030E | < 0.31 | 3.54 | 3.11 |
| A41030F | < 0.31 | > 5.0 | > 5.0 |

The acute toxicity of each of the antibiotic A41030 factors A, B, and C, has been determined in mice to be >300 mg./kg., when administered intraperitoneally.

The $LD_{50}$ of each of the antibiotic A41030 factors A, B, and C, has been determined in mice as being >300 mg./kg., when administered intraperitoneally.

The *in vivo* oral activity of each of the antibiotic A41030 factors A, B, and C, as determined against *S. pyogenes* in mice, is >300 mg./kg. × 2.

In one of its aspects this invention provides a method for treating infections in a warm-blooded animal which comprises administering to said animal a chemotherapeutically-effective amount, for example between about 25 mg. and about 2,000 mg., of the A41030 antibiotic complex or factor thereof, or a pharmaceutically-acceptable salt thereof.

Factor A, or a pharmaceutically-acceptable salt thereof, can be used in the treatment of infections in man, but in general, the complex and other factors, and salts thereof, are best suited for use in the treatment of infections in other warm-blooded animals.

In the treatment of infections in man the antibiotic factor, preferably factor A, can be administered by the parenteral route, e.g., by i.m. injection, or i.v. infusion. For injection, the antibiotic or a pharmaceutically-acceptable salt thereof is dissolved in a physiologically acceptable diluent at the desired concentration and administered. Suitable diluents include for example, Waterfor-Injection, 0.9% saline, 5% dextrose, Ringer's solution, or other commonly employed diluent. For administration by i.v. infusion, the antibiotic or salt thereof can be made up on a physiological fluid or dilute nutrient at a suitable concentration; for example, at a concentration between about 5% and about 10%, and slowly infused with the fluid. Alternatively, the antibiotic may be administered by the "piggy-back" method.

The individual factors, combinations of the factors, or the whole complex of factors and the pharmaceutically-acceptable salts thereof can be made up in dosage unit formulations in hermetically sealed vials, sterile, rubber-stoppered vials, or in plastic pouches. Such unit dosage forms can contain excipients such as antioxidants, solubilizing agents, dispersing agents, buffers, and the like. One such dosage unit formulation comprises 100 mg. of factor A, or a pharmaceutically-acceptable salt thereof, in a rubber (butyl rubber) stoppered vial. Another dosage unit formulation comprises 250 mg. of factor A, or a salt thereof, in a sterile, sealed vial. For i.v. infusion a dosage unit formulation of this invention may comprise 5 g. of factor A, or a pharmaceutically-acceptable salt thereof, in a plastic pouch.

When used as an antibacterial agent, the A41030 complex or an A41030 factor may be administered either orally or parenterally. As will be appreciated by those skilled in the art, the A41030 complex or factor is commonly administered together with a pharmaceutically-acceptable carrier or diluent. The dosage of A41030 complex or factor will depend upon a variety of considerations, such as the nature and severity of the particular infection to be treated. Those skilled in the art will recognize that appropriate dosage ranges and/or dosage units for administration may be determined by considering the MIC and $ED_{50}$ values and toxicity data herein provided, together with factors such as the patent or host and the infecting organism.

The A41030 antibiotics are useful, *inter alia,* for suppressing the growth of *Staphylococcus, Streptococcus* and *Propionibacterium acnes* organisms, and the antibiotics could therefore be used, for example, in the treatment of acne. The A41030 individual factors, or mixtures thereof in the purified state, can be formulated in pharmaceutically-acceptable diluents such as isopropyl alcohol for application to the skin. Such solutions can be made up with antibiotic concentrations of from about 1 to about 15 percent weight per volume. Alternatively, the antibiotics can be made up into creams or lotions for application to the skin.

The A41030 antibiotics are also useful for suppressing the growth of *Clostridium difficile* organisms, which cause *Pseudomembranous colitis* in the intestine. The A41030 individual factors or mixtures thereof could be used in the treatment of *Pseudomembranous colitis* by the oral administration of an effective dose of these antibiotics or a pharmaceutically-acceptable salt thereof, prepared in a pharmaceutically-acceptable dosage form. For such use the antibiotic can be administered in gelatin capsules or in liquid suspension.

The antibiotics of this invention also can be used in verterinary medicine in the treatment of infectious diseases in domestic and farm animals. They are useful also in animal husbandry, e.g., in enhancing the growth of beef cattle and other ruminants. An especially valuable use for the antibiotics of this invention resides in their ability to increase the production of milk in dairy cattle. These uses are further aspects of this invention which are described in more detail in the following paragraphs.

The A41030 complex has shown activity against infectious canine hepatitis virus *in vitro* at 40 mcg./ml. The A41030 complex has also shown activity *in vitro* against pseudorabies at 20 mcg./ml; and A41030 factor A has shown activity *in vitro* against pseudorabies at 20 mcg./ml.

Antibiotic A41030 complex has shown activity as a growth promoter in chickens, the test being carried out as follows:

Test 1

Chicks, 8-day old Penobscot broilers, were utilized in this test. A total of 560 chicks were used, divided in groups of 7 birds each. There were 35 groups acting as controls, and 5 groups were treated with the antibiotic added to the standard chick ration at the rate of 20 g. of antibiotic A41030 complex per ton of feed. Feed and water were available to all groups *ad libitum* for 21 days. Two time replicates were run. The

criteria for activity: 3% increase in weight gain and/or 2% improvement in feed efficiency in one or both time replicates. The results are set forth in Table 23.

TABLE 23

| Treatment | Conc. g./T. | Weight Gain gm. | Weight Gain % Impr. | Feed Efficiency F/G | Feed Efficiency % Impr. |
|---|---|---|---|---|---|
| Control | - | 433 | - | 1.671 | - |
| A41030 | 20 | 451 | 4.16 | 1.601 | 4.19 |
| Control | - | 451 | - | 1.673 | - |
| A41030 | 20 | 464 | 2.88 | 1.651 | 1.32 |

F/G = Total feed consumed divided by total weight gain.

Accordingly, this invention provides a method of enhancing the growth of chickens which comprises administering to the chickens in their diet between about 20 and about 30 g. of an A41030 antibiotic factor or the A41030 antibiotic complex, or a pharmaceutically-acceptable salt thereof, per ton of feed. Alternatively, the antibiotic factors or the complex, in the form of a pharmaceutically-acceptable, non-toxic salt can be administered in the drinking water of the chickens.

Antibiotic A41030 also acts as a growth promoter when administered to weanling pigs. The antibiotic was tested in young pigs at several dosage levels, as later described.

Test 2

The antibiotic A41030 complex was tested at levels of 5, 20, 50 and 100 ppm. in the diet of pigs initially weighing about 21 pounds (5—7 weeks of age). The experiment was conducted in an environmentally controlled nursery facility with wire mesh floors in the pens. There were five replicates per treatment and five pigs per replicate for the 27-day experiment. The results of this experiment appear in Table 24:

Table 24

| Treatment | Level ppm | ADG lbs. | % Increase | ADF lbs. | % Increase | F/G | % Improvement |
|---|---|---|---|---|---|---|---|
| Basal | -- | 0.67 | | 1.25 | | 1.87 | |
| A41030 | 5 | 0.70 | +4.5 | 1.33 | 6.4 | 1.89 | -1.0 |
| A41030 | 20 | 0.67 | 0.0 | 1.25 | 0.0 | 1.93 | -3.2 |
| A41030 | 50 | 0.71 | +6.0 | 1.25 | 0.0 | 1.78 | 4.8 |
| A41030 | 100 | 0.77 | +14.9 | 1.34 | 7.2 | 1.74 | 7.4 |

ADG = average daily gain

ADF = average daily feed consumption

F/G = ratio of feed consumption to gain

In this trial the antibiotic appeared to elicit a dose-related improvement in growth performance in weanling pigs, as indicated by the response at 5 ppm and at 100 ppm.

Test 3

The antibiotic A41030 complex further was tested in weanling pigs (17 pounds, 4—6 weeks of age) at levels of 25, 50 and 100 gm./ton of feed for 35 days. There were four replicates of six pigs per treatment.

Antibiotic A41030 complex when administered to these weanling pigs at the rates taught, increased the rate of gain by 5.6 percent, 8.5 percent, and 7.0 percent; and improved the feed concersion efficiency by 6.6 percent, 9.2 percent, and 3.1 percent, when added to the diet at 25, 50 and 100 gm./ton, respectively. These results are recorded in Table 25.

Table 25

| Treatment | Level ppm | ADG lbs. | % Increase | ADF lbs. | % Increase | F/G | % Improvement |
|---|---|---|---|---|---|---|---|
| Basal | - | 0.71 | - | 1.39 | - | 1.96 | - |
| A41030 | 25 | 0.75 | 5.6 | 1.38 | -0.7 | 1.83 | 6.6 |
| A41030 | 50 | 0.77 | 8.5 | 1.38 | -0.7 | 1.78 | 9.2 |
| A41030 | 100 | 0.76 | 7.0 | 1.44 | 3.6 | 1.90 | 3.1 |

Thus in another aspect, this invention provides a method for promoting the growth of weanling pigs which comprises administering to the pigs in their diet between about 5 and about 100 ppm of the A41030 antibiotic complex, or an A41030 antibiotic factor, or a pharmaceutically-acceptable, non-toxic salt thereof, as well as the method of promoting the growth of the weanling pigs which comprises administering to the pigs between about 25 g. and about 100 g. of the A41030 antibiotic complex, or a pharmaceutically-acceptable, non-toxic salt thereof per ton of feed. The antibiotic A41030 complex, in the form of a pharmaceutically-acceptable, non-toxic salt, also could be administered to the pigs in the drinking water.

The A41030 antibiotics are also useful for increasing the efficiency of feed utilization in ruminant animals. It is known that the efficiency of carbohydrate utilization in ruminants is increased by treatments which stimulate the animals' rumen flora to produce propionate compounds rather than acetate or butyrate compounds (for a more complete discussion, see Church *et al.* in "Digestive Physiology and Nutrition of Ruminants," Vol. 2, 1971, pp. 622 and 625).

The effectiveness of antibiotic A41030 complex and A41030 factor A for modifying the ratio of volatile fatty acids produced in the rumen is shown by means of *in vitro* tests according to the procedure set forth below.

Test 4

Rumen fluid was obtained from a steer which has a surgically-installed fistula opening into the rumen. The steer was maintained on a high-grain ration, the composition of which follows:

| | |
|---|---|
| 69.95% | coarse ground corn |
| 10% | ground corncobs |
| 8% | soybean meal (50% protein) |
| 5% | alfalfa meal |
| 5% | molasses |
| 0.6% | urea |
| 0.5% | dicalcium phosphate |
| 0.5% | calcium carbonate |
| 0.3% | salt |
| 0.07% | Vitamin A and $D_2$ premix |
| 0.05% | Vitamin E premix |
| 0.03% | trace mineral premix |

A sample or rumen fluid was strained through 4 layers of cheesecloth and the filtrate was collected in a vacuum bottle. The particulate matter retained by the cheesecloth was resuspended in enough physiological buffer to return it to the original volume of the rumen fluid, and the suspension was again strained through cheesecloth. The buffer used is described below:

| | |
|---|---|
| 0.316 g./liter | $Na_2HPO_4$ |
| 0.152 g./liter | $KH_2PO_4$ |
| 2.260 g./liter | $NaHCO_3$ |
| 0.375 g./liter | KCl |
| 0.375 g./liter | NaCl |
| 0.112 g./liter | $MgSO_4$ |
| 0.038 g/liter | $CaCl_2$ |
| 0.008 g./liter | $FeSO_4 \cdot 7H_2O$ |
| 0.004 g./liter | $MnSO_4$ |
| 0.004 g./liter | $ZnSO_4 \cdot 7H_2O$ |
| 0.002 g./liter | $CuSO_4 \cdot 5H_2O$ |
| 0.001 g./liter | $CoCl_2$ |

Cheng *et al.*, J. Dairy Sci. *38*, 1225 (1955).

The two filtrates were pooled in a separatory funnel and allowed to stand till particulate matter rose to the top. The clear layer was then separated and diluted 1:1 with the same buffer, and adjusted to pH 7.0.

Ten ml. of the diluted rumen fluid thus prepared were placed in a 25 ml. flask with 90 mg. of finely-powdered high-grain ration, the composition of which is described above. The compound to be tested was weighed out and dissolved in the appropriate solvent, supra. The solution was placed on the finely-powdered ration in each test flask and dried.

Two sets of four untreated control flasks each were prepared also. One set of four untreated control flasks was incubated for 16 hours at 38°C. with the test flasks. The other set of four untreated control flasks were zero-time controls, the fermentation in which was stopped as soon as the flasks were prepared by addition of 2 ml. of 25 percent metaphosphoric acid to each flask.

Fermentation in the incubated test and control flasks was stopped at the end of 16 hours by addition of 2 ml. of 25 percent metaphosphoric acid to each flask.

All of the samples were allowed to settle, and the supernatant was analyzed by gas chromatographic methods for acetate, propionate, and butyrate.

The analysis for each volatile fatty acid found in the zero-time controls was subtracted from the analyses of the untreated controls and of the test flasks. The resulting values reflect the amount of each VFA produced during the 16-hour fermentation period.

The data in Table 26 are reported as the ratio of VFA's produced in treated flasks to VFA's produced in untreated control flasks. This method of reporting the data shows most clearly the results of the changes in the chemistry of the rumen brought about by the present novel method of feed utilization improvement.

Table 26

| Compound | Rate | Acetate | Propionate | Butyrate |
|---|---|---|---|---|
| A41030 | 5 mcg./ml. | 0.94 | 1.23 | 0.72 |
| A41030 | 5 mcg./ml. | 1.03 | 1.33 | 0.59 |
| A41030 | 5 mcg./ml. | 0.94 | 1.51 | 0.54 |
| A41030A | 2 mcg./ml. | 1.03 | 1.33 | 0.94 |
| A41030A | 1 mcg./ml. | 1.01 | 1.31 | 0.66 |

The data tabulated above shows that the antibiotics are effective in increasing propionate production in the rumen.

Administration of the antibiotic compounds useful in this method prevents and treats ketosis as well as improves feed utilization. The causative mechanism of ketosis is a deficient production of propionate compounds. A presently recommended treatment is administration of propionic acid or feeds which preferentially produce propionates. Obviously the method disclosed in this application, encourages propionate production from ordinary feeds, reducing the incidence of ketosis.

It has been found that the antibiotic compounds disclosed herein increase the efficiency of feed utilization in ruminant animals when administered in a propionate-increasing dose. The antibiotics, individually or as the whole complex, or as an economical, less-purified whole complex, are administered suitably by incorporation in the animal's feed. The antibiotic compounds, however, can be usefully administered in other ways. For example, they can be incorporated into tablets, drenches, boluses, or capsules, and dosed to the animals. Formulation of the antibiotic compounds in such dosage forms can be accomplished by means of methods well known in the veterinary pharmaceutical art. Each individual dosage unit should contain a quantity of the feed-efficiency-improving compound which has a direct relation to the proper daily dose for the animal to be treated.

Capsules are readily produced by filling gelatin capsules with any desired form of the desired antibiotic. If desired, the antibiotic can be diluted with an inert powdered diluent, such as a sugar, starch, or purified crystalline cellulose in order to increase its volume for convenience in filling capsules.

Tablets of the antibiotics are made by conventional pharmaceutical processes. Manufacture of tablets is a well-known and highly-advanced art. In addition to the active ingredient, a tablet usually contains a base, a disintegrator, an absorbent, a binder, and a lubricant. Typical bases include lactose, fine icing sugar, sodium chloride, starch and mannitol. Starch is also a good disintegrator, as is alginic acid. Surface active agents such as sodium lauryl sulfate and dioctyl sodium sulphosuccinate may be used also. Commonly used absorbents again include starch and lactose, while magnesium carbonate is also useful or oily substances. Frequently-used binders are galatin, gums, starch, dextrin and various cellulose derivatives. Among the commonly-used lubricants are magnesium stearate, talc, paraffin wax, various metallic soaps, and polyethylene glycol.

The antibiotic compound can be administered also as a slow-payout bolus. Such boluses are made as tablets are made, except that a means to delay the dissolution of the antibiotic is provided. Boluses are made to release for lengthy periods. The slow dissolution is assisted by choosing a highly water-insoluble form of the antibiotic. A substance such as iron filings is added to raise the density of the bolus and keep it static on the bottom of the rumen.

Dissolution of the antibiotic is delayed by use of a matrix of insoluble materials in which the drug is embedded. For example, substances such as vegetable waxes, purified mineral waxes, and water-insoluble polymeric materials are useful.

Drenches of the antibiotics are prepared most easily by choosing a water-soluble form of the antibiotic. If an insoluble form is desired for some reason, a suspension may be made. Alternatively, a drench may be formulated as a solution in a physiologically-acceptable solvent such as a polyethylene glycol.

Suspensions of insoluble forms of the antibiotics can be prepared in non-solvents such as vegetable oils such as peanut, corn, or sesame oil; in a glycol such as propylene glycol or a polyethylene glycol; or in water, depending on the form of the antibiotic chosen.

Suitable physiologically-acceptable adjuvants are necessary in order to keep the antibiotic suspended. The adjuvants can be chosen from among the thickeners, such as carboxymethylcellulose, polyvinylpyrrolidone, gelatin, and the alginates. Many classes of surfactants also serve to suspend antibiotics. For example, lecithin, alkylphenol polyethylene oxide adducts, naphthalenesulfonates, alkylbenzenesulfonates, and the polyoxyethylene sorbitan esters are useful for making suspensions in liquid nonsolvents.

In addition, many substances which effect the hydrophilicity, density, and surface tension of the liquid can assist in making suspensions in individual cases. For example, silicone antifoams, glycols, sorbitol, and sugars can be useful suspending agents.

The suspendable antibiotic may be offered to the animal grower as a suspension, or as a dry mixture of the antibiotic and adjuvents to be diluted before use.

The antibiotics may also be administered in the drinking water of the ruminants. Incorporation into drinking water is performed by adding a water-soluble or water-suspendable form of the desired antibiotic to the water in the proper amount. Formulation of the antibiotic for addition to drinking water follows the same principles as formulation of drenches.

The most practical way to treat animals with these antibiotic compounds is by the formulation of the compounds into the feed supply. Any type of feed may be medicated with the antibiotic compound, including common dry feeds, liquid feeds, and pelleted feeds.

The methods of formulating drugs into animal feeds are well known. It is usual to make a concentrated drug premix as a raw material for medicated feeds. For example, typical drug premixes may contain from about one to about 400 grams of drug per pound of premix. The wide range results from the wide range of concentration of drug which may be desired in the final feed. Premixes may be either liquid or solid.

The formulation of animal feeds containing the proper amounts of the antibiotic compounds for useful

treatment is mainly a matter or arithmetic. It is necessary only to calculate the amount of compound which it is desired to administer to each animal, to take into account the amount of feed per day which the animal eats, and the concentration of antibiotic compound in the premix to be used, and calculate the proper concentration of antibiotic compound in the feed.

All of the methods of formulating, mixing, and pelleting feeds which are normally used in the ruminant or nonruminant feed art are entirely appropriate for manufacturing feeds containing the antibiotic compounds usable in this method.

It has been found also that antibiotic A41030 complex not only increases the efficiency of feed utilization in ruminants utilized for meat production (as described hereinbefore), but also causes a surprising improvement in milk production without an adverse effect on milk quality when administered to lactating animals having a developed rumen process.

The requirements and objectives of feed utilization of lactating ruminants such as dairy cows differ considerably from those of ruminants raised for meat production. Ruminal volatile fatty acid (VFA) production is of course of primary importance, because it relates directly to the normal maintenance of the animal, as well as to the quality and quantity of the milk produced by the animal. In the lactating ruminant, however, energy for lactation is the most limiting factor in milk production. Acetate is required for milk fat synthesis, while propionate is utilized to produce glucose, which in turn is required for lactose synthesis, and also has a minor role in milk fat production. Butyrate is more glycogenic than lipogenic, the lipogenic aspect being indirect since butyrate must first be degraded to acetate units before it can be utilized for long chain fatty acid synthesis, i.e. milk fat.

Accordingly, in order to increase milk production in lactating ruminants, it is necessary to increase propionate production, but not at a large expense of acetate and butyrate production. Significantly reduced acetate and butyrate levels result in drastically reduced milk fat content, thereby rendering milk production less efficient with respect to both quality and economically (bulk milk prices are determined in part by milk fat content).

The present improvement in milk production is manifested in increased protein content of the milk, without appreciable change of fat content. The method of accomplishing the improvement comprises orally administering to a lactating ruminant a propionate-increasing amount of antibiotic A41030 complex.

The antibiotic A41030 complex can be formulated for convenient oral administration to a ruminant, the formulation being as a feed pre-mix, a feed additive, a lick, a water additive, or if desired, the antibiotic A41030 complex can be formulated for slow release over a prolonged period of time following a single administration.

That administering a propionate-increasing amount of antibiotic A41030 complex to a lactating ruminant having a developed rumen function improves the milk production without adversely affecting the milk composition has been demonstrated by *in vivo* studies.

Test 5

Two trials were conducted using Holstein cows which had just calved.

In the first trial, twelve cows were employed, divided into two groups of six cows.

Two weeks after calving, the test animals began a one week preliminary period during which all the animals received a daily ration of feed and water, with no antibiotic being administered. All the cows received a standard feed ration containing 50% corn silage, 20% alfalfa hay, and 30% concentrate, the concentrate having the following composition:

Concentrate

| Ingredient | Percent | Lbs/Ton |
|---|---|---|
| Ground Corn, Yellow | 23.50 | 470.0 |
| Dicalcium Phosphate | 1.50 | 30.0 |
| Limestone | 1.25 | 25.0 |
| Molasses (Dehy) | 2.15 | 43.0 |
| Mon Na Phosphate | 0.10 | 2.0 |
| Soybean Oil Meal | 47.00 | 940.0 |
| Vitamin A+D3 Premix (Ca-01) (1) | 0.35 | 7.0 |
| Vitamin E Premix (2) | 0.10 | 2.0 |
| Selenium Premix (3) | 0.05 | 1.0 |
| Salt | 1.00 | 20.0 |
| Sodium Bicarbonate | 1.00 | 20.0 |
| Distillers Dried Solubles (Corn) | 22.00 | 440.0 |
| | 100.00 | 2000.0 |

(1) Each pound of Vitamin A and D3 premix contains 2,000,000 USP units Vitamin A and 225, 750 USP units Vitamin D3

(2) Each pound of Vitamin E premix contains 20,000 IU Vitamin E

(3) Each kg of selenium premix contains 200 mg. of selenium as sodium selenite. The calculated analysis for selenium is from the premix only.

All the cows were fed the standard ration at the rate of 2.95 percent of body weight (the body weight having been determined at the start of the one week preliminary period) plus two pounds of a formulation called a topdress, having the following composition:

Topdress

| Ingredient | Percent | Lbs/Ton |
|---|---|---|
| Ground Corn, Yellow | 20.00 | 400.0 |
| Grit-O-Cobs | 38.00 | 760.0 |
| Cane Molasses | 2.50 | 50.0 |
| Oats | 11.00 | 220.0 |
| Soybean Oil Meal | 25.00 | 500.0 |
| Vitamin A+D3 Premix (CA—01) (1) | 1.10 | 22.0 |
| Salt | 0.40 | 8.0 |
| Animal Fat | 2.00 | 40.0 |
| | 100.00 | 2000.0 |

(1) Each pound of Vitamin A and D3 premix contains 2,000,000 USP units Vitamin A and 225,750 USP units Vitamin D3.

During the one week preliminary period, all cows received control topdress. Beginning with the treatment period, test animals received their respective treatments for eight weeks with antibiotic A41030 complex (the test compound) mixed into the topdress. Control cows continued receiving the unmedicated topdress, identical to the topdress fed in the preliminary period. Control topdress and the topdress containing the antibiotic A41030 complex were fed to the experimental cows at the rate of two pounds daily within one hour of the morning feeding of the standard feed ration described above. The treated group received 600 mg./head/day of the antibiotic.

This trial was repeated, following the general procedure described above, and labelled Trial 2.

The average daily milk production, milk composition, and persistency of milk production of both trials were analyzed and the data are reported in Table 27. The persistency values were calculated by dividing the average milk production during the eight week treatment period by average production during the preliminary nontreatment period. Persistency values express how well the treatment groups performed after initiation of treatment compared to performance before treatment initiation. Normal persistency for the trial was expected to be about 94 to about 96 percent. In each experiment, cows fed A41030 complex had higher persistencies. This shows an observable response to treatment (i.e., maintenance of the preliminary period milk production at a higher level than control cows).

The analysis of milk composition reported in Table 27 demonstrates that milk fat is not different between the treated cows and the non-treated control cows, whereas protein content is enhanced. Increasing milk protein is an especially desirable response since even a small increase would significantly increase the amount of cheese that could be made from the milk.

Table 27

| Treatment | n | Milk lbs./Day Prel. | Milk lbs./Day Trt | Persistency % | Milk Fat % | Milk Protein % | Milk Solids % |
|---|---|---|---|---|---|---|---|
| **Trial 1** | | | | | | | |
| Control | 6 | 61.2 | 58.2 | $95.6^a$ | 3.52 | $3.29^c$ | 11.57 |
| A41030 | 6 | 52.0 | 53.3 | $103.9^a$ | 3.50 | $3.36^b$ | 11.63 |
| **Trial 2** | | | | | | | |
| Control | 7 | 50.6 | 48.5 | $95.9^a$ | - | - | - |
| A41030 | 8 | 54.0 | 52.9 | $98.3^a$ | - | - | - |

n = number of animals.

a = Persistency is calculated by dividing the average milk production during the treatment period by the production during the preliminary period. The persistency values are the result of using data of individual cows and represent the mean.

b and c = These values are different ($P<0.05$).

\- = not available.

The antibiotic A41030 complex and factors A, B, C, D, E, F, and G can be isolated for use in animal feeds by the methods disclosed in the Examples set forth earlier. It is also possible, if desired, after the production of A41030 antibiotic activity, to simply dry the whole fermentation broth and mix the dried medium directly into the feed or feed premix.

Further, it is possible that the herein disclosed A41030 antibiotic complex and factors can be combined with a synthetic tanning agent which is a sulfited phenol formaldehyde syntan, such as a sold by A. J. & O. J. Pilar Inc. of Newark, N. J., under the tradename TruTan RT Regular. The combination of the antibiotic and the synthetic tanning agent, the antibiotic-syntan complex, can be used without separation of the constituents, in the animal feed supplement compositions described above.

It is usual to treat economic animals, including ruminants, with a variety of growth promoters, disease-preventives, and disease treatments throughout their lives. Such drugs are often used in combination. The instant method may be practiced in combination with other treatments.

As has been shown, antibiotic A41030 complex and A41030 factor A beneficially alter the production of propionates relative to the production of actates in the rumen. The same treament also benefits monogastric animals which ferment fibrous vegetable matter in the cecum. The monogastric animals here referred to are those which consume fibrous vegetable food and digest at least part of it by microbioligical fermentation in the cecum. The cecal fermentation follows a chemical pathway similar to rumen fermentation.

Horses, swine, and rabbits are exemplary animals which digest a part of their food by cecal fermentation. The overall feed utilization of such animals is improved by the oral administration of these antibiotics which cause a beneficial change in the propionate/acetate ratio. Horses and rabbits are exemplary of animals in which cecal fermentation is a major part of the total digestive process, and in which these antibiotics are accordingly particularly beneficial.

**Claims for the Contracting States: BE CH DE GB IT LI LU NL SE**

1. A method of producing the A41030 antibiotic complex or Factor A, B, C, D, E, F or G, which comprises cultivating *Streptomyces virginiae* NRRL 15156 or 12525, or an A41030-producing variant or mutant thereof, in a culture medium containing assimilable sources of carbon, nitrogen, and inorganic salts under submerged aerobic fermentation conditions.

2. A41030 complex, or Factor A, B, C, D, E, F, or G obtainable by the method of Claim 1, or a pharmaceutically-acceptable salt thereof.

3. A41030 factor A of structure:

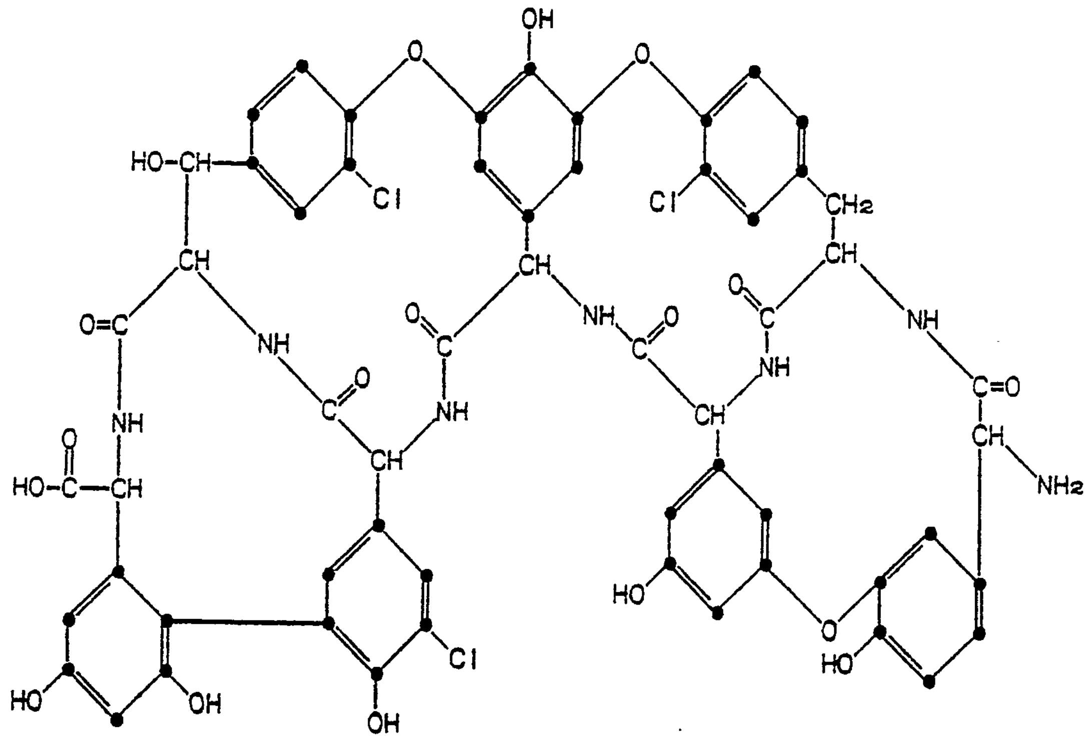

or a pharmaceutically-acceptable salt thereof.

4. A41030 factor B of structure:

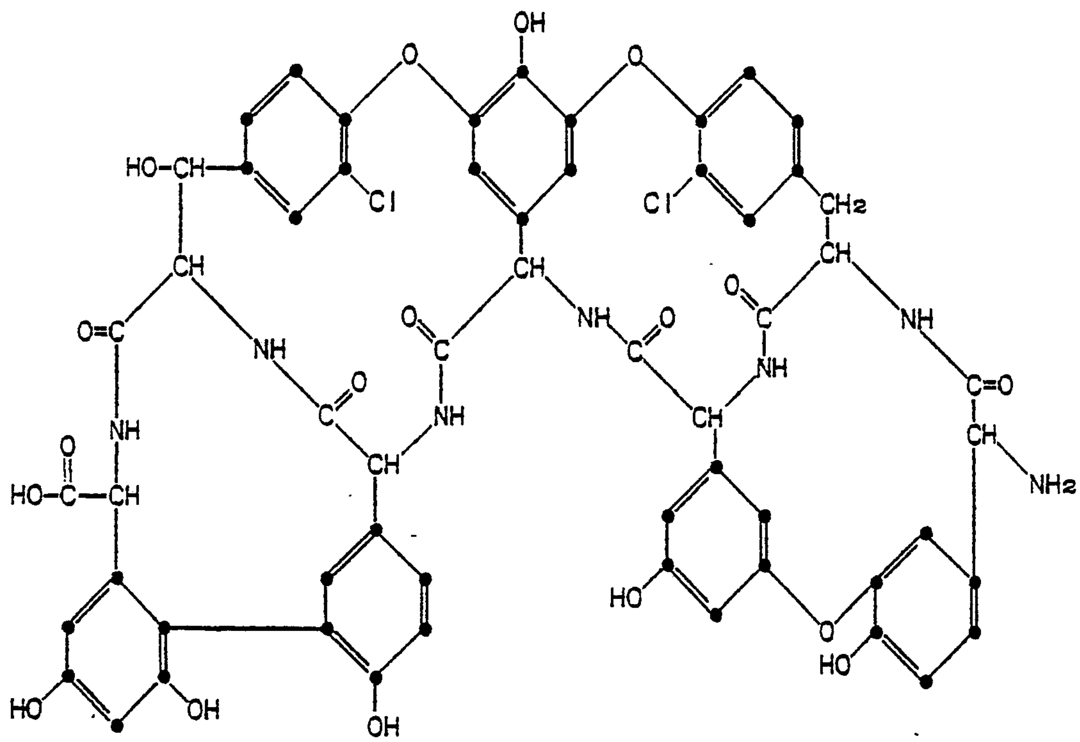

or a pharmaceutically-acceptable salt thereof.

5. A41030 factor C of structure:

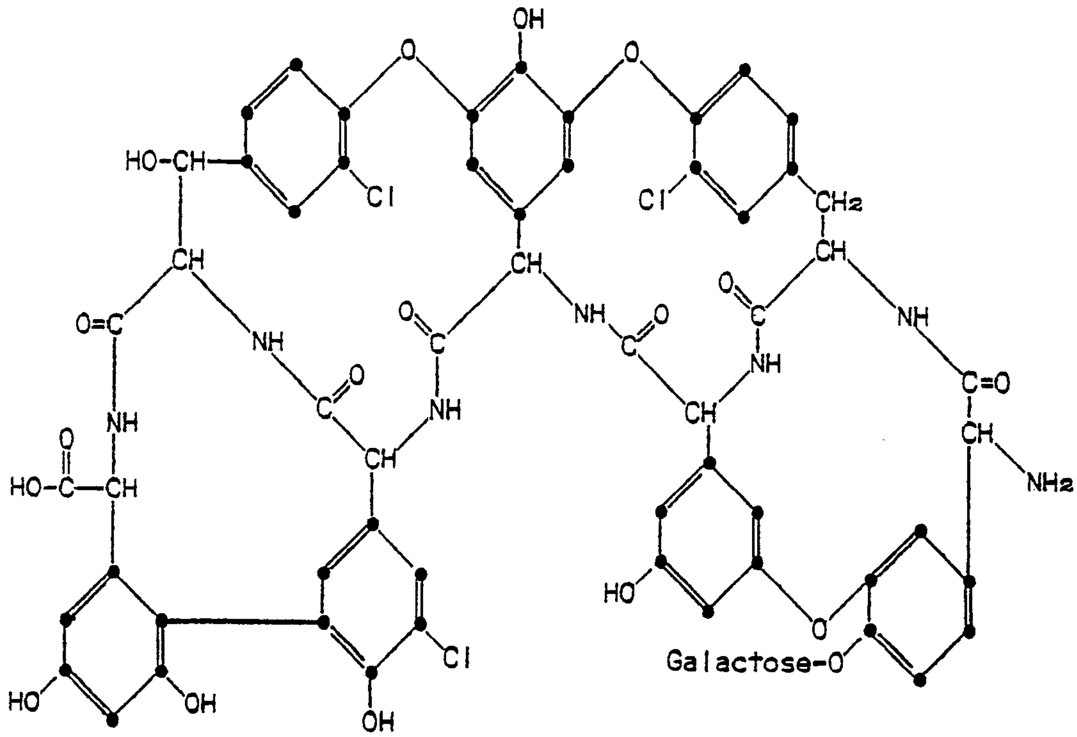

or a pharmaceutically-acceptable salt thereof.

6. Antibiotic A41030 factor D, which is a white, amorphous solid, having:

(a) an approximate elemental analysis of 54.46% carbon, 4.35% hydrogen, 7.58% nitrogen, 4.27% chlorine, and by difference, 29.34% oxygen;

(b) an observed molecular weight of about 1326, as determined by fast atom bombardment mass spectrometry;

(c) an infrared absorption spectrum having the following distinguishable maxima: 3448—3226 (strong broad), 2959 (weak), 1661 (strong), 1592 (strong), 1511 (strong), 1429 (weak), 1290 (weak), 1227 (weak), 1212 (medium), 1163 (weak), 1143 (weak), 1053 (medium), and 1010 (strong) $cm^{-1}$;

(d) ultraviolet absorption spectra with an absorption maximum, in acidic or neutral methanol:water (1:1), at 278 nm (ε 10,600) and, in basic methanol:water (1:1), at 298 nm (ε 19,900);

(e) two titratable groups in 66% aqueous dimethylformamide with $pK_a$ values of about 5.5 and 7.6;

(f) is soluble in alcohol-water mixtures, in dimethylsulfoxide, in dimethylformamide, in dimethylsulfoxide-water mixtures, in di-methylformamide-water mixtures, in dilute aqueous acid, or in dilute aqueous base; and

(g) the pharmaceutically-acceptable, non-toxic salts of said A41030 factor D.

7. A41030 factor E of structure:

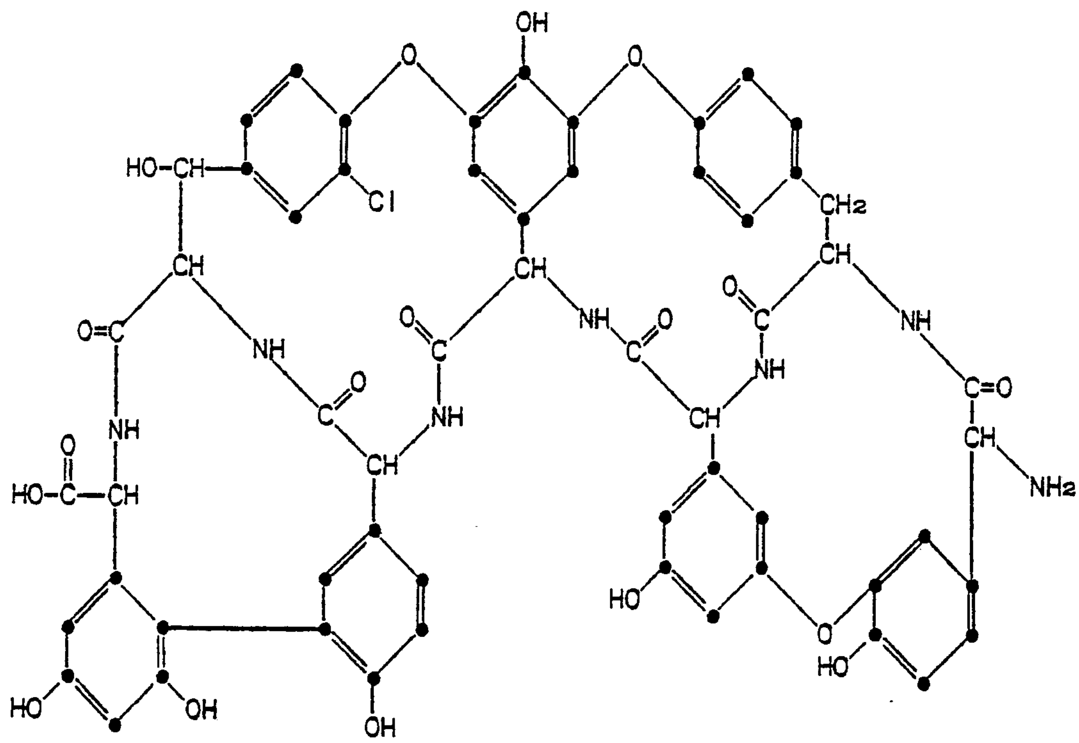

or a pharmaceutically-acceptable salt thereof.

8. A41030 factor F of structure:

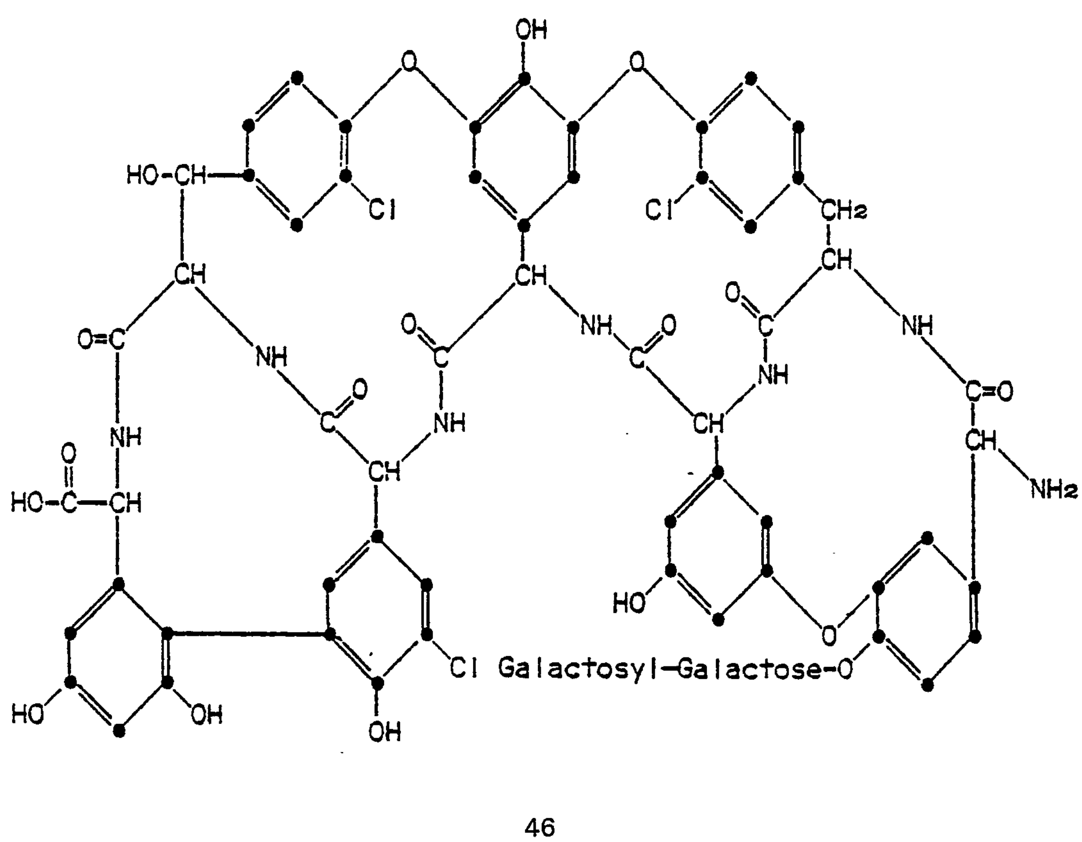

or a pharmaceutically-acceptable salt thereof.

9. Antibiotic A41030 factor G, which is a white solid having:

(a) an approximate elemental analysis of 50.02% carbon, 4.61% hydrogen, 4.74% chlorine, 6.11% nitrogen, and 30.70% oxygen;

(b) an observed molecular weight of about 1688 as determined by fast atom bombardment mass spectrometry;

(c) an infrared absorption spectrum having the following distinguishable absorption maxima: 3320 (very broad, strong), 2975 (sharp, weak), 2920 (sharp, weak), 1659 (normal, strong), 1594 (broad, strong), 1512 (sharp, strong), 1492 (shoulder), 1430 (sharp, weak), 1386 (broad, weak), 1337 (broad, weak), 1308 (sharp, weak), 1264 (sharp, weak), 1230 (broad, medium), 1145 (broad, medium), 1077 (sharp, medium), 1062 (sharp, medium), 1014 (sharp, medium), and 846 (broad, medium) $cm^{-1}$;

(d) ultraviolet absorption spectra with an absorption maximum, in acidic or neutral methanol:water (1:1), at 278 nm (ε 15,000) and, in basic methanol:water (1:1), at 298 nm (ε 18,000);

(e) two titratable groups in 66% aqueous dimethylformamide with $pK_a$ values of about 5.4 and 7.0;

(f) is soluble in alcohol-water mixtures, in dimethylsulfoxide, in dimethylformamide, in dimethylsulfoxide-water mixtures, in di-methylformamide-water mixtures, in dilute aqueous acid, or in dilute aqueous base; and

(g) the pharmaceutically-acceptable, non-toxic salts of said A41030 factor G.

10. A method according to claim 1 followed by separation of the complex from the culture medium.

11. A method according to claim 10, in which the factors A, B, C, D, E, F or G are isolated from the complex.

12. A41030 complex, or Factor A, B, C, D, E, F or G according to claim 2 or a pharmaceutically-acceptable salts thereof, for use in veterinary or a pharmaceutical chemotherapy.

13. A41030 complex, or Factor A, B, C, D, E, F or G according to claim 2 or a pharmaceutically-acceptable salts thereof for use in improving milk production in lactating ruminants.

14. A feed premix which comprises as an active ingredient A41030 complex, A41030 factor A, B, C, D, E, F or G according to claim 2 or a pharmaceutically-acceptable salts thereof.

15. A41030 complex, or Factor A, B, C, D, E, F or G according to claim 2 or a pharmaceutically-acceptable salts thereof, for use in increasing the feed utilization efficiency of ruminant animals.

16. A41030 complex, A41030 factor A, B, C, D, E, F or G according to claim 2 or a pharmaceutically-acceptable salt thereof for use in promoting growth in warm-blooded animals.

17. The axenic culture of the microorganism *Streptomyces virginiae* NRRL 12525.

18. *Streptomyces virginiae* NRRL 12525.

19. The axenic culture of *Streptomyces virginiae* NRRL 15156.

20. A pharmaceutical formulation which comprises as an active ingredient A41030 Factor A, B, C, D, E, F or G according to claim 2 or a pharmaceutically-acceptable salt thereof, associated with one or more pharmaceutically-acceptable carriers or diluents therefor.

21. A verterinary formulation which comprises as an active ingredient A41030 complex, or Factor A, B, C, D, E, F or G according to claim 2 or a pharmaceutically-acceptable salts thereof, associated with one or more pharmaceutically-acceptable carriers or diluents therefor.

**Claims for the Contracting State: AT**

1. A process for preparing A41030 complex, or Factor A, B, C, D, E, F, or G, or a pharmaceutically-acceptable salt thereof, which comprises cultivating *Streptomyces virginiae* NRRL 15156 or 12525, or an A41030-producing variant or mutant thereof, in a culture medium containing assimilable sources of carbon, nitrogen, and inorganic salts under submerged aerobic fermentation conditions.

2. A method according to claim 1 followed by separation of the complex from the culture medium.

3. A method according to claim 2, in which the factors A, B, C, D, E, F or G are isolated from the complex.

4. A process according to claim 3 for preparing A41030 factor A of structure:

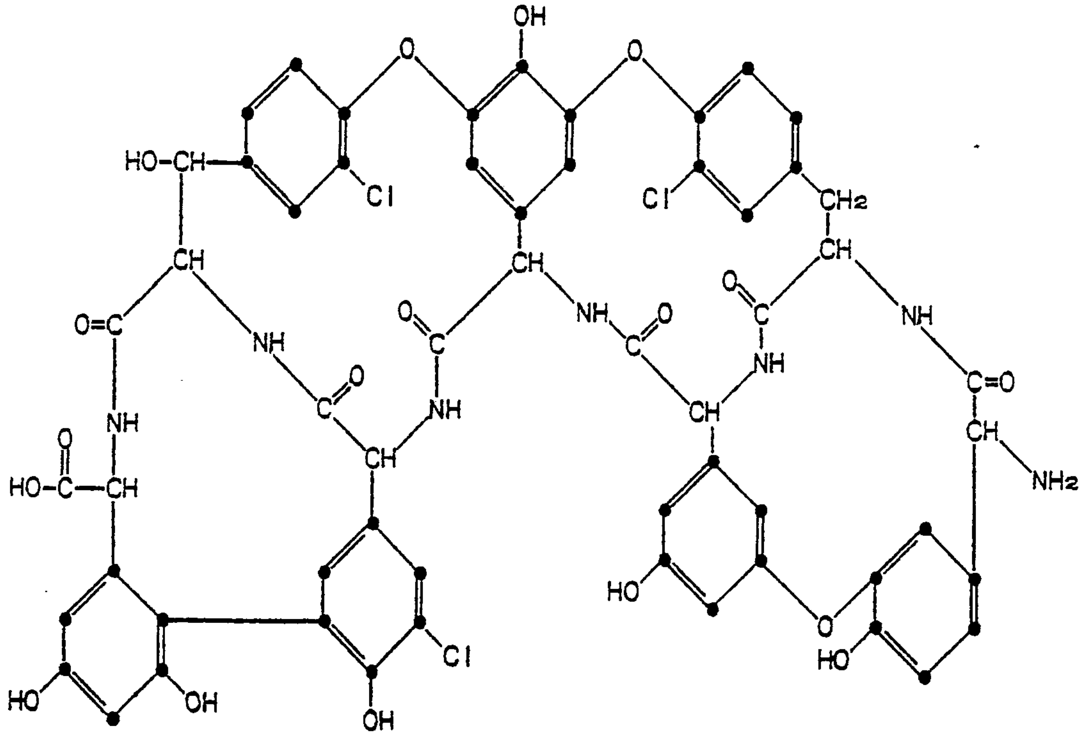

or a pharmaceutically-acceptable salt thereof.

5. A process according to claim 3 for preparing A41030 factor B of structure:

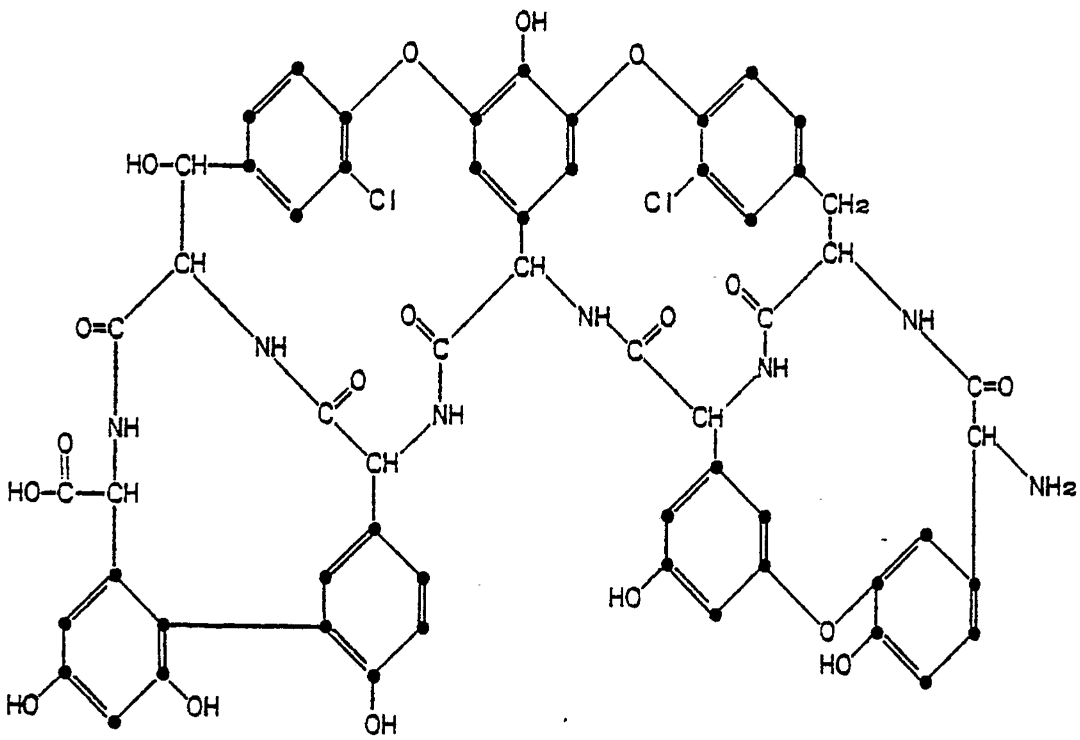

or a pharmaceutically-acceptable salt thereof.

6. A process according to claim 3 for preparing A41030 factor C of structure:

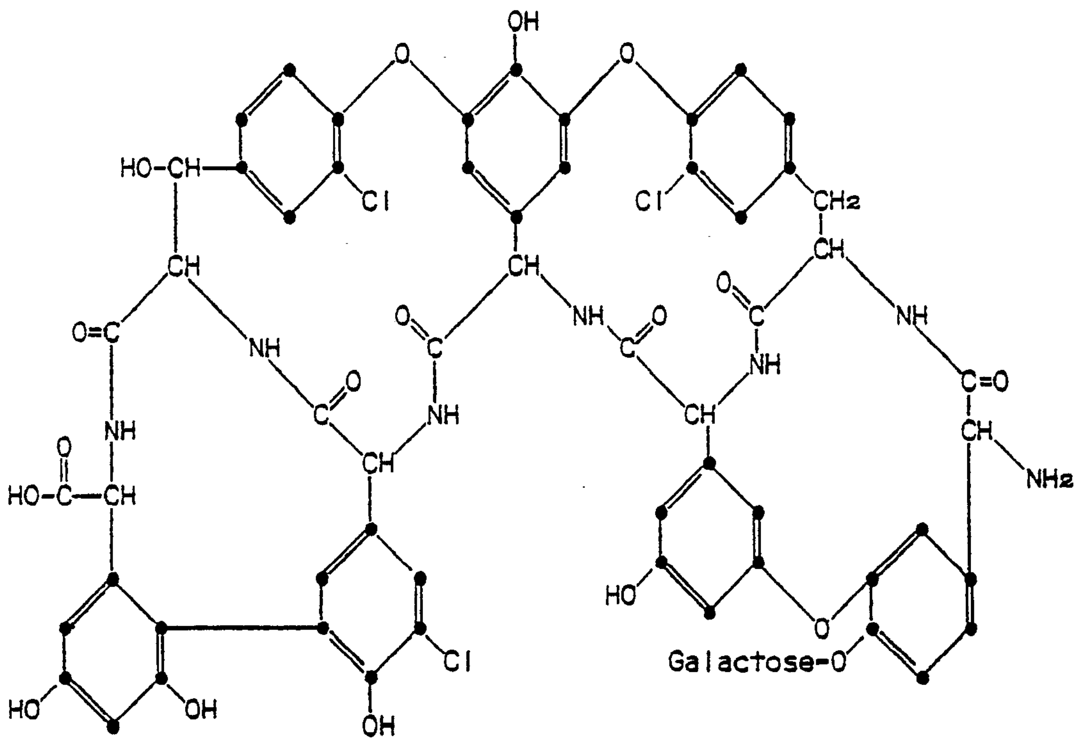

or a pharmaceutically-acceptable salt thereof.

7. A process according to claim 3 for preparing antibiotic A41030 factor D, which is a white, amorphous solid, having:

(a) an approximate elemental analysis of 54.46% carbon, 4.35% hydrogen, 7.58% nitrogen, 4.27% chlorine, and by difference, 29.34% oxygen;

(b) an observed molecular weight of about 1326, as determined by fast atom bombardment mass spectrometry;

(c) an infrared absorption spectrum having the following distinguishable maxima: 3448—3226 (strong broad), 2959 (weak), 1661 (strong), 1592 (strong), 1511 (strong), 1429 (weak), 1290 (weak), 1227 (weak), (medium), 1163 (weak), 1143 (weak), 1053 (medium), and 1010 (strong) $cm^{-1}$;

(d) ultraviolet absorption spectra with an absorption maximum, in acidic or neutral methanol:water (1:1), at 278 nm (ε 10,600) and, in basic methanol:water (1:1), at 298 nm (ε 19,900);

(e) two titratable groups in 66% aqueous dimethylformamide with $pK_a$ values of about 5.5 and 7.6;

(f) is soluble in alcohol-water mixtures, in dimethylsulfoxide, in dimethylformamide, in dimethylsulfoxide-water mixtures, in di-methylformamide-water mixtures, in dilute aqueous acid, or in dilute aqueous base; and

(g) the pharmaceutically-acceptable, non-toxic salts of said A41030 factor D.

8. A process according to claim 3 for preparing A41030 factor E of structure:

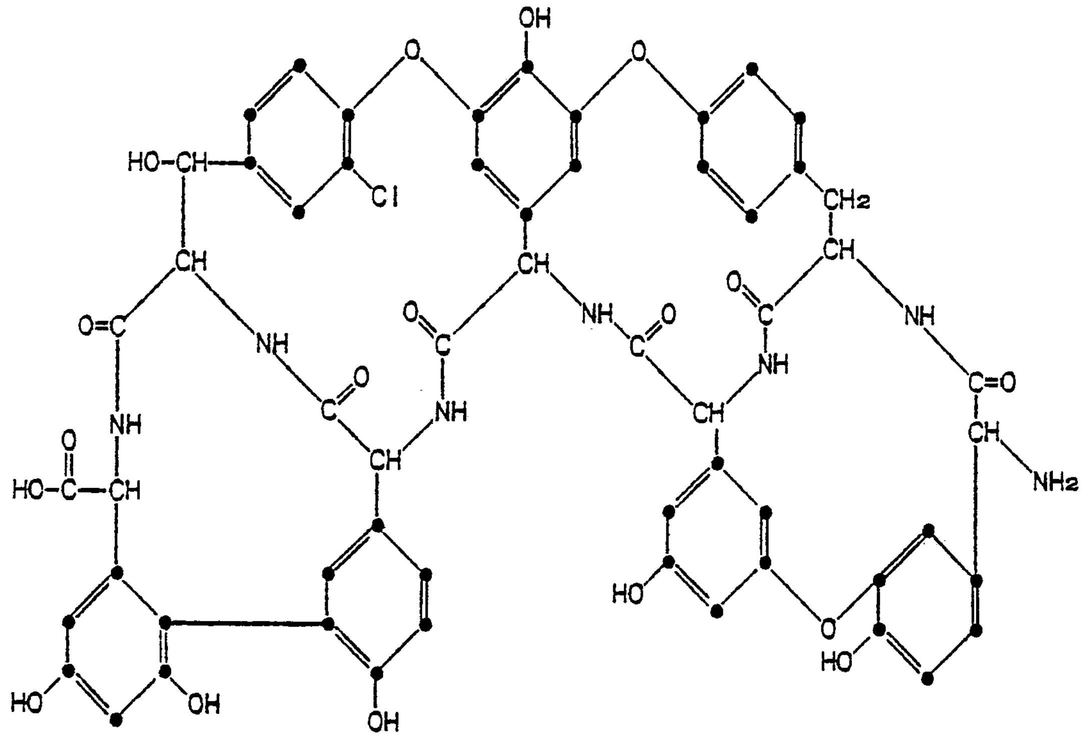

or a pharmaceutically-acceptable salt thereof.

9. A process according to claim 3 for preparing A41030 factor F of structure:

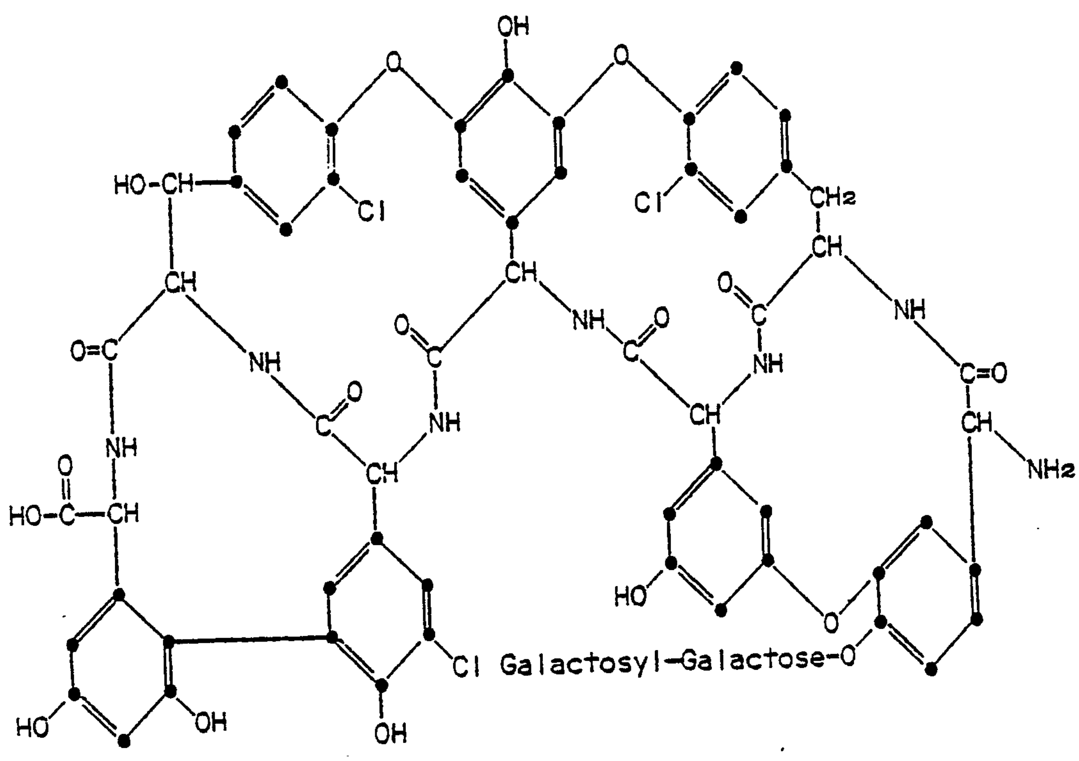

or a pharmaceutically-acceptable salt thereof.

10. A process according to claim 3 for preparing antibiotic A41030 factor G, which is a white solid having:

(a) an approximate elemental analysis of 50.02% carbon, 4.61% hydrogen, 4.74% chlorine, 6.11% nitrogen, and 30.70% oxygen;

(b) an observed molecular weight of about 1688 as determined by fast atom bombardment mass spectrometry;

(c) an infrared absorption spectrum having the following distinguishable absorption maxima: 3320 (Very broad, strong), 2975 (sharp, weak), 2920 (sharp, weak), 1659 (normal, strong), 1594 (broad, strong), 1512 (sharp, strong), 1492 (shoulder), 1430 (sharp, weak), 1386 (broad, weak), 1337 (broad, weak), 1308 (sharp, weak), 1264 (sharp, weak), 1230 (broad, medium), 1145 (broad, medium), 1077 (sharp, medium), 1062 (sharp, medium), 1014 (sharp, medium), and 846 (broad, medium) $cm^{-1}$;

(d) ultraviolet absorption spectra with an absorption maximum, in acidic or neutral methanol:water (1:1), at 278 nm (ε 15,000) and, in basic methanol:water (1:1), at 298 nm (ε 18,000);

(e) two titratable groups in 66% aqueous dimethylformamide with $pK_a$ values of about 5.4 and 7.0;

(f) is soluble in alcohol-water mixtures, in dimethylsulfoxide, in dimethylformamide, in dimethylsulfoxide-water mixtures, in dimethylformamide-water mixtures, in dilute aqueous acid, or in dilute aqueous base; and

(g) the pharmaceutically-acceptable, non-toxic salts of said A41030 factor G.

11. A41030 antibiotic complex or Factor A, B, C, D, E, F or G obtainable by the process of Claim 1, or a pharmaceutically-acceptable salt thereof, whenever prepared by a process according to any one of claims 1 to 10.

12. The axenic culture of the microorganism *Streptomyces virginiae* NRRL 12525.

13. *Streptomyces virginiae* NRRL 12525.

14. The axenic culture of *Streptomyces virginiae* NRRL 15156.

15. The use of A41030 complex or Factor A, B, C, D, E, F or G obtainable by the process of Claim 1, or a pharmaceutically-acceptable salt thereof, for use in improving milk production in a lactating ruminant.

16. The use of A41030 complex or Factor A, B, C, D, E, F or G obtainable by the process of Claim 1, or a pharmaceutically-acceptable salt thereof, for use in increasing feed utilization efficiency in a ruminant animal.

17. The use of A41030 complex or Factor A, B, C, D, E, F or G obtainable by the process of Claim 1, or a pharmaceutically-acceptable salt thereof, as a growth promoter in warm-blooded animals.

**Patentansprüche für die Vertragsstaaten: BE CH DE GB IT LI LU NL SE**

1. Verfahren zur Herstellung des Antibiotikumkomplexes A 41030 oder seiner Faktoren A, B, C, D, E, F und G, dadurch gekennzeichnet, daß Streptomyces virginiae NRRL 15156 oder 12525 oder eine A 41030 produzierende Variante oder Mutante hiervon in einem Kulturmedium, das assimilierbare Quellen für Kohlenstoff, Stickstoff und anorganische Salze enthält, unter submersen aeroben Fermentationsbedingungen gezüchtet wird.

2. Komplex von A 41030 oder die Faktoren A, B, C, D, E, F und G hiervon, erhältlich nach dem Verfahren von Anspruch 1, oder ein pharmazeutisch annehmbares Salz hiervon.

3. Faktor A von A 41030 mit der Struktur

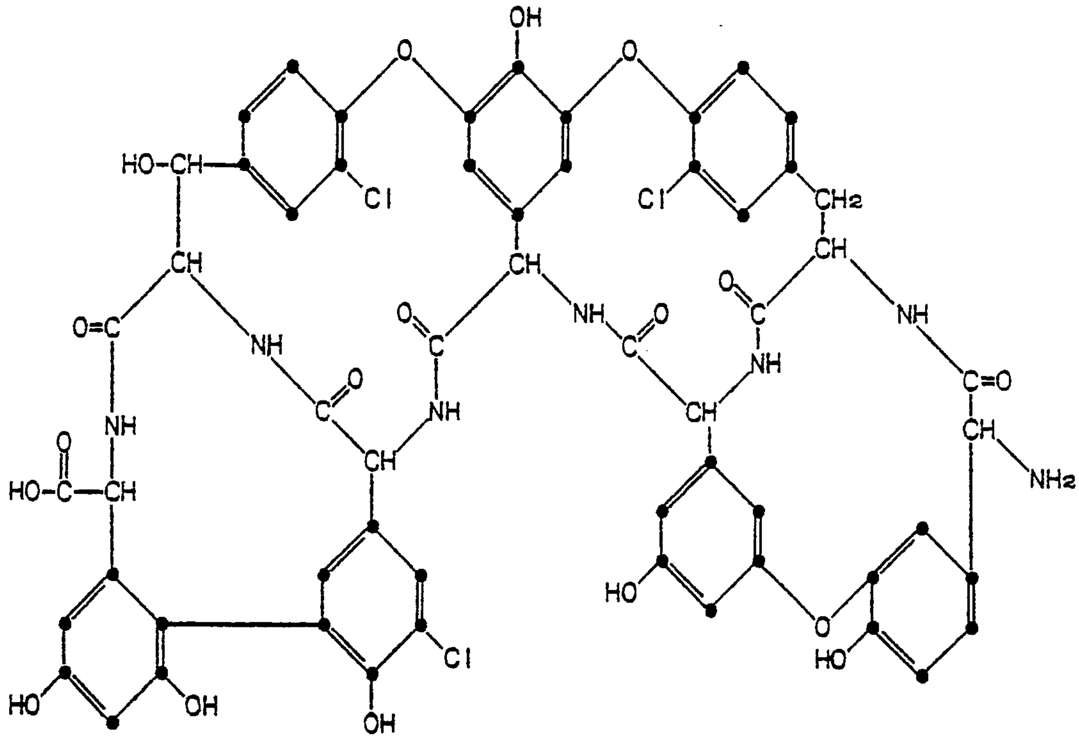

oder ein pharmazeutisch annehmbares Salz hiervon.

4. Faktor B von A 41030 mit der Struktur

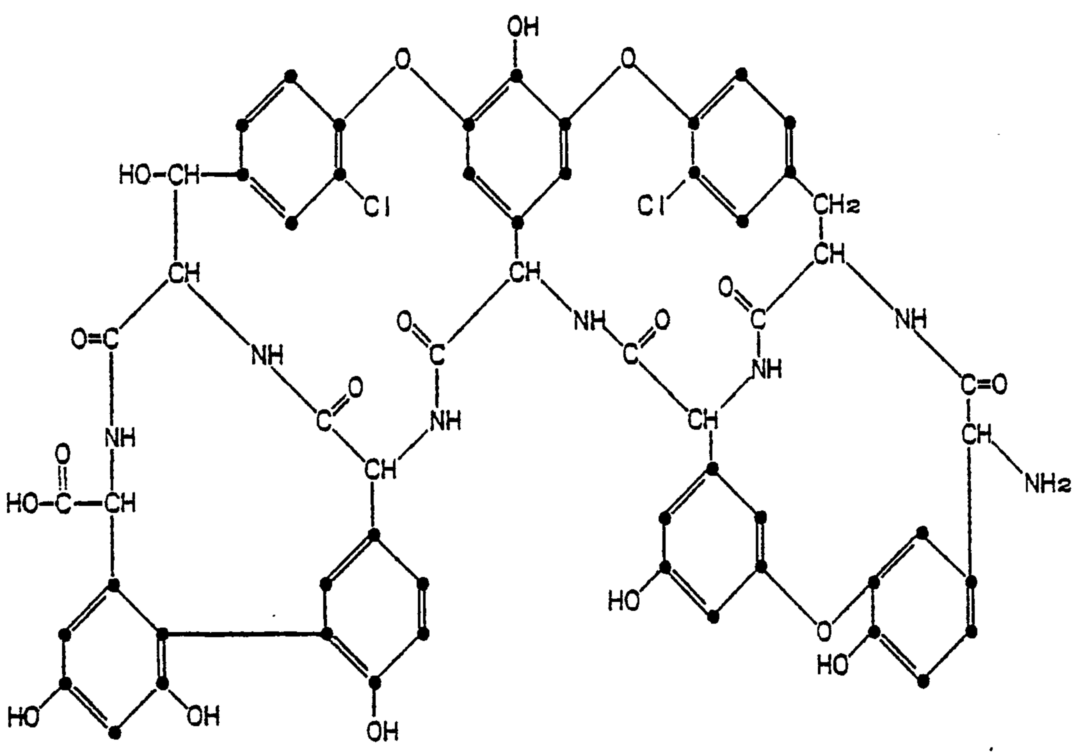

oder ein pharmazeutisch annehmbares Salz hiervon.

5. Faktor C von A 41030 mit der Struktur

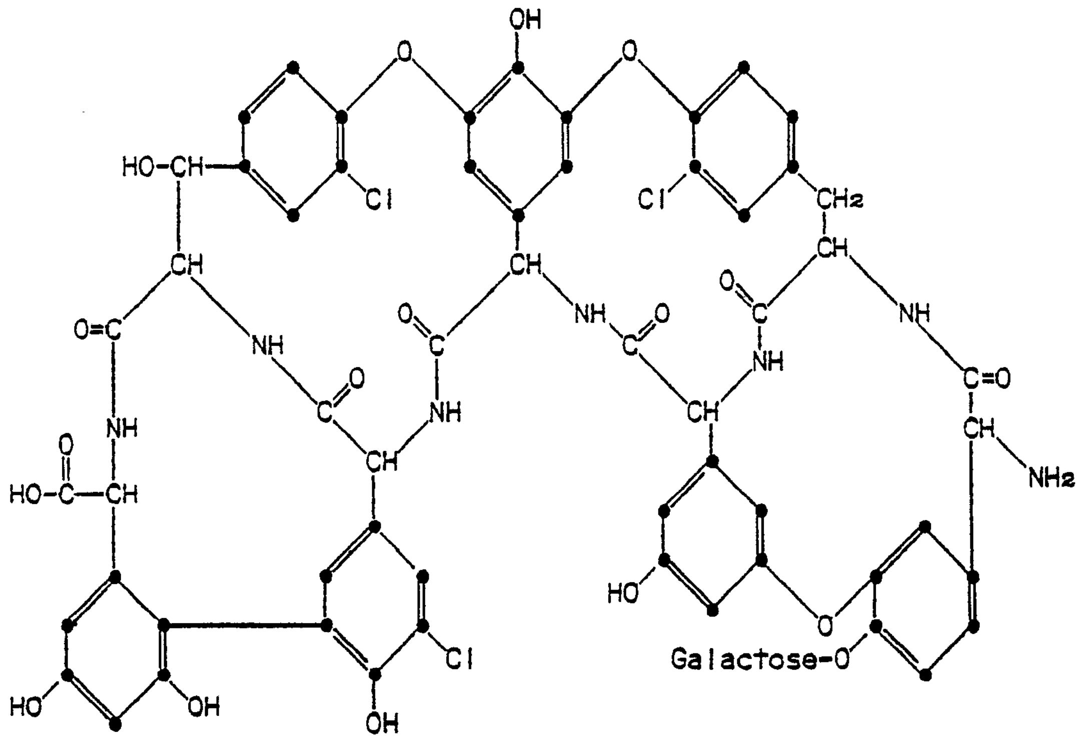

oder ein pharmazeutisch annehmbares Salz hiervon.

6. Faktor D des Antibiotikums A 41030, der ein weißer amorpher Feststoff ist und folgende weitere physikalische Daten aufweist:

(a) eine ungefähre Elementaranalyse von 54,46% Kohlenstoff, 4,35% Wasserstoff, 7,58% Stickstoff, 4,27% Chlor und — durch Differenzbildung — 29,35% Sauerstoff,

(b) ein durch Massenspektroskopie mittels Bombardierung mit schnellen Atomen bestimmtes Molekulargewicht von etwa 1326,

(c) ein Infrarotabsorptionsspektrum mit folgenden unterscheidbaren Maxima: 3448 bis 3226 (stark, breit), 2959 (schwach), 1661 (stark), 1592 (stark), 1511 (stark), 1429 (schwach), 1290 (schwach), 1227 (schwach), 1212 (mittel), 1163 (schwach), 1143 (schwach), 1053 (mittel) und 1010 (stark) $cm^{-1}$,

(d) Ultraviolettabsorptionsspektren mit einem Absorptions-maximum in sauren oder neutralen Gemischen von Methanol und Wasser (1:1) bei 278 nm ($\varepsilon$ = 10600) und in basischen Gemischen von Methanol und Wasser (1:1) bei 298 nm ($\varepsilon$ = 19900),

(e) zwei titrierbare Gruppen in 66-prozentigem wäßrigem Dimethylformamid mit $pK_a$-Werten von etwa 5,5 und 7,6,

(f) Löslichkeit in Gemischen von Alkohol und Wasser, in Dimethylsulfoxid, in Dimethylsulfoxid in Gemischen von Dimethylsulfoxid und Wasser, in Gemischen von Dimethylformamid und Wasser, in verdünnter wäßriger Säure oder in verdünnter wäßriger Base, und

(g) die pharmazeutisch annehmbaren, nichttoxischen Salze des Faktors D des Antibiotikums A 41030.

7. Faktor E von A 41030 mit der Struktur

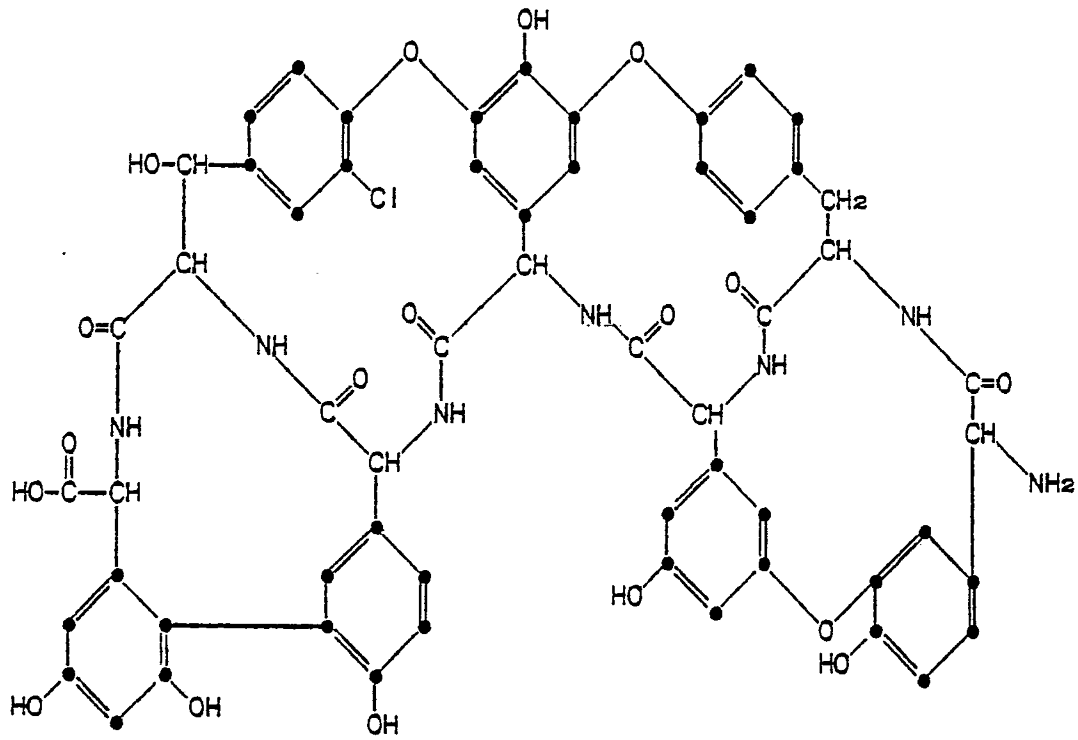

oder ein pharmazeutisch annehmbares Salz hiervon.

8. Faktor F von A 41030 mit der Struktur

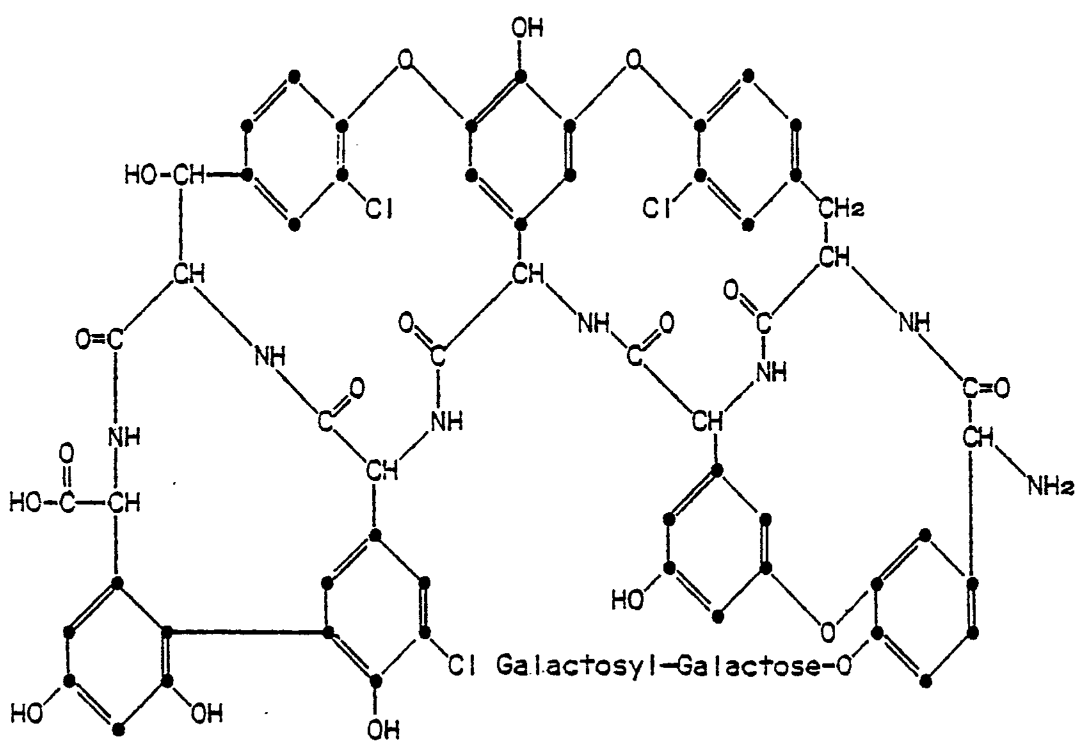

oder ein pharmazeutisch annehmbares Salz hiervon.

9. Faktor G des Antibitikums A 41030, der ein weißer Feststoff ist und folgende weitere physikalische Daten aufweist:

(a) eine ungefähre Elementaranalyse von 50,02% Kohlenstoff, 4,61% Wasserstoff, 4,74% Chlor, 6,11% Stickstoff und 30,70% Sauerstoff,

(b) ein durch Massenspektroskopie mittels Bombardierung mit schnellen Atomen bestimmtes Molekulargewicht von etwa 1688,

(c) ein Infrarotabsorptionsspektrum mit folgenden unterscheidbaren Maxima: 3320 (sehr breit, stark), 2975 (scharf, schwach), 2920 (scharf, schwach), 1659 (normal, stark), 1594 (breit, stark), 1512 (scharf, stark), 1492 (Schulter), 1430 (scharf, schwach), 1386 (breit, schwach), 1137 (breit, schwach), 1308 (scharf, schwach), 1264 (scharf, schwach), 1230 (breit, mittel), 1145 (breit, mittel), 1077 (scharf, mittel), 1062 (scharf, mittel), 1014 (scharf, mittel) und 846 (breit, mittel) $cm^{-1}$.

(d) Ultraviolettabsorptionsspektren mit einem Absorptionsmaximum in sauren oder neutralen Gemischen von Methanol und Wasser (1:1) bei 278 nm ($\varepsilon$ = 15000) und in basischen Gemischen von Methanol und Wasser (1:1) bei 298 nm ($\varepsilon$ = 18000),

(e) zwei titrierbare Gruppen in 66-prozentigem wäßrigem Dimethylformamid mit $pK_a$-Werten von etwa 5,4 und 7,0,

(f) Löslichkeit in Gemischen von Alkohol und Wasser, in Dimethylsulfoxid, in Dimethylformamid, in Gemischen von Dimethylsulfoxid und Wasser, in Gemischen von Dimethylformamid und Wasser, in verdünnter wäßriger Säure oder in vedünnter wäßriger Base, und

(g) die pharmazeutisch annehmbaren, nichttoxischen Salze des Faktors G des Antibiotikums A 41030.

10. Verfahren nach Anspruch 1, dadurch gekennzeichnet, daß der Komplex aus dem Kulturmedium abtrennt wird.

11. Verfahren nach Anspruch 10, dadurch gekennzeichnet, daß die Faktoren A, B, C, D, E, F oder G aus dem Komplex isoliert werden.

12. Komplex von A 41030 oder Faktoren A, B, C, D, E, F oder G nach Anspruch 2 oder ein pharmazeutisch annehmbares Salz hiervon zur Anwendung bei der veterinären oder pharmazeutischen chemotherapeutischen Behandlung.

13. Komplex von A 41030 oder Faktoren A, B, C, D, E, F oder G nach Anspruch 2 oder ein pharmazeutisch annehmbares Salz hiervon zur Anwendung bei der Verbesserung der Milchproduktion milchgebender Wiederkäuer.

14. Futtervormischung, dadurch gekennzeichnet, daß sie den Komplex A 41030 oder die Faktoren A, B, C, D, E, F oder G von A 41030 nach Anspruch 2 oder ein pharmazeutisch annehmbares Salz hiervon enthält.

15. Komplex von A 41030 oder die Faktoren A, B, C, D, E, F oder G nach Anspruch 2 oder ein pharmazeutisch annehmbares Salz hiervon zur Anwendung bei der Erhöhung der Futterverwertung von Wiederkäuern.

16. Komplex von A 41030 oder die Faktoren A, B, C, D, E, F oder G nach Anspruch 2 oder ein pharmmazeutisch annehmbares Salz hiervon zur Anwendung für die Förderung des Wachstums bei warmblütigen Tieren.

17. Axinische Kultur des Mikroorganismus Streptomyces virginiae NRRL 12525.

18. Streptomyces virginiae NRRL 12525.

19. Axinische Kultur von Streptomyces virginiae NRRL 15156.

20. Pharmazeutische Formulierung, dadurch gekennzeichnet, daß sie die Faktoren A, B, C, D, E, F oder G von A 41030 nach Ansprüche 2 oder ein pharmazeutisch annehmbares Salz hiervon als Wirkstoff in Verbindung mit einem oder mehreren pharmazeutisch annehmbaren Trägern oder Verdünnungsmitteln hierfür enthält.

21. Veterinäre Formulierung, dadurch gekennzeichnet, daß sie den Komplex A 41030 oder die Faktoren A, B, C, D, E, F oder G nach Anspruch 2 oder ein pharmazeutisch annehmbares Salz hiervon als Wirkstoff in Verbindung mit einem oder mehreren pharmazeutisch annehmbaren Trägern oder Verdünnungsmitteln hierfür enthält.

**Patentansprüche für den Vertragsstaat: AT**

1. Verfahren zur Herstellung des Antibiotikumkomplexes A 41030 oder seiner Faktoren A, B, C, D, E, F und G eines pharmaeutisch annehmbaren Salzes hiervon, dadurch gekennzeichnet, daß Streptomyces virginiae NRRL 15156 oder 12525 oder ein A 41030 produzierende Variante oder Mutante hiervon in einem Kulturmedium, das assimilierbare Quellen für Kohlenstoff, Stickstoff und anorganische Salz enthält, unter submersen aeroben Fermentationsbedingungen gezüchtet wird.

2. Verfahren nach Anspruch 1, dadurch gekennzeichnet, daß der Komplex vom Kulturmedium abgetrennt wird.

3. Verfahren nach Anspruch 2, dadurch gekennzeichnet, daß die Faktoren A, B, C, D, E, F oder G aus dem Komplex isoliert werden.

4. Verfahren nach Anspruch 3, dadurch gekennzeichnet, daß der Faktor A von A 41030 mit der Struktur

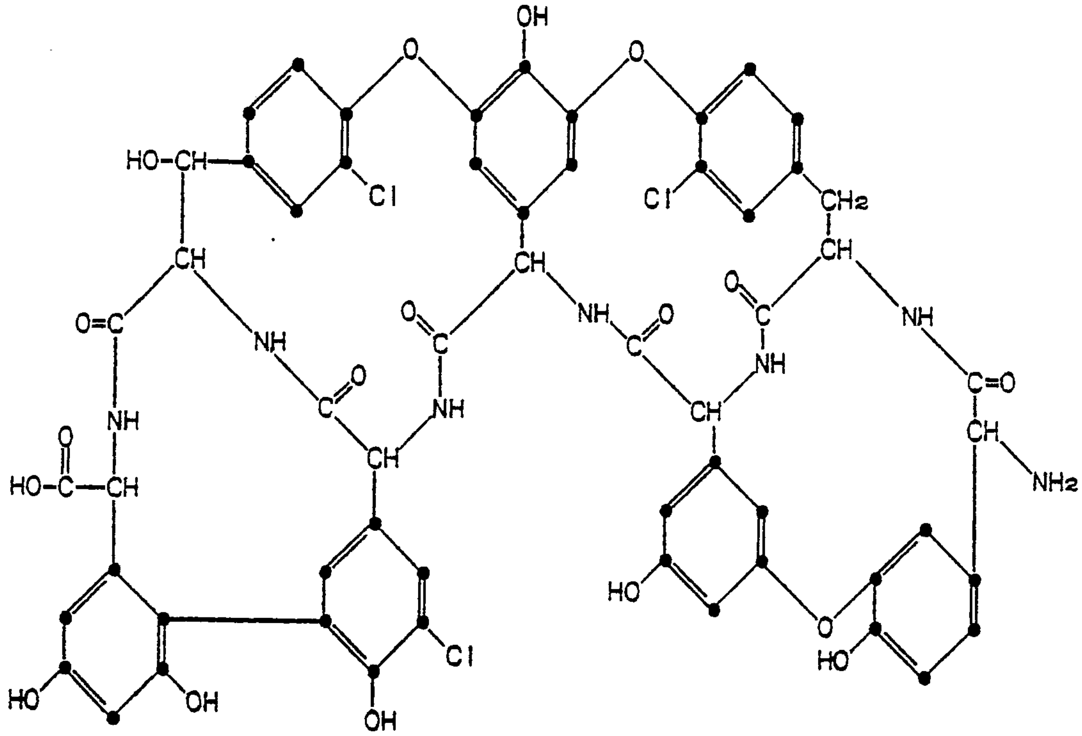

oder ein pharmazeutisch annehmbares Salz hiervon hergestellt wird.

5. Verfahren nach Anspruch 3, dadurch gekennzeichnet, daß der Faktor B von A 41030 mit der Struktur

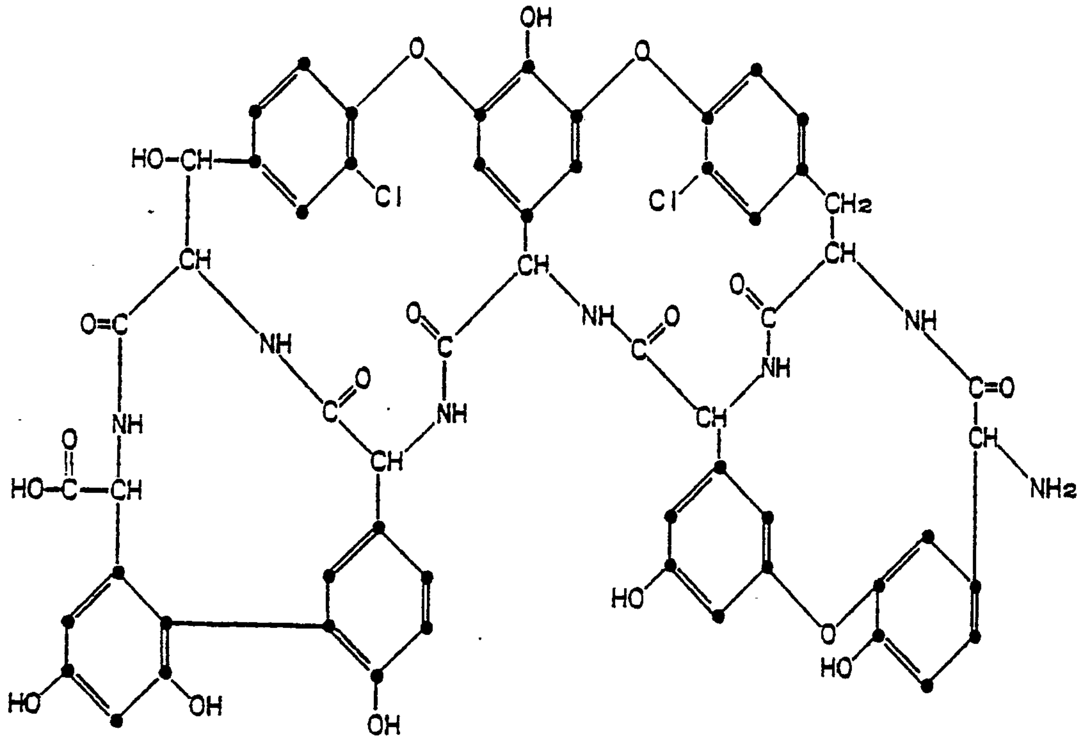

oder ein pharmazeutisch annehmbares Salz hiervon hergestellt wird.

6. Verfahren nach Anspruch 3, dadurch gekennzeichnet, daß der Faktor C von A 41030 mit der Struktur

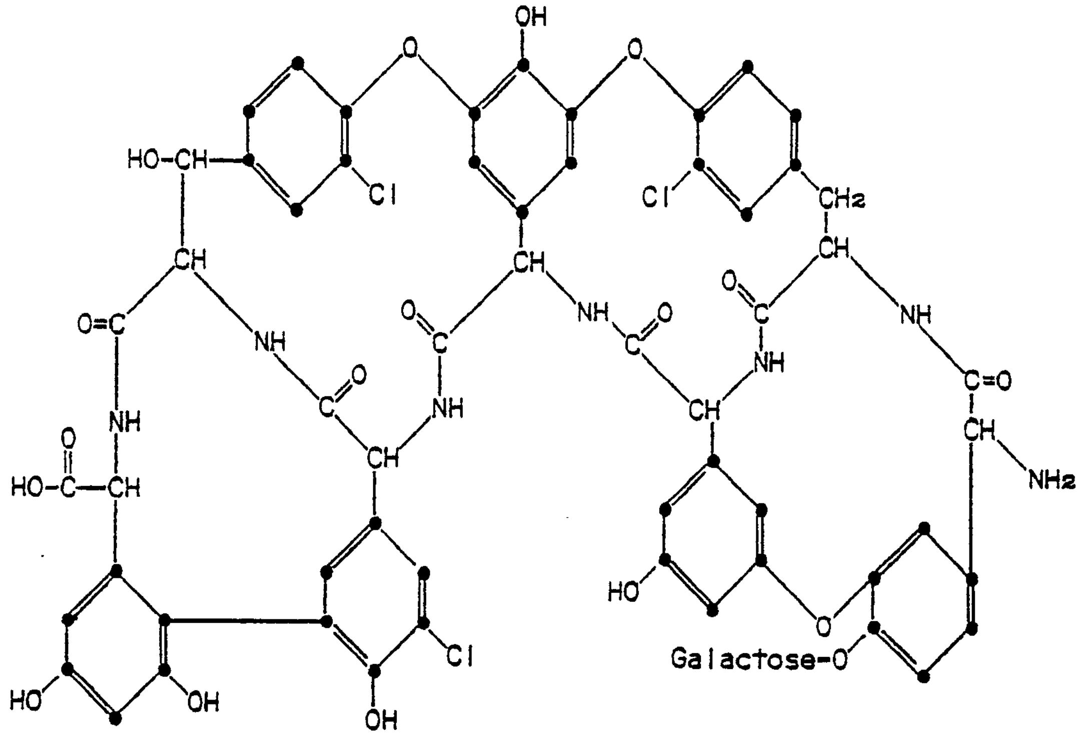

oder ein pharmazeutisch annehmbares Salz hiervon hergestellt wird.

7. Verfahren nach Anspruch 3, dadurch gekennzeichnet, daß der Faktor D des Antibiotikums A 41030 hergestellt wird, der ein weißer amorpher Feststoff ist und folgende weitere physikalische Daten aufweist:

(a) eine ungefähre Elementaranalyse von 54,46% Kohlenstoff, 4,35% Wasserstoff, 7,58% Stickstoff, 4,27% Chlor und — durch Differenzbildung — 29,35% Sauerstoff,

(b) ein durch Massenspektroskopie mittels Bombardierung mit schnellen Atomen bestimmtes Molekulargewicht von etwa 1326,

(c) ein Infrarotabsorptionsspektrum mit folgenden unterscheidbaren Maxima: 3448 bis 3226 (stark, breit), 2959 (schwach), 1661 (stark), 1592 (stark), 1511 (stark), 1429 (schwach), 1290 (schwach), 1227 (schwach), 1212 (mittel), 1163 (schwach), 1143 (schwach), 1053 (mittel) und 1010 (stark) $cm^{-1}$,

(d) Ultraviolettabsorptionsspektren mit einem Absorptionsmaximum in sauren oder neutralen Gemischen von Methanol und Wasser (1:1) bei 278 nm ($\varepsilon$ = 10600) und in basischen Gemischen von Methanol und Wasser (1:1) bei 298 nm ($\varepsilon$ = 19900),

(e) zwei titrierbare Gruppen in 66-prozentigem wäßrigem Dimethylformamid mit $pK_a$-Werten von etwa 5,5 und 7,6,

(f) Löslichkeit in Gemischen von Alkohol und Wasser, in Dimethylsulfoxid, in Dimethylformamid, in Gemischen von in Dimethylsulfoxid und Wasser, in Gemischen von Dimethylformamid und Wasser, in verdünnter wäßriger Säure oder in verdünnter wäßriger Base, und

(g) die pharmazeutisch annehmbaren, nichttoxischen Salze des Faktors D des Antibiotikums A 41030.

8. Verfahren nach Anspruch 3, dadurch gekennzeichnet, daß der Faktor E von A 41030 mit der Struktur

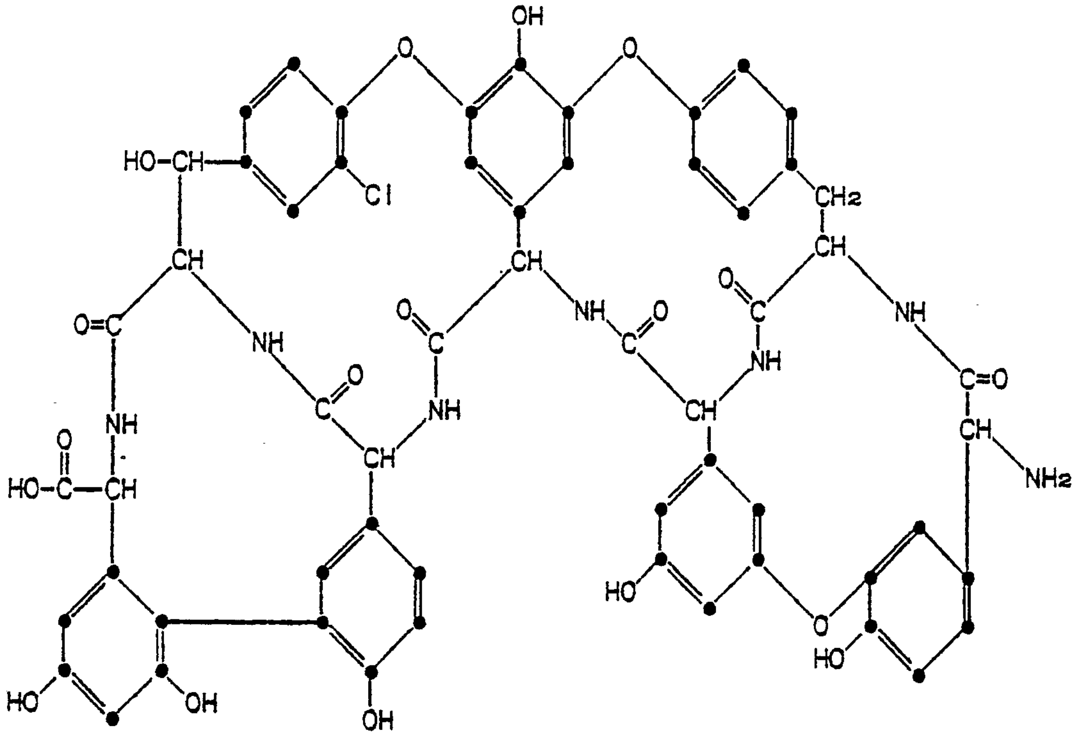

oder ein pharmazeutisch annehmbares Salz hiervon hergestellt wird.

9. Verfahren nach Anspruch 3, dadurch gekennzeichnet, daß der Faktor F von A 41030 mit der Struktur

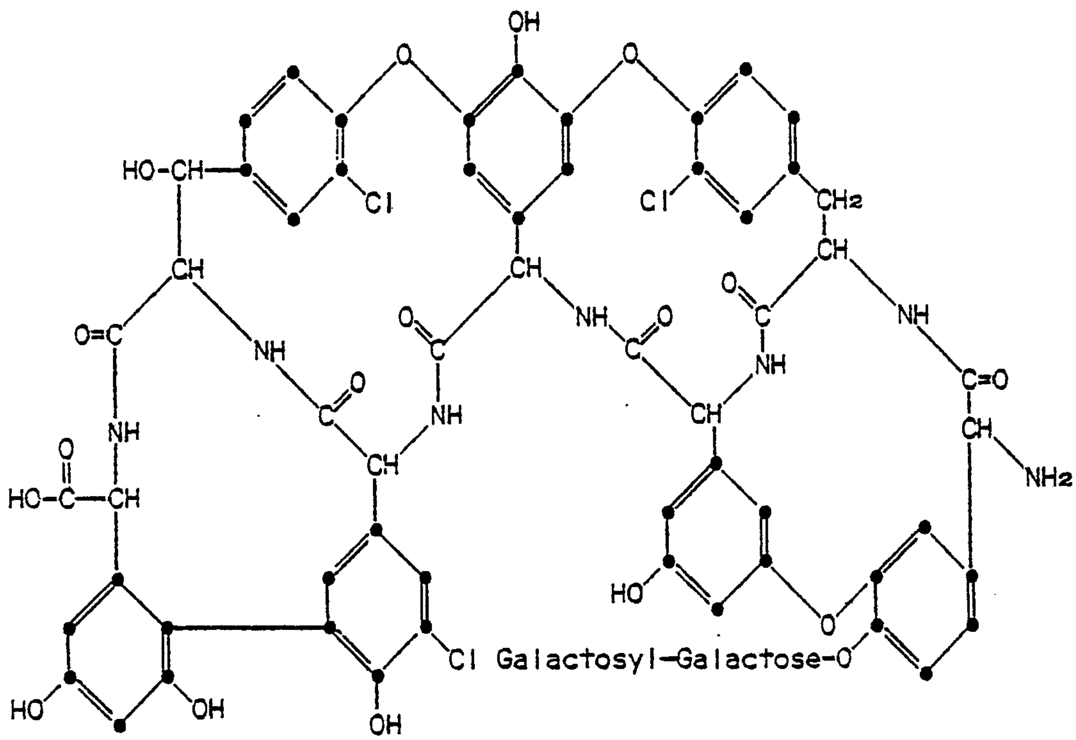

oder ein pharmazeutisch annehmbares Salz hiervon hergestellt wird.

10. Verfahren nach Anspruch 3, dadurch gekennzeichnet, daß der Faktor G des Antibiotikums A 41030 hergestellt wird, der ein weißer Feststoff ist und folgende weitere physikalische Daten aufweist:

(a) eine ungefähre Elementaranalyse von 50,02% Kohlenstoff, 4,61% Wasserstoff, 4,74% Chlor, 6,11% Stickstoff und 30,70% Sauerstoff,

(b) ein durch Massenspektroskopie mittels Bombardierung mit schnellen Atomen bestimmtes Molekulargewicht von etwa 1688,

(c) ein Infrarotabsorptionsspektrum mit folgenden unterscheidbaren Maxima: 3320 (sehr breit, stark), 2975 (scharf, schwach), 2920 (scharf, schwach), 1659 (normal, stark), 1594 (breit, stark), 1512 (scharf, stark), 1492 (Schulter), 1430 (scharf, schwach), 1386 (breit, schwach), 1137 (breit, schwach), 1308 (scharf, schwach), 1264 (scharf, schwach), 1230 (breit, mittel), 1145 (breit, mittel), 1077 (scharf, mittel), 1062 (scharf, mittel), 1014 (scharf, mittel) und 846 (breit, mittel) $cm^{-1}$.

(d) Ultraviolettabsorptionsspektren mit einem Absorptionsmaximum in sauren oder neutralen Gemischen von Methanol und Wasser (1:1) bei 278 nm ($\varepsilon$ = 15000) und in basischen Gemischen von Methanol und Wasser (1:1) bei 298 nm ($\varepsilon$ = 18000),

(e) zwei titrierbare Gruppen in 66-prozentigem wäßrigem Dimethylformamid mit $pK_a$-Werten von etwa 5,4 und 7,0,

(f) Löslichkeit in Gemischen von Alkohol und Wasser, in Dimethylsulfoxid, in Dimethylsulfoxid, in Dimethylformamid, in Gemischen von Dimethylsulfoxid und Wasser, in Gemischen von Dimethylformamid und Wasser, in verdünnter wäßriger Säure oder in vedünnter wäßriger Base, und

(g) die pharmazeutisch annehmbaren, nichttoxischen Salze des Faktors G des Antibiotikums A 41030.

11. Antibiotikumkomplex von A 41030 oder die Faktoren A, B, C, D, E, F oder G, erhältlich nach dem Verfahren von Anspruch 1, oder ein pharmazeutisch annehmbares Salz hiervon, dadurch gekennzeichnet, daß sie nach einem Verfahren gemäß irgendeinem der Ansprüche 1 bis 10 hergestellt worden sind.

12. Axinische Kultur des Mikroorganismus Streptomyces virginiae NRRL 12525.

13. Streptomyces virginiae NRRL 12525.

14. Axinische Kultur von Streptomyces virginiae NRRL 15156.

15. Verwendung des Komplexes von A 41030 oder der Faktoren A, B, C, D, E, F oder G, erhältlich nach dem Verfahren von Anspruch 1, oder eines pharmazeutisch annehmbaren Salzes hiervon zur Verbesserung der Milchproduktion bei einem milchgebwnden Wiederkäuer.

16. Verwendung des Komplexes von A 41030 oder der Faktoren A, B, C, D, E, F oder G, erhältlich nach dem Verfahren von Anspruch 1, oder eines pharmazeutisch annehmbaren Salzes hiervon zur Erhöhrung der Futtervertung bei einem Wiederkäur.

17. Verwendung des Komplexes von A 41030 oder der Faktoren A, B, C, D, E, F oder G, erhältlich nach dem Verfahren von Anspruch 1, oder eines pharmazeutisch annehmbaren Salzes hiervon zur Förderung des Wachstrums bei warmblütigen Tieren.

**Revendications pour les Etats contractants: BE CH DE GB IT LI LU NL SE**

1. Procédé de préparation du complexe antibiotique A41030 ou du facteur A, B, C, D, E, F, ou G, ce procédé consistant à cultiver Streptomyces virginiae NRRL 15156 ou 12525, ou un de ses variants ou encore un de ses mutants producteur de A410301, dans un milieu de culture contenant des sources assimilables de carbone, d'azote et de sels inorganiques, dans des conditions de fermentation aérobie submergée.

2. Complexe A41030, ou un de ses facteurs A, B, C, D, E, F, ou G, que l'on peut obtenir par le procédé selon la revendication 1, ou encore un de leurs sels pharmaceutiquement acceptables.

3. Antibiotique A41030 facteur A de structure:

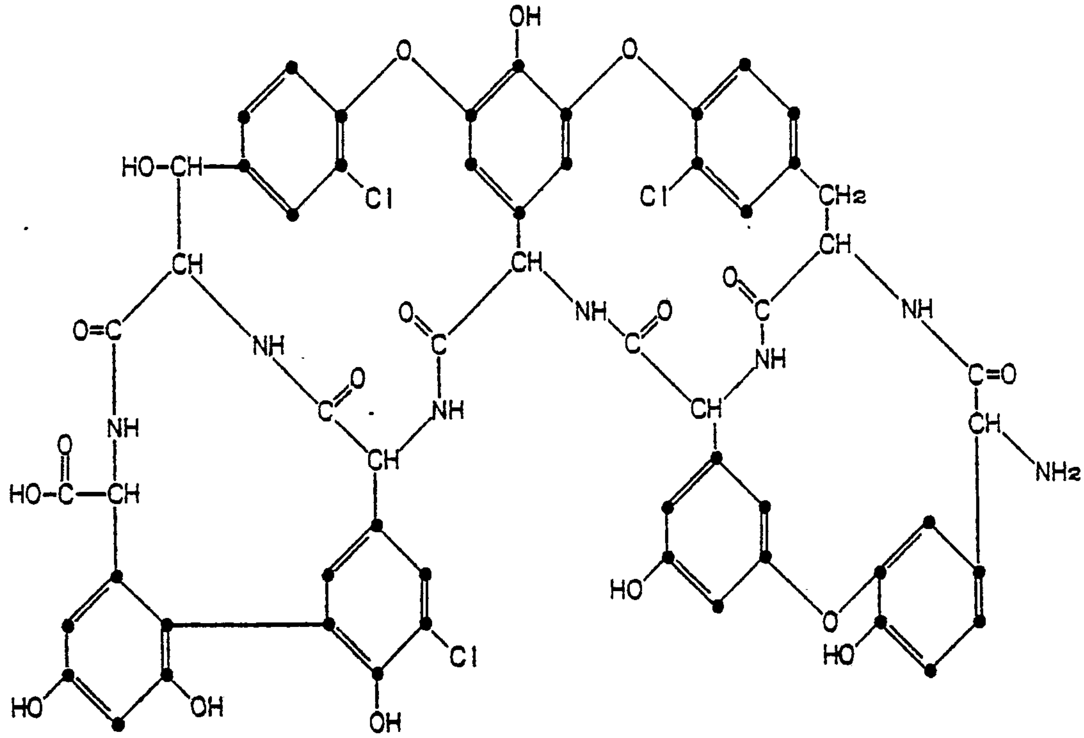

ou un de ses sels pharmaceutiquement acceptables.

4. Antibiotique A41030 facteur B de structure:

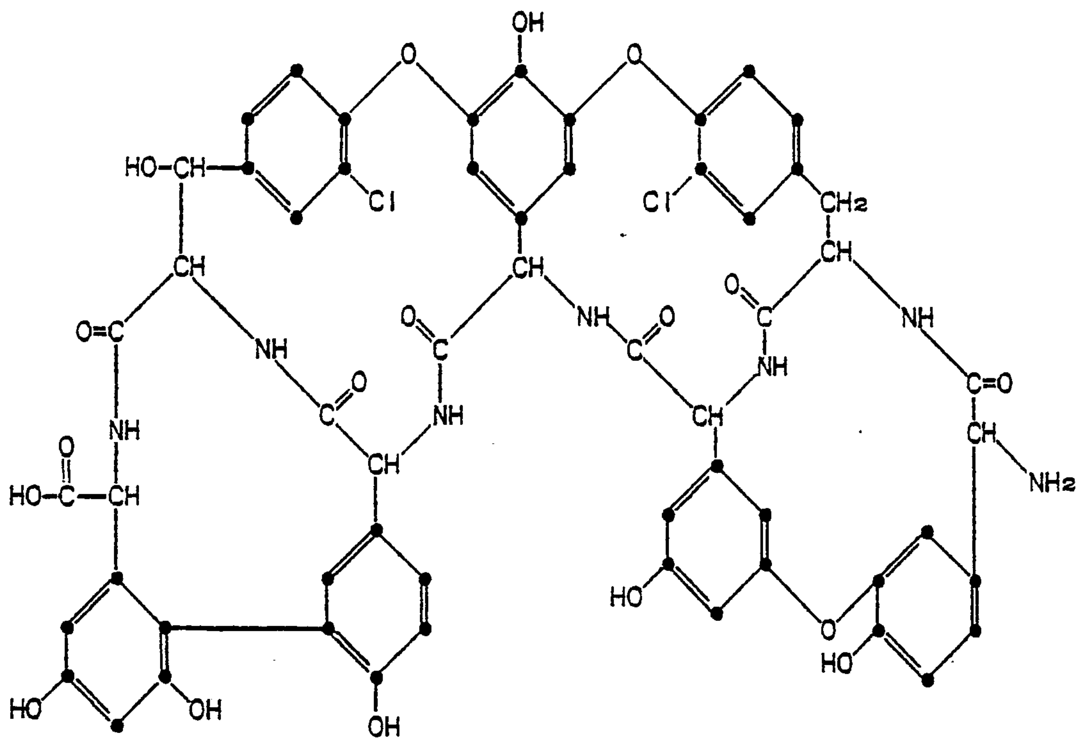

ou un de ses sels pharmaceutiquement acceptables.

5. Antibiotique A41030 facteur C de structure:

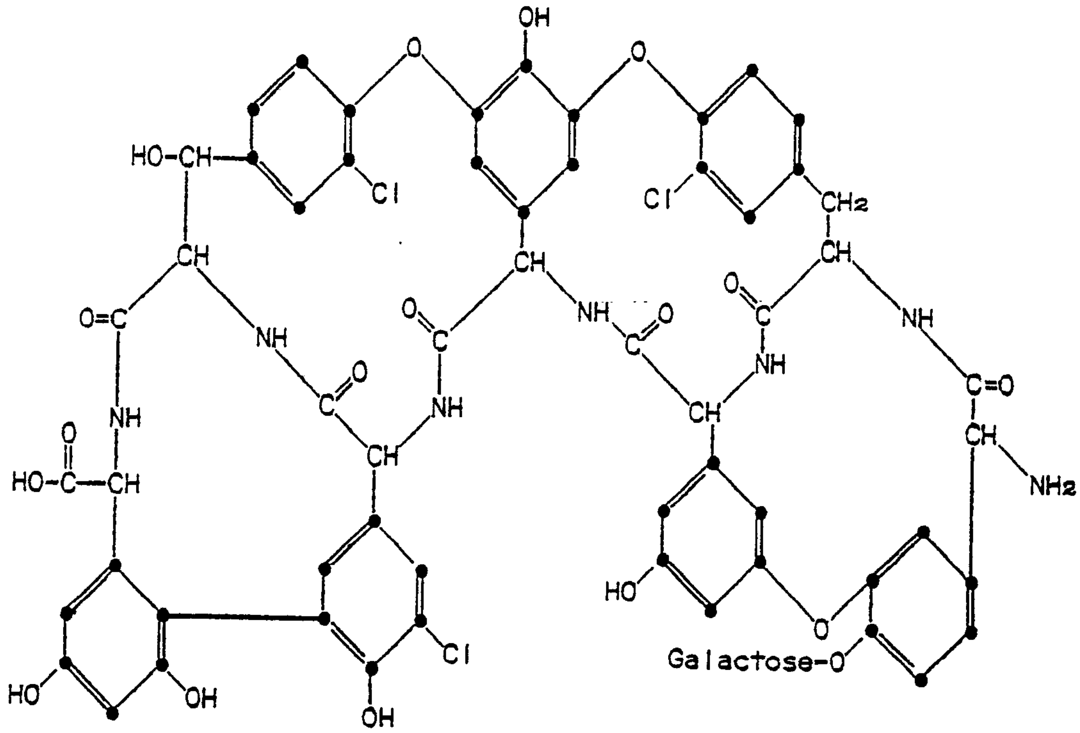

ou un de ses sels pharmaceutiquement acceptables.

6. Antibiotique A41030 facteur D, à savoir un solide blanc, amorphe ayant:

(a) une analyse élémentaire approximative de 54,46% de carbone, 4,35% d'hydrogène, 7,58% d'azote, 4,27% de chlore et, par différence, 29,34% d'oxygène;

(b) un poids moléculaire observé d'environ 1326, tel qu'il a été déterminé par spectrométrie de masse, via bombardement d'atomes rapides;

(c) un spectre d'absorption des rayons infrarouges ayant les maxima que l'on peut distinguer suivants: 3448—3226 (fort, large), 2959 (faible), 1661 (fort), 1592 (fort), 1511 (fort), 1429 (faible), 1290 (faible), 1227 (faible), 1212 (moyen), 1163 (faible), 1143 (faible), 1053 (moyen), et 1010 (fort $cm^{-1}$;

(d) des spectres d'absorption de l'ultraviolet avec une absorption maximale, dans un mélange méthanol:eau (1:1), dans des conditions acides ou basiques, à 278 nm (ε 10.600) et, dans un mélange méthanol:eau (1:1), dans des conditions basiques, à 298 nm (ε 19.900);

(e) deux groupes titrables dans du diméthylformamide aqueux à 66%, avec des valeurs $pK_a$ d'environ 5,5 et 7,6;

(f) tout étant soluble dans des mélanges alcool-eau, dans du diméthylsulfoxyde, dans du diméthylformamide, dans des mélanges diméthylsulfoxyde-eau, dans des mélanges diméthylformamide-eau, dans de l'acide aqueux dilué, ou dans une base aqueuse diluée; ainsi que

(g) les sels non toxiques, et pharmaceutiquement acceptables de cet antibiotique A41030 facteur D.

7. Antibiotique A41030 facteur E de structure:

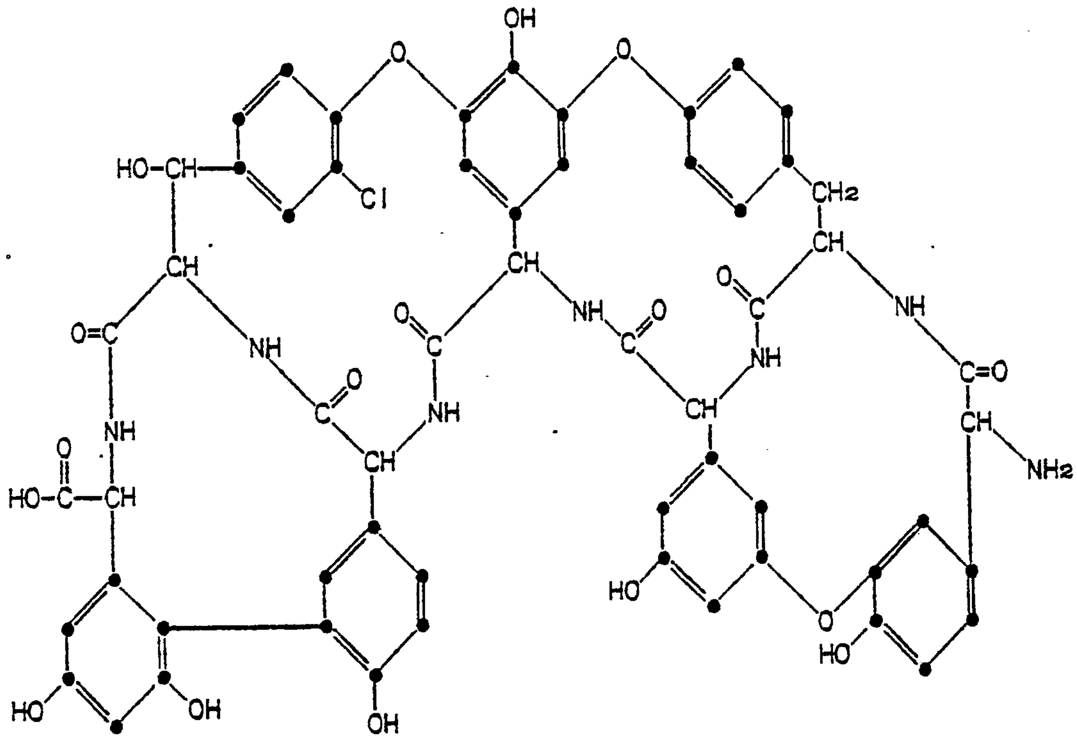

ou un de ses sels pharmaceutiquement acceptables.

8. Antibiotique A41030 facteur F de structure:

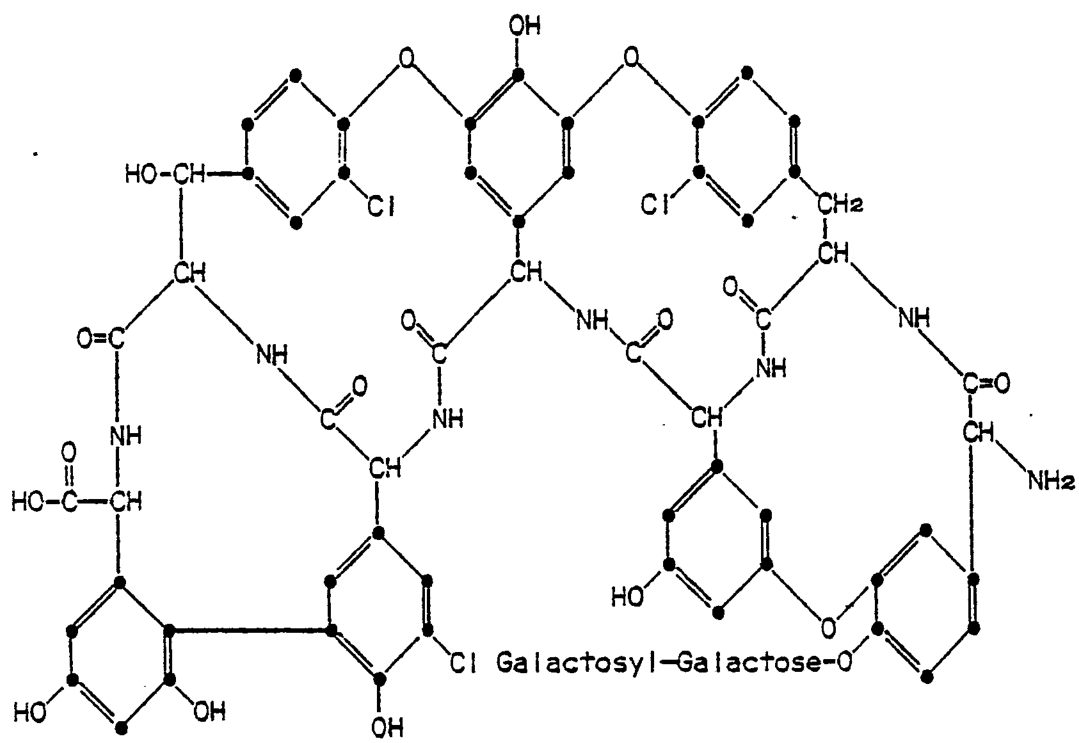

ou un de ses sels pharmaceutiquement acceptables.

9. Antibiotique A41030 facteur G, à savoir un solide blanc ayant:

(a) une analyse élémentaire approximative de 50,02% de carbone, 4,61% d'hydrogène, 4,74% de chlore, 6,11% d'azote, et 30,70% d'oxygène;

(b) un poids moléculaire observé d'environ 1688, tel qu'il a été déterminé par spectrométrie de masse via bombardement d'atomes rapides;

(c) un spectre d'absorption des rayons infrarouges ayant les maxima que l'on peut distinguer suivants: 3220 (très large, fort), 2975 (net, faible), 2920 (net, faible), 1659 (normal, fort), 1594 (large, fort), 1512 (net, fort), 1492 (saillie), 1430 (net, faible), 1386 (large, faible), 1337 (large, faible), 1308 (net, faible), 1264 (net, faible), 1230 (large, moyen), 1145 (large moyen), 1077 (net, moyen), 1062 (net, moyen), 1014 (net, moyen), et 846 (large, moyen) $cm^{-1}$;

(d) des spectres d'absorption de l'ultraviolet avec une absorption maximale, dans un mélange méthanol:eaqu (1:1), dans des conditions acides ou neutres, à 278 nm (ε 15.000) et, dans un mélange méthanol:eau (1:1), dans des conditions basiques, à 298 nm (ε 18.000);

(e) deux groups titrables dans du diméthylformamide aqueux à 66%, avec des valeurs $pK_a$ d'environ 5,4 et 7,0;

(f) tout en étant soluble dans des mélanges alcool-eau, dans du diméthylsulfoxyde, dans du diméthylformamide, dans des mélanges diméthylsulfoxyde-eau, dans des mélanges diméthylformamide-eau, dans de l'acide aqueux dilué, ou dans une base aqueuse diluée; ainsi que

(g) les sels non toxiques et pharmaceutiquement acceptables de cet antibiotique A41030 facteur G.

10. Procédé selon la revendication 1, suivi de la séparation du complexe hors du milieu de culture.

11. Procédé selon la revendication 10, caractérisé en ce que l'on isole les facteurs A, B, C, D, E, F ou G hors du complexe.

12. Complexe A41030, ou ses facteurs A, B, C, D, E, F ou G, selon la revendication 2, ou encore un de leurs sels pharmaceutiquement acceptables, à des fins d'utilisation dans la chimothérapie pharmaceutique ou vétérinaire.

13. Complexe A41030, ou ses facteurs A, B, C, D, E, F ou G, selon la revendication 2, ou encore un de leurs sels pharmaceutiquement acceptables, à des fins d'amélioration de la production laitière chez les ruminants en période d'allaitement.

14. Pré-mélange alimentaire comprenant, comme ingrédient actif, le complexe A41030, l'antibiotique A41030, facteur A, B, C, D, E, F ou G, selon la revendication 2, ou encore un de leurs sels pharmaceutiquement acceptables.

15. Complexe A41030, ou ses facteurs A, B, C, D, E, F ou G, selon la revendication 2, ou encore un de leurs sels pharmaceutiquement acceptables, à des fins d'augmentation de l'efficacité du rendement alimentaire chez les ruminants.

16. Complexe A41030, antibiotique A41030 facteurs A, B, C, D, E, F ou G, selon la revendication 2, ou encore un de leurs sels pharmaceutiquement acceptables, pour activer la croissance chez les animaux à sang chaud.

17. Culture axène du micro-organisme Streptomyces virginiae NRRL 12525.

18. Streptomyces virginiae NRRL 12525.

19. Culture axène de Streptomyces virginiae NRRL 15156.

20. Formulation pharmaceutique comprenant, comme ingrédient actif, l'antibiotique A41030 facteur A, B, C, D, E, F ou G, selon la revendication 2, ou un de leurs sels pharmaceutiquement acceptables, en association avec un diluant ou un support pharmaceutiquement acceptables, ou plus, en vue de cette formulation.

21. Formulation vétérinaire comprenant, comme ingrédient actif, le complex A41030, ou ses facteurs A, B, C, D, E, F ou G selon la revendication 2, ou encore un des leurs sels pharmaceutiquement acceptables, en association avec un diluant ou un support pharmaceutiquement acceptable, ou plus, en vue de cette formulation.

**Revendications pour l'Etat contractant: AT**

1. Procédé de préparation du complexe antibiotique A41030 ou du facteur A, B, C, D, E, F, ou G, ce procédé consistant à cultiver Streptomyces virginiae NRRL 15156 ou 12525, ou un de ses variants ou encore un de ses mutants producteur de A410301, dans un milieu de culture contenant des sources assimilables de carbone, d'azote et de sels inorganiques, dans des conditions de fermentation aérobie submergée.

2. Procédé selon la revendication 1, suivi de la séparation du complexe hors du milieu de culture.

3. Procédé selon la revendication 2, caractérisé en ce que l'on isole les facteurs A, B, C, D, E, F ou G hors du complexe.

4. Procédé de préparation, selon la revendication 3, pour la préparation de l'antibiotique A41030 facteur A de structure:

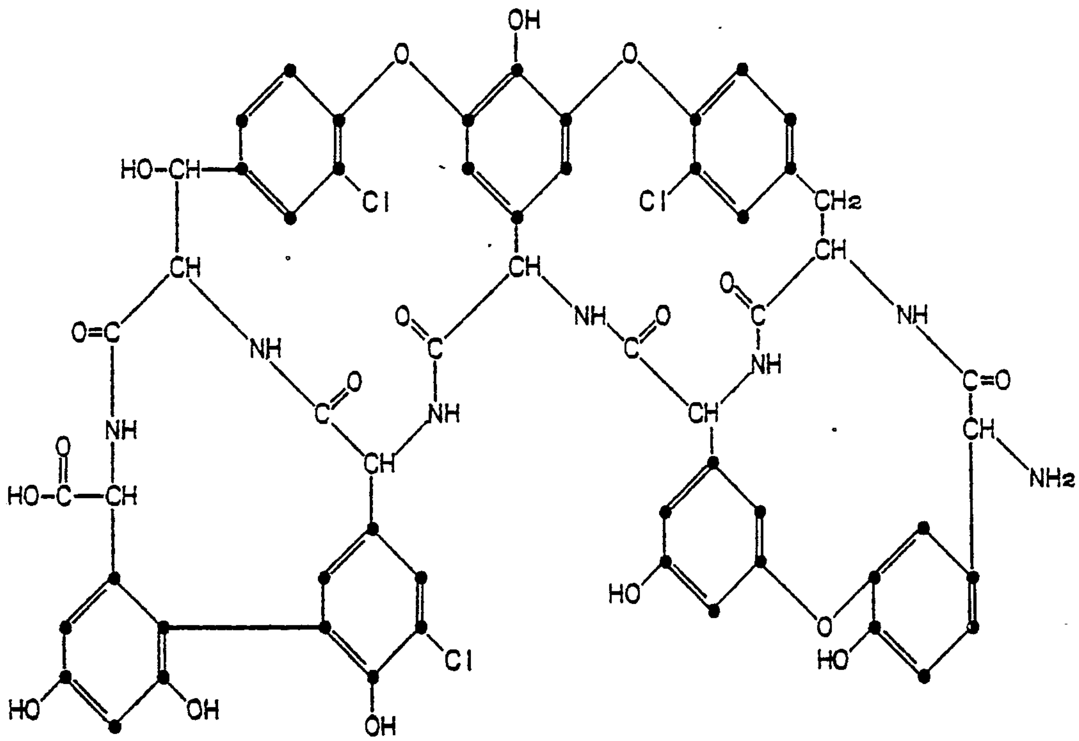

ou un de ses sels pharmaceutiquement acceptables.

5. Procédé, selon la revendication 3, en vue de préparer l'antibiotique A41030 facteur B de structure:

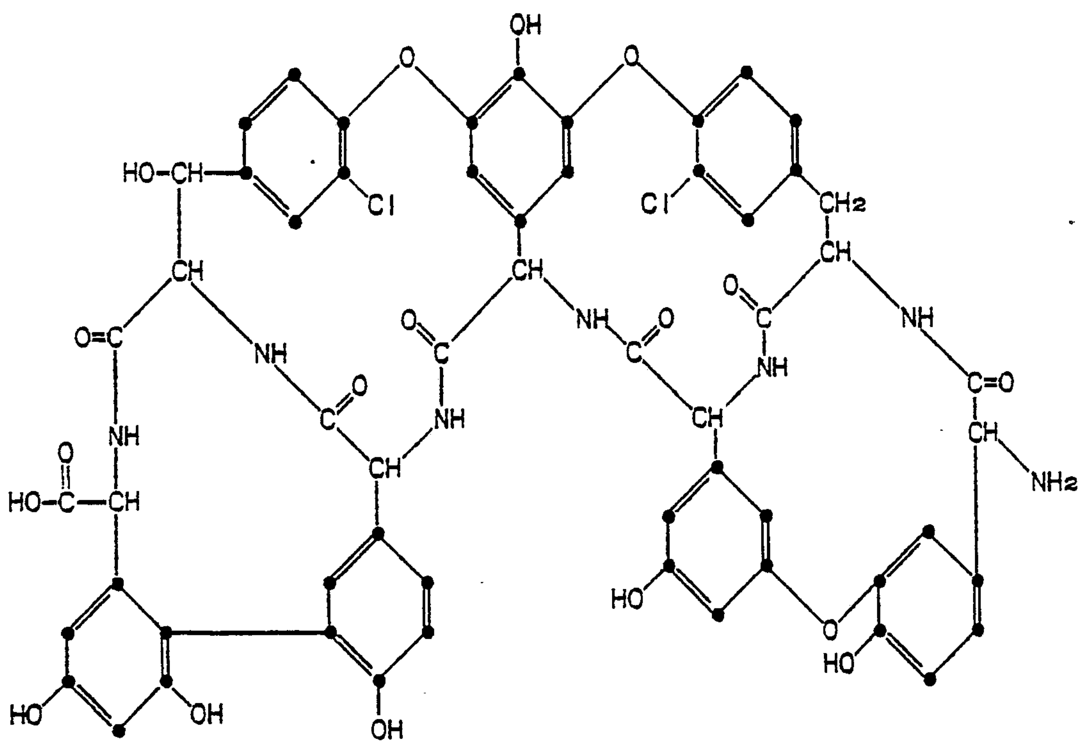

ou un de ses sels pharmaceutiquement acceptables.

6. Procédé, selon la revendication 3, en vue de préparer l'antibiotique A41030 facteur C de stucture:

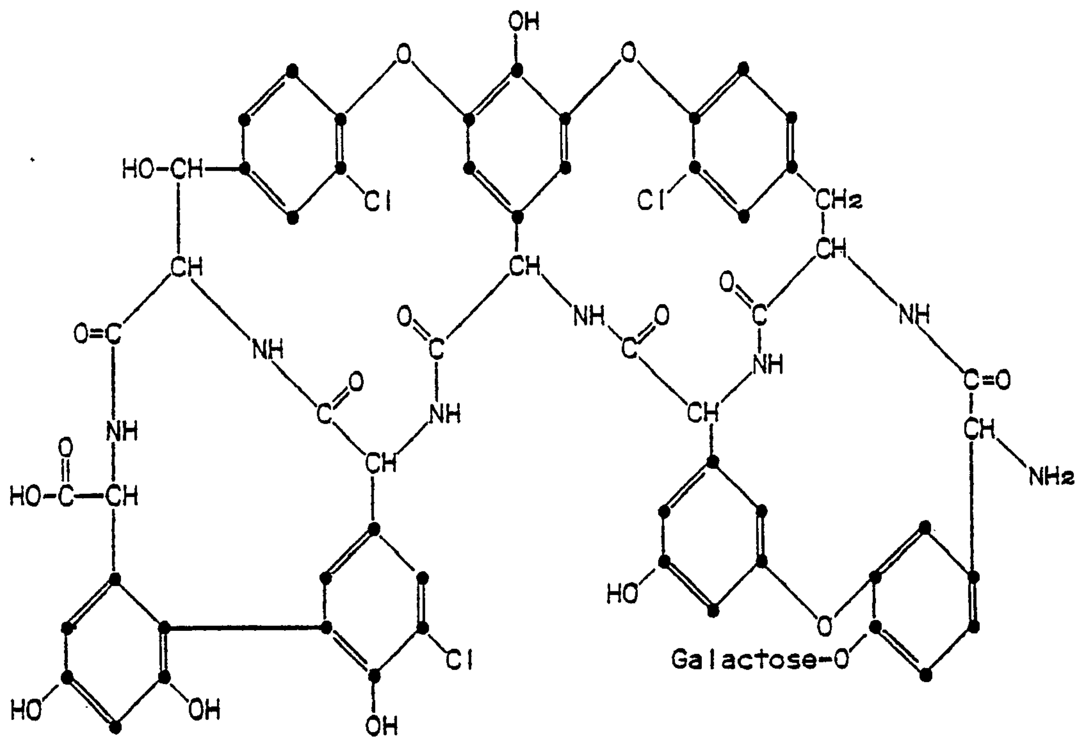

ou un de ses sels pharmaceutiquement acceptables.

7. Procédé, selon la revendication 3, en vue de préparer l'antibiotique A41030 facteur D, à savoir un solide blanc, amorphe ayant:

(a) une analyse élémentaire approximative de 54,46% de carbone, 4,35% d'hydrogène, 7,58% d'azote, 4,27% de chlore et, par différence, 29,34% d'oxygène;

(b) un poids moléculaire observé d'environ 1326, tel qu'il a été déterminé par spectrométrie de masse, via bombardement d'atomes rapides;

(c) un spectre d'absorption des rayons infrarouges ayant les maxima que l'on peut distinguer suivants: 3448—3226 (fort, large), 2959 (faible), 1661 (fort), 1592 (fort), 1511 (fort), 1429 (faible), 1290 (faible), 1227 (faible), 1212 (moyen), 1163 (faible), 1143 (faible), 1053 (moyen), et 1010 (fort) $cm^{-1}$;

(d) des spectres d'absorption de l'ultraviolet avec une absorption maximale, dans un mélange méthanol:eau (1:1), dans des conditions acides ou basiques, à 278 nm (ε 10.600) et, dans un mélange méthanol:eau (1:1), dans des conditions basiques, à 298 nm (ε 19.900);

(e) deux groupes titrables dans du diméthylformamide aqueux à 66%, avec des valeurs $pK_a$ d'environ 5,5 et 7,6;

(f) tout étant soluble dans des mélanges alcool-eau, dans du diméthylsulfoxyde, dans du diméthylformamide, dans des mélanges diméthylsulfoxyde-eau, dans des mélanges diméthylformamide-eau, dans de l'acide aqueux dilué, ou dans une base aqueuse diluée; ainsi que

(g) les sels non toxiques, et pharmaceutiquement acceptables de cet antibiotique A41030 facteur D.

8. Procédé, selon la revendication 3, en vue de préparer l'antibiotique A41030 facteur E de structure:

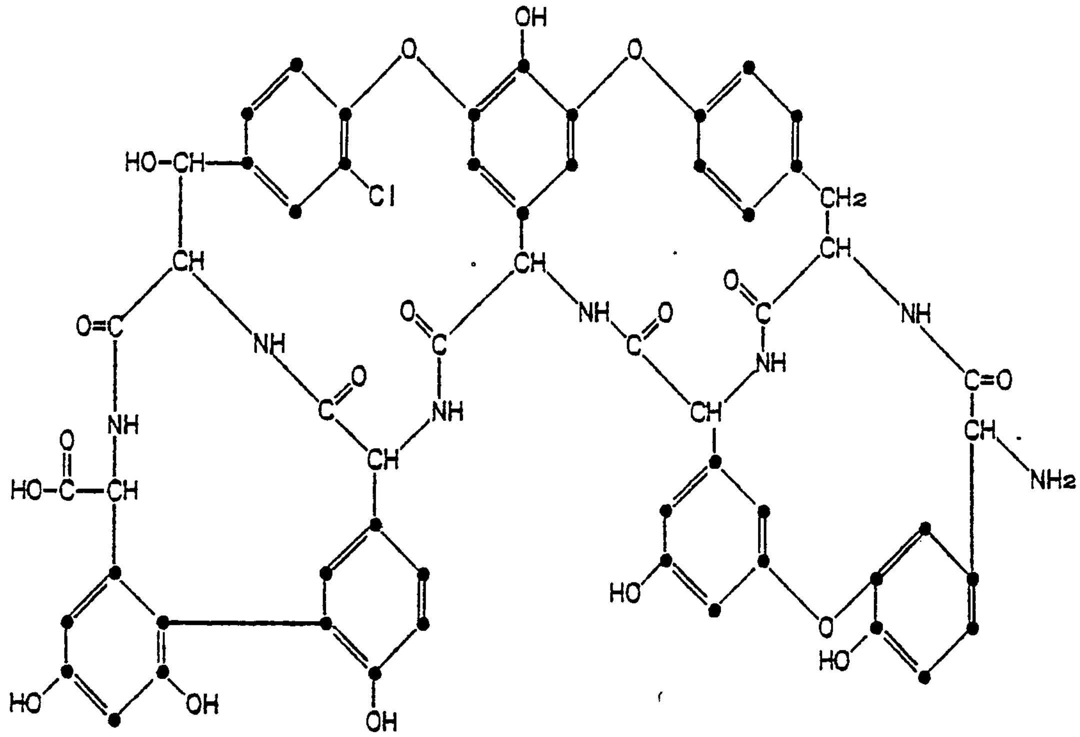

ou un de ses sels pharmaceutiquement acceptables.

9. Procédé, selon la revendication 3, en vue de préparer l'antibiotique A41030 facteur F de structure:

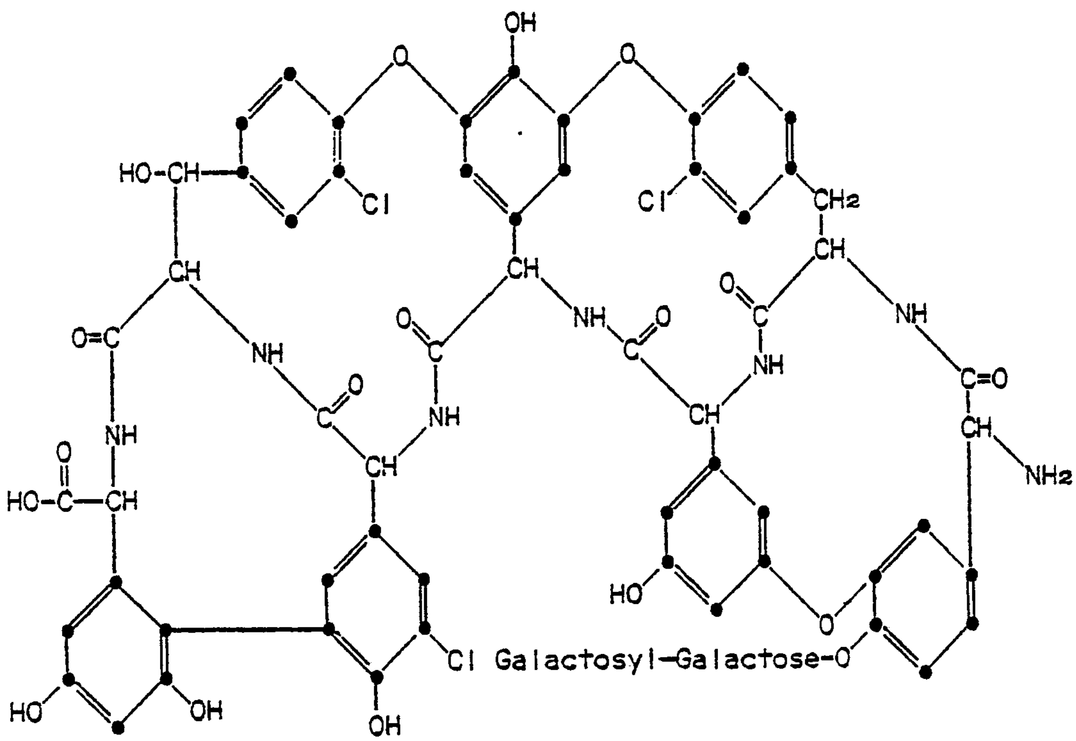

ou un de ses sels pharmaceutiquement acceptables.

10. Procédé, selon la revendication 3, en vue de préparer l'antibiotique A41030 facteur G, à savoir un solide blanc ayant:

(a) une analyse élémentaire approximative de 50,02% de carbone, 4,61% d'hydrogène, 4,74% de chlore, 6,11% d'azote, et 30,70% d'oxygène;

(b) un poids moléculaire observé d'environ 1688, tel qu'il a été déterminé par spectrométrie de masse via bombardement d'atomes rapides;

(c) un spectre d'absorption des rayons infrarouges ayant les maxima que l'on peut distinguer suivants: 3220 (très large, fort), 2975 (net, faible), 2920 (net, faible), 1659 (normal, fort), 1594 (large, fort), 1512 (net, fort), 1492 (saillie), 1430 (net, faible), 1386 (large, faible), 1337 (large, faible), 1308 (net, faible), 1264 (net, faible), 1230 (large, moyen), 1145 (large moyen), 1077 (net, moyen), 1062 (net, moyen), 1014 (net, moyen), et 846 (large, moyen) $cm^{-1}$;

(d) des spectres d'absorption de l'ultraviolet avec une absorption maximale, dans un mélange méthanol:eaqu (1:1), dans des conditions acides ou neutres, à 278 nm (ε 15.000) et, dans un mélange méthanol:eau (1:1), dans des conditions basiques, à 298 nm (ε 18.000);

(e) deux groups titrables dans du diméthylformamide aqueux à 66%, avec des valeurs $pK_a$ d'environ 5,4 et 7,0;

(f) tout en étant soluble dans des mélanges alcool-eau, dans du diméthylsulfoxyde, dans du diméthylformamide, dans des mélanges diméthylsulfoxyde-eau, dans des mélanges diméthylformamide-eau, dans de l'acide aqueux dilué, ou dans une base aqueuse diluée; ainsi que

(g) les sels non toxiques et pharmaceutiquement acceptables de cet antibiotique A41030 facteur G.

11. Complexe antibiotique A41030 ou ses facteurs A, B, C, D, E, F ou G, que l'on peut obtenir via le procédé selon la revendication 1, ou un de leurs sels pharmaceutiquement acceptables, tels que préparés selon n'importe lequel des porocédés de l'une quelconque des revendications 1 à 10.

12. Culture axène du micro-organisme Streptomyces virginiae NRRL 12525.

13. Strpetomyces virginiae NRRL 12525.

14. Culture axène de Streptomyces virginiae NRRL 15156.

15. Utilisation du complexe A41030 ou de ses facteurs A, B, C, D, E, F ou G, que l'on peut obtenir via la procédé selon la revendication 1, ou encore d'un de leurs ses pharmaceutiquement acceptables, à des fins d'amélioration de la production laitière chez un ruminant en période d'allaitement.

16. Utilisation du complexe A41030 ou de ses facteurs A, B, C, D, E, F ou G, que l'on peut obtenir via la procédé selon la revendication 1, ou encore d'un de leurs ses pharmaceutiquement acceptables, à des fins d'augmentation de l'efficacité du rendement alimentaire chez un ruminant.

17. Utilisation du complex A41030, de l'antibiotique A41030 facteurs A, B, C, D, E, F ou G, que l'on peut obtenir via le procédé selon la revendication 1, ou encore d'un de leurs sels pharmaceutiquement acceptables, pour activer la croissance chez les animaux à sang chaud.

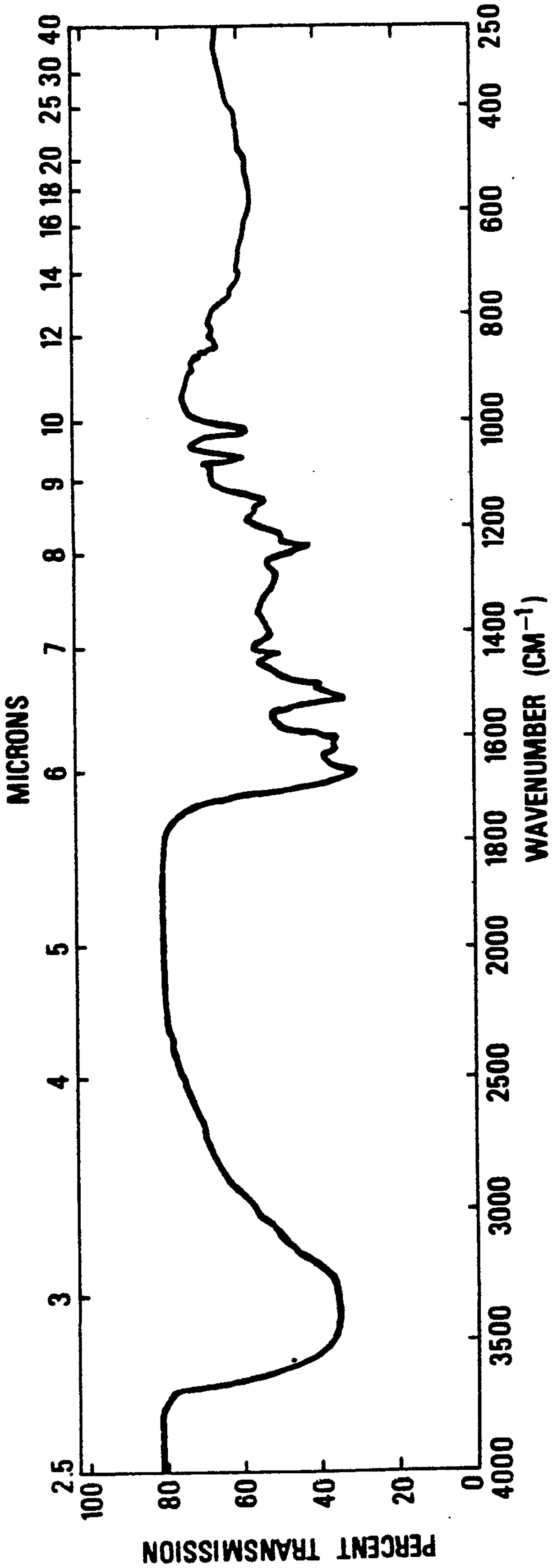
MICRONS
2.5 3 4 5 6 7 8 9 10 12 14 16 18 20 25 30 40
PERCENT TRANSMISSION
100 80 60 40 20 0
WAVENUMBER (CM$^{-1}$)
4000 3500 3000 2500 2000 1800 1600 1400 1200 1000 800 600 400 250

FIG. 1

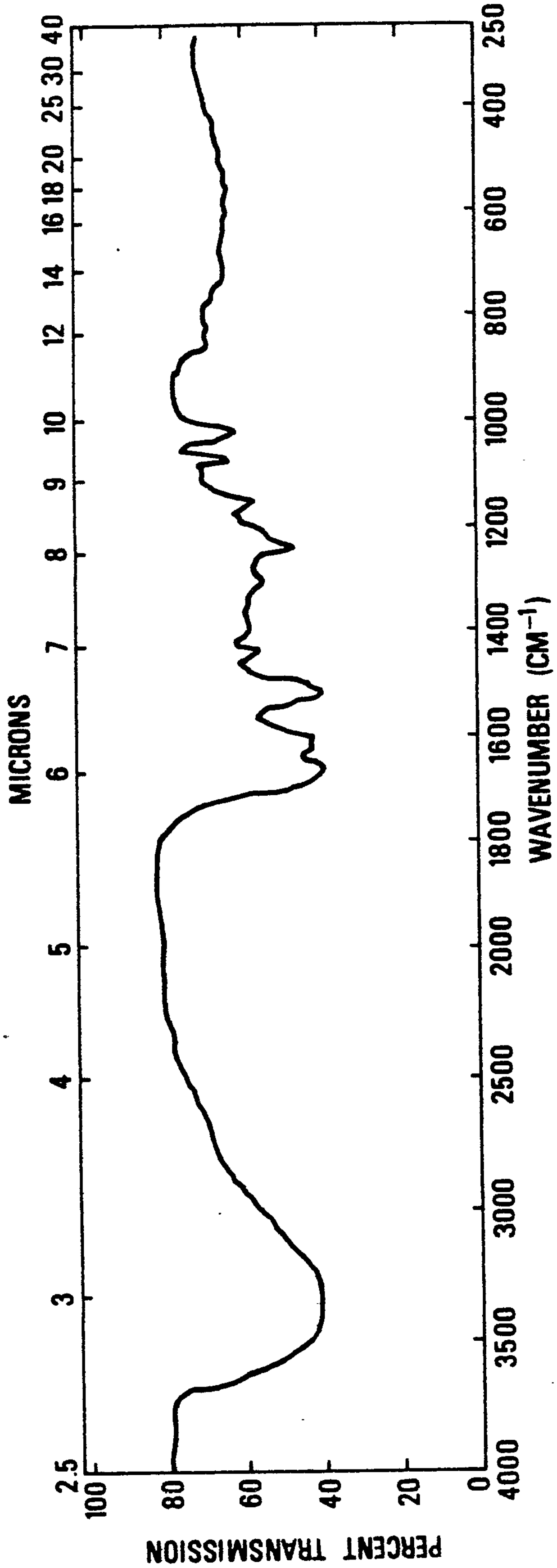
MICRONS
2.5
3
4
5
6
7
8
9
10
12
14
16
18
20
25
30
40
WAVENUMBER (CM$^{-1}$)
4000
3500
3000
2500
2000
1800
1600
1400
1200
1000
800
600
400
250
PERCENT TRANSMISSION
100
80
60
40
20
0

FIG. 2

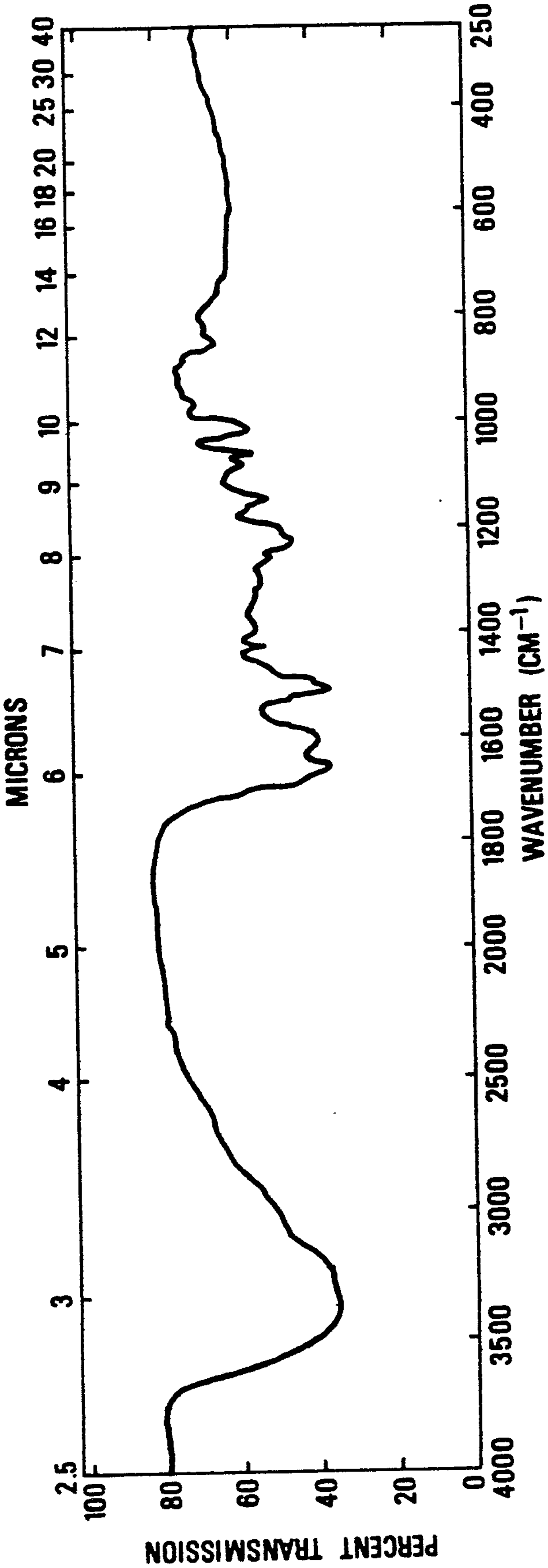
MICRONS
2.5
3
4
5
6
7
8
9
10
12
14
16 18 20
25 30 40
PERCENT TRANSMISSION
100
80
60
40
20
0
WAVENUMBER (CM⁻¹)
4000
3500
3000
2500
2000
1800
1600
1400
1200
1000
800
600
400
250

FIG. 3

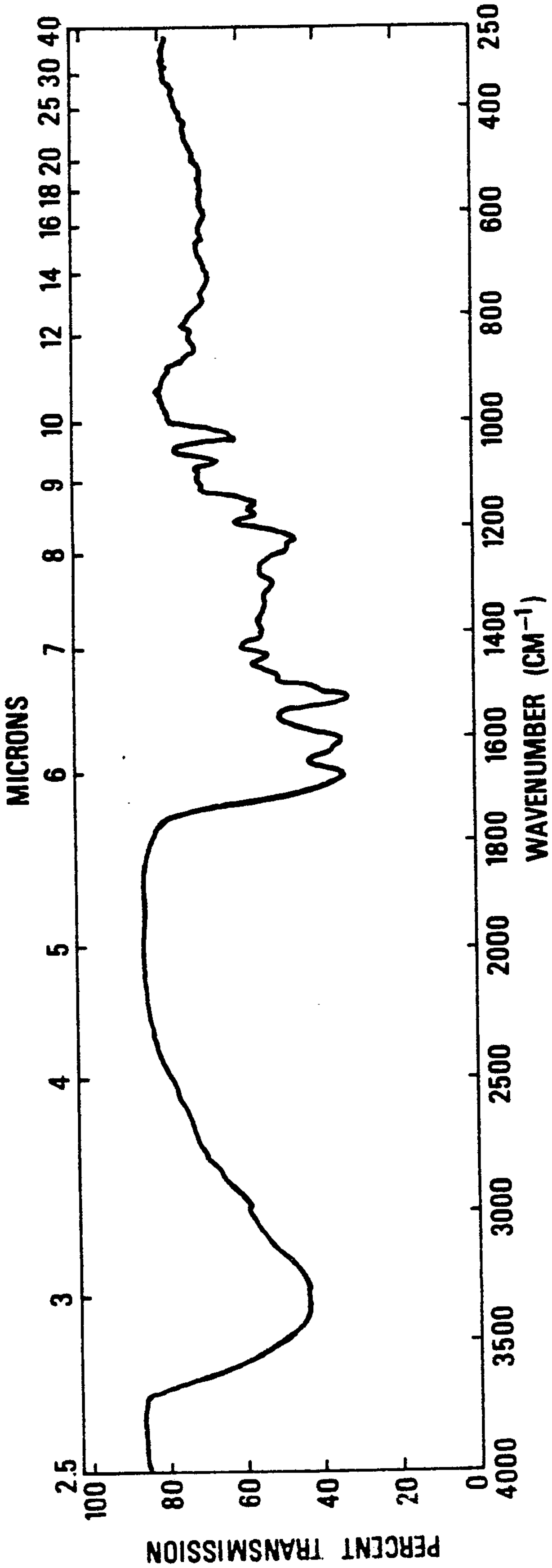
MICRONS
2.5
3
4
5
6
7
8
9
10
12
14
16 18 20
25 30 40
PERCENT TRANSMISSION
100
80
60
40
20
0
WAVENUMBER (CM$^{-1}$)
4000
3500
3000
2500
2000
1800
1600
1400
1200
1000
800
600
400
250

FIG. 4

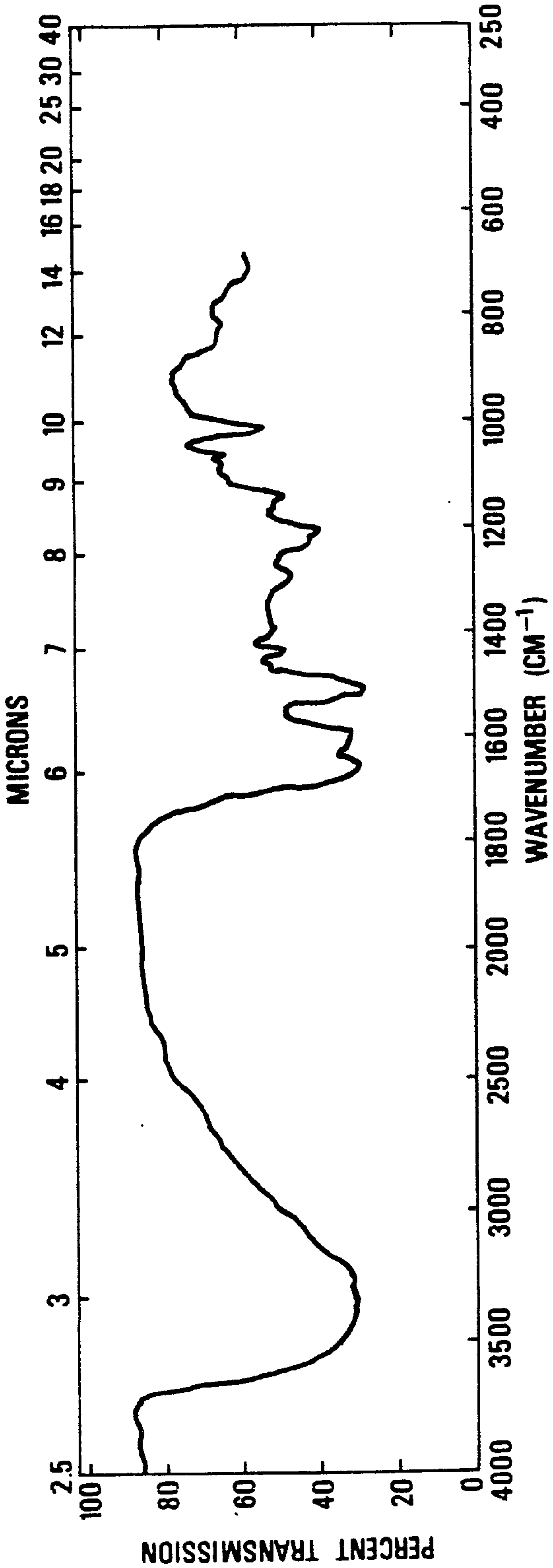
MICRONS
2.5
3
4
5
6
7
8
9
10
12
14
16 18 20
25 30 40
PERCENT TRANSMISSION
100
80
60
40
20
0
WAVENUMBER (CM⁻¹)
4000
3500
3000
2500
2000
1800
1600
1400
1200
1000
800
600
400
250

FIG. 5

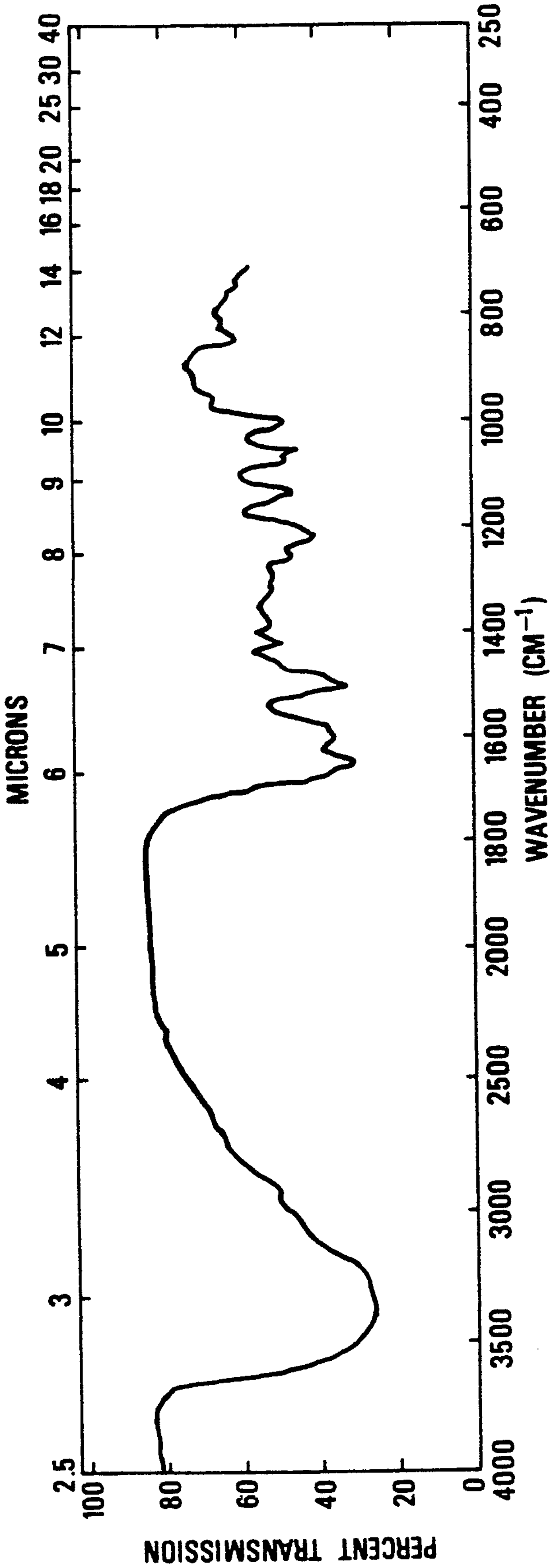
MICRONS
2.5
3
4
5
6
7
8
9
10
12
14
16 18 20
25 30 40
WAVENUMBER ($CM^{-1}$)
4000
3500
3000
2500
2000
1800
1600
1400
1200
1000
800
600
400
250
PERCENT TRANSMISSION
100
80
60
40
20
0

FIG. 6

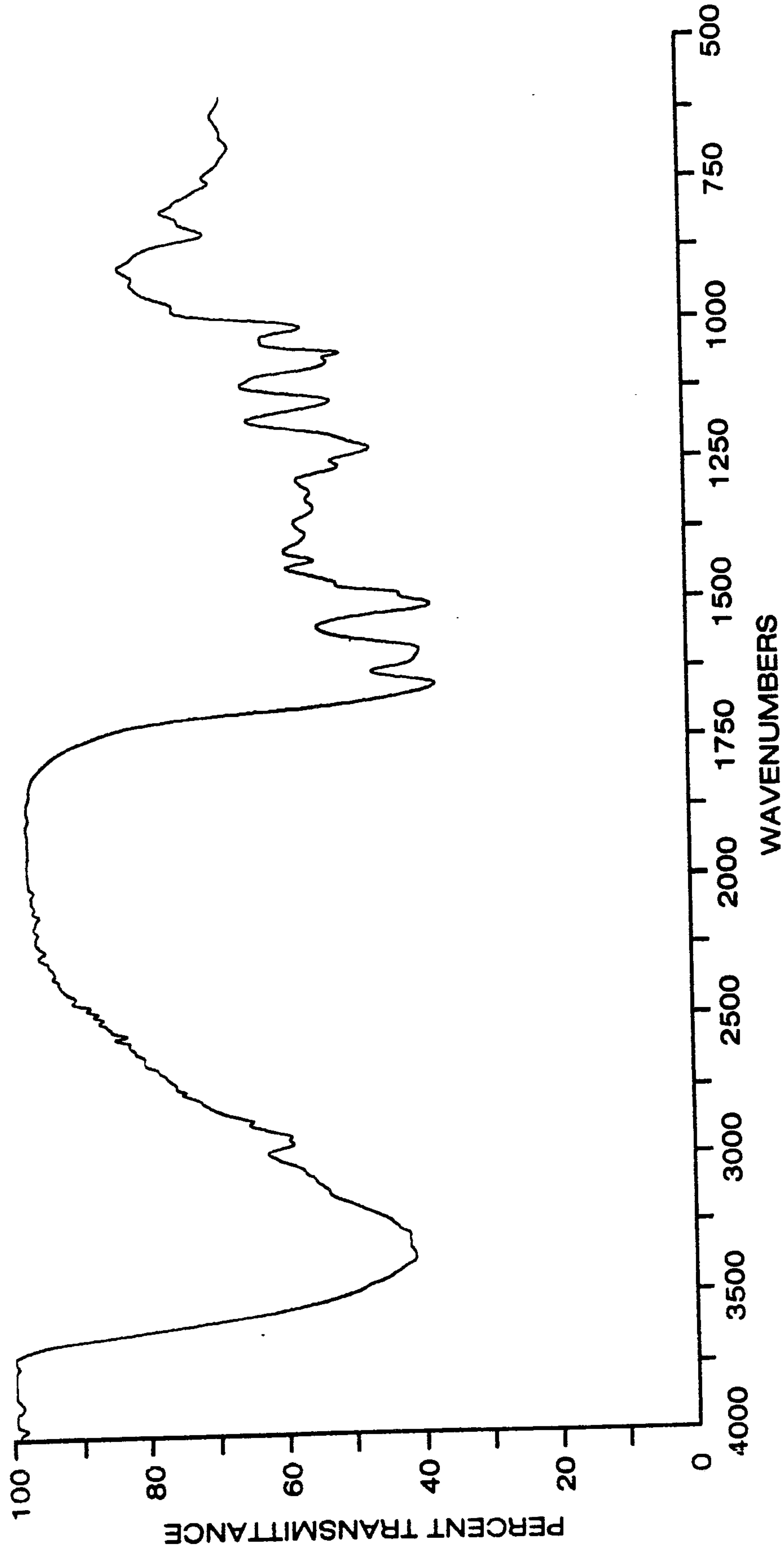
PERCENT TRANSMITTANCE
0
20
40
60
80
100
WAVENUMBERS
4000
3500
3000
2500
2000
1750
1500
1250
1000
750
500

FIG. 7